# EUROPEAN PATENT SPECIFICATION

(11) **EP 1 532 120 B1**
(45) Date of publication and mention of the grant of the patent: **14.01.2009**
(21) Application number: 03784243.2
(22) Date of filing: 04.08.2003
(51) Int. Cl.: C07D 237/24, C07D 237/08, C07D 237/20, C07D 237/14, C07D 401/10, C07D 405/10, C07D 403/10, C07D 403/12, C07D 401/12, C07D 409/04, C07D 401/04, C07D 405/14, C07D 409/14, C07D 401/14, C07D 417/14, C07D 403/04, A61K 31/4965, A61K 31/497

(54) **PHENYLPYRIDAZINE DERIVATIVES AS LIGANDS FOR GABA RECEPTORS**
PHENYLPYRIDAZIN DERIVATIVE ALS LIGANDEN FÜR GABA-REZEPTOREN
DERIVES DE PHENYLPYRIDAZINE UTILISES EN TANT QUE LIGANDS POUR DES RECEPTEURS GABA

(30) Priority: 13.08.2002 GB 0218874; 19.12.2002 GB 0229591
(43) Date of publication of application: 25.05.2005
(73) Proprietor: MERCK SHARP & DOHME LTD., Hoddesdon, Hertfordshire EN11 9BU (GB)
(72) Inventor: BLACKABY, Wesley Peter, Essex CM20 2QR (GB); BLURTON, Peter, Essex CM20 2QR (GB); BURKAMP, Frank, Essex CM20 2QR (GB); FLETCHER, Stephen Robert, Essex CM20 2QR (GB); JENNINGS, Andrew, Essex CM20 2QR (GB); LEWIS, Richard Thomas, Essex CM20 2QR (GB); MACLEOD, Angus Murray, Essex CM20 2QR (GB); STREET, Leslie Joseph, Essex CM20 2QR (GB); THOMAS, Steve, Essex CM20 2QR (GB); VAN NIEL, Monique Bodil, Essex CM20 2QR (GB); WILSON, Kevin, Essex CM20 2QR (GB)
(74) Representative: Horgan, James Michael Frederic
(86) International application number: PCT/GB2003/003376
(87) International publication number: WO 2004/014865

(56) References cited:
- EP-A- 0 072 726
- WO-A-02/22605
- WO-A-02/38568
- US-A- 4 810 705
- US-B1- 6 255 305
- WERMUTH, CAMILLE GEORGES ET AL: "Synthesis and structure-activity relationships of a series of aminopyridazine derivatives of.gamma.-aminobutyric acid acting as selective GABA-A antagonists" JOURNAL OF MEDICINAL CHEMISTRY (1987), 30(2), 239-49, XP002257939
- AURICCHIO, SERGIO ET AL: "New cleavage of the azirine ring by single electron transfer: the synthesis of 2H-imidazoles, pyridazines and pyrrolines" EUROPEAN JOURNAL OF ORGANIC CHEMISTRY (2001), (6), 1183-1187, XP002257940
- RIVAL, YVELINE ET AL: "5-HT3 Antagonists Derived from Aminopyridazine-type Muscarinic M1 Agonist" JOURNAL OF MEDICINAL CHEMISTRY (1998), 41(3), 311-317, XP002257941
- SOUTH, MICHAEL S. ET AL: "Synthesis and Reactions of Haloazodienes. A New and General Synthesis of Substituted Pyridazines" JOURNAL OF ORGANIC CHEMISTRY (1996), 61(25), 8921-8934, XP002257942
- BLY, RUTA K. ET AL: "Heterocyclic studies. XIII. The aldol condensation of 2,3-dihydro-5-methy 6-phenyl-4H-1,2diazepin-4-one and rearrangement to a pyridazine" JOURNAL OF ORGANIC CHEMISTRY (1964), 29(8), 2128-35, XP002257943
- DATABASE CA [Online] CHEMICAL ABSTRACTS SERVICE, COLUMBUS, OHIO, US; ATKINSON, C. M. ET AL: "Triazaphenanthrenes. III. Synthesis of some 9-aryl-2,3,10- triazaphenanthrenes" retrieved from STN Database accession no. 53:67737 XP002257945 & JOURNAL OF THE CHEMICAL SOCIETY, ABSTRACTS (1959) 1-5,
- DATABASE CA [Online] CHEMICAL ABSTRACTS SERVICE, COLUMBUS, OHIO, US; SAMSONOV, V. A. ET AL: "Preparation and reactions of pyrroline N-oxides obtained by reaction of isonitroso ketones with enamines" retrieved from STN Database accession no. 126:18740 XP002257946 & KHIMIYA GETEROTSIKLICHESKIKH SOEDINENII (1996), (8), 1055-1061,
- HARRIS, R.L.N. ET AL.: "The effect of C-2-carboxyphenyl Derivatives of Certain Heterocyclic Compounds on Root Geotropism in Cress Seedlings" PESTICIDE SCIENCE, vol. 11, 1980, pages 439-444, XP009019048
- OLIVER, J.E ET AL.: "Alkylation and Rearrangement of Lithiated 3-Methyl-1,2-benzisoxazoles" JOURNAL OF ORGANIC CHEMISTRY, vol. 54, 1989, pages 4970-4973, XP002257944
- BREUKELMAN, S.P: "Preparation and some Reactions of 4- and 5-Aryl-4,5-dihydropyrazin-3(2H)-ones" J. CHEM. SOC. PERKIN TRANS., 1985, pages 1627-1635, XP009019047
- JOJMA, T. ET AL.: "Pyridazine I. Novel Intramolecular Cycloaddition of 3-Chloro-6-(2-allylphenoxy)pyridazines" CHEMICAL&PHARMACEUTICAL BULLETIN, vol. 20, no. 10, - 1972 pages 2191-2203, XP009019056

## Description

The present invention relates to a class of substituted pyridazine derivatives and to their use in therapy. More particularly, this invention is concerned with 4-phenylpyridazine analogues. These compounds are ligands for GABA_{A} receptors and are therefore useful in the therapy of deleterious neurological complaints.

Receptors for the major inhibitory neurotransmitter, gamma-aminobutyric acid (GABA), are divided into two main classes: (1) GABA_{A} receptors, which are members of the ligand-gated ion channel superfamily; and (2) GABA_{B} receptors, which may be members of the G-protein linked receptor superfamily. Since the first cDNAs encoding individual GABA_{A} receptor subunits were cloned the number of known members of the mammalian family has grown to include at least six α subunits, four β subunits, three γ subunits, one δ subunit, one ε subunit and two ρ subunits.

Although knowledge of the diversity of the GABA_{A} receptor gene family represents a huge step forward in our understanding of this ligand-gated ion channel, insight into the extent of subtype diversity is still at an early stage. It has been indicated that an α subunit, a β subunit and a γ subunit constitute the minimum requirement for forming a fully functional GABA_{A} receptor expressed by transiently transfecting cDNAs into cells. As indicated above, δ, ε and ρ subunits also exist, but are present only to a minor extent in GABA_{A} receptor populations.

Studies of receptor size and visualisation by electron microscopy conclude that, like other members of the ligand-gated ion channel family, the native GABA_{A} receptor exists in pentameric form. The selection of at least one α, one β and one γ subunit from a repertoire of seventeen allows for the possible existence of more than 10,000 pentameric subunit combinations. Moreover, this calculation overlooks the additional permutations that would be possible if the arrangement of subunits around the ion channel had no constraints (i.e. there could be 120 possible variants for a receptor composed of five different subunits).

Receptor subtype assemblies which do exist include, amongst many others, α1β2γ2, α2βγ1, α2β2/3γ2, α3βγ2/3, α4βδ, α5β3γ2/3, α6βγ2 and α6βδ. Subtype assemblies containing an α1 subunit are present in most areas of the brain and are thought to account for over 40% of GABA_{A} receptors in the rat. Subtype assemblies containing α2 and α3 subunits respectively are thought to account for about 25% and 17% of GABA_{A} receptors in the rat. Subtype assemblies containing an α5 subunit are expressed predominantly in the hippocampus and cortex and are thought to represent about 4% of GABA_{A} receptors in the rat.

A characteristic property of all known GABA_{A} receptors is the presence of a number of modulatory sites, one of which is the benzodiazepine (BZ) binding site. The BZ binding site is the most explored of the GABA_{A} receptor modulatory sites, and is the site through which anxiolytic drugs such as diazepam and temazepam exert their effect. Before the cloning of the GABA_{A} receptor gene family, the benzodiazepine binding site was historically subdivided into two subtypes, BZ1 and BZ2, on the basis of radioligand binding studies. The BZ1 subtype has been shown to be pharmacologically equivalent to a GABA_{A} receptor comprising the α1 subunit in combination with a β subunit and γ2. This is the most abundant GABA_{A} receptor subtype, and is believed to represent almost half of all GABA_{A} receptors in the brain.

Two other major populations are the α2βγ2 and α3βγ2/3 subtypes. Together these constitute approximately a further 35% of the total GABA_{A} receptor repertoire. Pharmacologically this combination appears to be equivalent to the BZ2 subtype as defined previously by radioligand binding, although the BZ2 subtype may also include certain α5-containing subtype assemblies. The physiological role of these subtypes has hitherto been unclear because no sufficiently selective agonists or antagonists were known.

It is now believed that agents acting as BZ agonists at α1βγ2, α2βγ2 or α3βγ2 subtypes will possess desirable anxiolytic properties. Compounds which are modulators of the benzodiazepine binding site of the GABA_{A} receptor by acting as BZ agonists are referred to hereinafter as "GABA_{A} receptor agonists". The α1-selective GABA_{A} receptor agonists alpidem and zolpidem are clinically prescribed as hypnotic agents, suggesting that at least some of the sedation associated with known anxiolytic drugs which act at the BZ1 binding site is mediated through GABA_{A} receptors containing the α1 subunit. Accordingly, it is considered that GABA_{A} receptor agonists which interact more favourably with the α2 and/or α3 subunit than with α1 will be effective in the treatment of anxiety with a reduced propensity to cause sedation. Moreover, agents which are inverse agonists of the α5 subunit are likely to be beneficial in enhancing cognition, for example in subjects suffering from dementing conditions such as Alzheimer's disease. Also, agents which are antagonists or inverse agonists at α1 might be employed to reverse sedation or hypnosis caused by α1 agonists.

WO-A-0238568 (Merck Sharp & Dohme Ltd.) discloses imidazo-triazine derivatives as ligands for GABA-A receptors containing the α2 and/or α3 and/or α5 submit, mainly for treating anxiety, convulsions and cognitive disorders.

WO-A-0222605 (Vertex Pharmaceuticals Inc.) discloses substituted aminopyrazoles as protein kinase inhibitors for treating cancer, diabetes and AD.

EP-A-0072726 (Sanofi s.a.) discloses aminopyridazine derivatives active on the CNS.

Wermuth et al, J. Med. Chem., 1987, 30, 239-249 discloses a series of aminopyridazine derivatives which are selective GABA-A antagonists.

Auricchio et al, Eur. J. Org. Chem., 2001, 1183-1187 discloses 2H-pyridazines but no pharmaceutical indication is given.

Rival et al, J. Med. Chem., 1998, 41, 311-317 discloses aminopyridazine derivatives as 5-HT₃ (and muscarinic M1) agonists.

South et al, J. Org. Chem., 1996, 61, 8921-8934 discloses substituted pyridazines but no pharmaceutical indication is given.

Bly et al, J. Org. Chem., 1964, 29, 2128-35 discloses rearrangement of 2,3-dihydro-5-methyl-6-phenyl-4H-1,2-diazepin-4-one to a pyridazine. No pharmaceutical indication is given.

Atkinson and Rodway, J. Chem. Soc. Abstracts, 1959, 1-5, discloses phenylpyridazines as antibiotics.

Samsonov et al, Khim. Geterosikl. Soedin., 1996, 8, 1055-1061 discloses one pyridazine derivative. No pharmaceutical indication is given.

Harris et al, Pestic. Sci., 1980, 11, 439-444 discloses pyridazine derivatives as plant geotropic response inhibitors.

Oliver et al, J. Org. Chem., 1989, 54, 4970-4973 discloses substituted pyridazines derived from setal exudates of lace bugs.

Breukelman et al, J. Chem. Soc. Perkin Trans., 1985, 1627-1635 discloses 4- and 5-phenyl-4,5-dihydropyridazin-3(2H)-ones.

Jojima et al, Chem. Pharm. Bull., 20(10), 1972, 2191-2203 discloses a phenylpyridazine but no activity for it is given.

US-A-4810705 (Sanofi) discloses 7-substituted 1,2,4-triazolo[4,3-b]pyridazines as antidotes to benzodiazepines.

US-B-6255305 discloses substituted triazolopyridazines as ligands for GABA-A receptors containing α2 and/or α3 submits.

None of the above documents discloses compounds of the present invention.

The compounds of the present invention, being selective ligands for GABA_{A} receptors, are therefore of use in the treatment and/or prevention of a variety of disorders of the central nervous system. Such disorders include anxiety disorders, such as panic disorder with or without agoraphobia, agoraphobia without history of panic disorder, animal and other phobias including social phobias, obsessive-compulsive disorder, stress disorders including post-traumatic and acute stress disorder, and generalized or substance-induced anxiety disorder; neuroses; convulsions; migraine; depressive or bipolar disorders, for example single-episode or recurrent major depressive disorder, dysthymic disorder, bipolar I and bipolar II manic disorders, and cyclothymic disorder; psychotic disorders including schizophrenia; neurodegeneration arising from cerebral ischemia; attention deficit hyperactivity disorder; Tourette's syndrome; speech disorders, including stuttering; and disorders of circadian rhythm, e.g. in subjects suffering from the effects of jet lag or shift work.

Further disorders for which selective ligands for GABA_{A} receptors may be of benefit include pain and nociception; emesis, including acute, delayed and anticipatory emesis, in particular emesis induced by chemotherapy or radiation, as well as motion sickness, and post-operative nausea and vomiting; eating disorders including anorexia nervosa and bulimia nervosa; premenstrual syndrome; muscle spasm or spasticity, e.g. in paraplegic patients; hearing disorders, including tinnitus and age-related hearing impairment; urinary incontinence; and the effects of substance abuse or dependency, including alcohol withdrawal. Selective ligands for GABA_{A} receptors may be beneficial in enhancing cognition, for example in subjects suffering from dementing conditions such as Alzheimer's disease; and may also be effective as pre-medication prior to anaesthesia or minor procedures such as endoscopy, including gastric endoscopy.

In addition, the compounds in accordance with the present invention may be useful as radioligands in assays for detecting compounds capable of binding to the human GABA_{A} receptor.

The present invention provides a class of pyridazine derivatives which possess desirable binding properties at various GABA_{A} receptor subtypes. The compounds in accordance with the present invention have good affinity as ligands for the α2 and/or α3 and/or α5 subunit of the human GABA_{A} receptor. The compounds of this invention may interact more favourably with the α2 and/or α3 subunit than with the α1 subunit; and/or may interact more favourably with the α5 subunit than with the α1 subunit.

The compounds of the present invention are GABA_{A} receptor subtype ligands having a binding affinity (Kᵢ) for the α2 and/or α3 and/or α5 subunit, as measured in the assay described hereinbelow, of 200 nM or less, typically of 100 nM or less, and ideally of 20 nM or less. The compounds in accordance with this invention may possess at least a 2-fold, suitably at least a 5-fold, and advantageously at least a 10-fold, selective affinity for the α2 and/or α3 and/or α5 subunit relative to the α1 subunit. However, compounds which are not selective in terms of their binding affinity for the α2 and/or α3 and/or α5 subunit relative to the α1 subunit are also encompassed within the scope of the present invention; such compounds will desirably exhibit functional selectivity in terms of zero or weak (positive or negative) efficacy at the α1 subunit and (i) a full or partial agonist profile at the α2 and/or α3 subunit, and/or (ii) an inverse agonist profile at the α5 subunit.

The present invention provides a compound of formula I, or an N-oxide thereof or a pharmaceutically acceptable salt thereof: 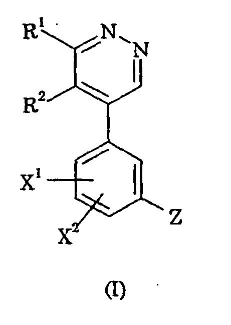 wherein
X¹ represents hydrogen, halogen, C₁₋₆alkyl, trifluoromethyl or C₁₋₆ alkoxy;
X² represents hydrogen or halogen;
Z represents an optionally substituted aryl or heteroaryl group;
R¹ represents hydrocarbon, a heterocyclic group -OR^{a}, -OSO₂CF₃, -SR^{a}, -NR^{a}R^{b} or -CO₂R^{a};
R² represents hydrogen or C₂₋₆ alkoxycarbonyl;
R^{a} is C₁₋₆alkyl, C₂₋₆alkenyl, C₃₋₇cycloalkyl or aryl(C₁₋₆)alkyl (optionally substituted by C₁₋₆alkoxy);
R^{b} is hydrogen or C₁₋₆alkyl;
heteroaryl is pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl or tetrazolyl.

The present invention also provides a compound of formula I as depicted above, or an *N*-oxide thereof or a pharmaceutically acceptable salt thereof, wherein
R¹ represents hydrogen, hydrocarbon, a heterocyclic group, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b} or -CR^{a}=NOR^{b}; and
X¹, X², Z, R², R^{a} and R^{b} are as defined above.

Where Z in the compounds of formula I above represents aryl or heteroaryl this group may be unsubstituted, or substituted by one or more substituents. Typically, the group Z will be unsubstituted, or substituted by one or two substituents. Typical substituents on the group Z include halogen, cyano, trifluoromethyl, nitro, C₁₋₆ alkyl, hydroxy, C₁₋₆ alkoxy, oxy, C₁₋₆ alkylsulphonyl, amino, aminocarbonyl, formyl, C₂₋₆ alkoxycarbonyl and -CR⁸=NOR^{b}, wherein R^{a} and R^{b} are as defined above. Illustrative substituents on Z include halogen, cyano, trifluoromethyl and C₁₋₆ alkyl.

For use in medicine, the salts of the compounds of formula I will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

The term "hydrocarbon" as used herein includes straight-chained, branched and cyclic groups containing up to 18 carbon atoms, suitably up to 15 carbon atoms, and conveniently up to 12 carbon atoms. Suitable hydrocarbon groups include C₁₋₆ alkyl, C₂₋₆ alkenyl, C₂₋₆ alkynyl, C₃₋₇ cycloalkyl, C₃₋₇ cyloalkyl(C₁₋₆)alkyl, indanyl, aryl and aryl(C₁₋₆)akyl.

The expression "a heterocyclic group" as used herein includes cyclic groups containing up to 18 carbon atoms and at least one heteroatom preferably selected from oxygen, nitrogen and sulphur. The heterocyclic group suitably contains up to 15 carbon atoms and conveniently up to 12 carbon atoms, and is preferably linked through carbon. Examples of suitable heterocyclic groups include C₃₋₇ heterocycloalkyl, C₃₋₇ heterocycloalkenyl, C₃₋₇ heterocyloakyl(C₁₋₆)akyl, heteroaryl and heteroaryl(C₁₋₆)alkyl groups.

Suitable alkyl groups include straight-chained and branched alkyl groups containing from 1 to 6 carbon atoms. Typical examples include methyl and ethyl groups, and straight-chained or branched propyl, butyl and pentyl groups. Particular alkyl groups are methyl, ethyl, n-propyl, isopropyl, isobutyl, tert-butyl and 2,2-dimethylpropyl. Derived expressions such as "C₁₋₆ alkoxy", "C₁₋₆ alkylamino" and "C₁₋₆ alkylsulphonyl" are to be construed accordingly.

Suitable alkenyl groups include straight-chained and branched alkenyl groups containing from 2 to 6 carbon atoms. Typical examples include vinyl, allyl and dimethylallyl groups.

Suitable alkynyl groups include straight-chained and branched alkynyl groups containing from 2 to 6 carbon atoms. Typical examples include ethynyl and propargyl groups.

Suitable cycloalkyl groups include groups containing from 3 to 7 carbon atoms. Particular cycloalkyl groups are cyclopropyl and cyclohexyl.

Typical examples of C₃₋₇ cyloalkyl(C₁₋₆)alkyl groups include cyclopropylmethyl, cyclohexylmethyl and cyclohexylethyl.

Particular indanyl groups include indan-1-yl and indan-2-yl.

Particular aryl groups include phenyl and naphthyl, preferably phenyl.

Particular aryl(C₁₋₆)alkyl groups include benzyl, phenylethyl, phenylpropyl and naphthylmethyl.

Suitable heterocycloalkyl groups include azetidinyl, pyrrolidinyl, piperidinyl, piperazinyl, morpholinyl and thiomorpholinyl groups.

A typical heterocycloalkenyl group is dihydropyrrolyl.

Suitable heteroaryl groups include pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl and tetrazolyl groups.

The expression "heteroaryl(C₁₋₆)alkyl" as used herein includes furylmethyl, furylethyl, thienylmethyl, thienylethyl, pyrazolylmethyl, oxazolylmethyl, oxazolylethyl, thiazolylmethyl, thiazolylethyl, imidazolylmethyl, imidazolylethyl, oxadiazolylmethyl, oxadiazolylethyl, thiadiazolylmethyl, thiadiazolylethyl, triazolylmethyl, triazolylethyl, tetrazolylmethyl, tetrazolylethyl, pyridinylmethyl, pyridinylethyl, pyrimidinylmethyl, pyrazinylmethyl, quinolinylmethyl and isoquinolinylmethyl.

The hydrocarbon and heterocyclic groups may in turn be optionally substituted by one or more groups selected from C₁₋₆ alkyl, adamantyl, phenyl, halogen, C₁₋₆ haloalkyl, C₁₋₆ aminoalkyl, trifluoromethyl, hydroxy, C₁₋₆ alkoxy, aryloxy, keto, C₁₋₃ alkylenedioxy, nitro, cyano, carboxy, C₂₋₆ alkoxycarbonyl, C₂₋₆ alkoxycarbonyl(C₁₋₆)alkyl, C₂₋₆ alkylcarbonyloxy, arylcarbonyloxy, aminocarbonyloxy, C₂₋₆ alkylcarbonyl, arylcarbonyl, C₁₋₆ alkylthio, C₁₋₆ alkylsulphinyl, C₁₋₆ alkylsulphonyl, arylsulphonyl, -NR^{v}R^{w}, -NR^{v}COR^{w}, -NR^{v}CO₂R^{w}, -NR^{v}SO₂R^{w}, -CH₂NR^{v}SO₂R^{w}, -NHCONR^{v}R^{w}, -CONR^{v}R^{w}, -SO₂NR^{v}R^{w} and -CH₂SO₂NR^{v}R^{w}, in which R^{v} and R^{w} independently represent hydrogen, C₁₋₆ alkyl, aryl or aryl(C₁₋₆)alkyl.

The term "halogen" as used herein includes fluorine, chlorine, bromine and iodine especially fluoro or chloro.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

Suitable values for the X¹ substituent include hydrogen, fluoro, chloro, methyl, trifluoromethyl and methoxy; in particular hydrogen or fluoro; and especially fluoro.

Typical values of X² include hydrogen and fluoro, especially hydrogen.

In one favoured embodiment, Z represents an optionally substituted phenyl group, in particular monosubstituted or disubstituted phenyl. In another favoured embodiment, Z represents optionally substituted pyridinyl, especially unsubstituted, monosubstituted or disubstituted pyridin-2-yl, pyridin-3-yl or pyridin-4-yl.

Examples of suitable substituents on the group Z include fluoro, chloro, cyano, trifluoromethyl, nitro, methyl, hydroxy, methoxy, oxy, metlianesulphonyl, amino, aminocarbonyl, formyl, methoxycarbonyl and -CH=NOH.

Examples of particular substituents on the group Z include fluoro, cyano, trifluoromethyl and methyl, especially fluoro or cyano.

Detailed values of Z include cyanophenyl, (cyano)(fluoro)phenyl, (chloro)(cyano)phenyl, nitrophenyl, methoxyphenyl, methanesulphonylphenyl, pyridinyl, fluoro-pyridinyl, difluoro-pyridinyl, (amino)(chloro)pyridinyl, cyano-pyridinyl, methyl-pyridinyl, hydroxy-pyridinyl, methoxy-pyridinyl, oxy-pyridinyl, aminocarbonyl-pyridinyl, pyridazinyl, pyrimidinyl, pyrazinyl, cyano-thienyl, aminocarbonyl-thienyl, formyl-thienyl, methoxycarbonyl-thienyl, thienyl-CH=NOH, thiazolyl, isothiazolyl, pyrrolyl, pyrazolyl, imidazolyl, triazolyl and methyl-tetrazolyl. Additional values of Z include fluorophenyl, difluorophenyl and trifluoromethyl-phenyl.

Representative values of Z include fluorophenyl, difluorophenyl, cyanophenyl, (cyano)(fluoro)phenyl, trifluoromethyl-phenyl, pyridinyl, fluoro-pyridinyl, difluoro-pyridinyl, cyano-pyridinyl and triazolyl

Specific values of Z include (cyano)(fluoro)phenyl, pyridinyl, fluoro-pyridinyl, difluoro-pyridinyl and triazoly

Typically, R¹ represents hydrocarbon, a heterocyclic group, -OR^{a}, -NR^{a}R^{b} or -CO₂R^{a}.

Suitably, R^{a} represents methyl, ethyl, *n*-propyl, isopropyl, allyl, cyclopropyl, cyclohexyl, benzyl or methoxybenzyl. Additionally; R^{a} may represent *tert*-butyl.

Suitably, R^{b} represents hydrogen or methyl, especially hydrogen.

Favourably, R¹ represents aryl or heteroaryl, either of which groups may be substituted by one or more substituents. Typically, the aryl or heteroaryl group R¹ will be unsubstituted, or substituted by one or two substituents. Typical substituents include halogen, C₁₋₆ alkyl, trifluoromethyl, C₁₋₆ alkoxy, cyano and oxo. Suitable substituents include halogen, C₁₋₆ alkyl, trifluoromethyl, C₁₋₆ alkoxy and cyano. Particular substituents include fluoro, chloro, methyl, trifluoromethyl, methoxy and cyano, especially fluoro.

Illustrative values of R¹ include phenyl, halophenyl, dihalophenyl, trihalophenyl, (C₁₋₆alkyl)(halo)phenyl, (trifluoromethyl)(halo)phenyl, C₁₋₆ alkoxyphenyl, (C₁₋₆ alkoxy)(halo)phenyl, cyanophenyl, (cyano)(halo)phenyl, C₃₋₇ heterocycloalkyl (optionally substituted by oxo), C₃₋₇ heterocycloalkenyl, heteroaryl (optionally substituted by one or more halogen atoms, and/or by oxo), C₁₋₆ alkoxy, C₂₋₆ alkenyloxy, aryl(C₁₋₆)alkoxy, triflyloxy, C₁₋₆ alkylthio, C₁₋₆ alkylamino, C₂₋₆ alkenylamino, C₃₋₇ cycloalkylamino, aryl(C₁₋₆)alkylamino (optionally substituted by C₁₋₆ alkoxy) and C₂₋₆ alkoxycarbonyl.

Typical values of R¹ include phenyl, halophenyl, dihalophenyl, (C₁₋₆ alkyl)(halo)phenyl, (trifluoromethyl)(halo)phenyl, C₁₋₆ alkoxyphenyl, (C₁₋₆ alkoxy)(halo)phenyl, cyanophenyl, (cyano)(halo)phenyl, C₃₋₇ heterocycloalkenyl, heteroaryl, (optionally substituted by one or more halogen atoms), C₁₋₆ alkoxy, C₂₋₆ alkenyloxy, aryl(C₁₋₆)alkoxy, triflyloxy, C₁₋₆ alkylthio, C₁₋₆ alkylamino, C₂₋₆ alkenylamino, C₃₋₇ cycloalkylamino, aryl(C₁₋₆)alkylamino (optionally substituted by C₁₋₆ alkoxy) and C₂₋₆ alkoxycarbonyl.

Suitable values of R¹ include phenyl, halophenyl, dihalophenyl, C₁₋₆ alkoxyphenyl, cyanophenyl, (cyano)(halo)phenyl, C₃₋₇ heterocycloalkenyl, heteroaryl (optionally substituted by halo), C₁₋₆ alkoxy, C₂₋₆ alkenyloxy, aryl(C₁₋₆)alkoxy, C₁₋₆ alkylamino, C₂₋₆ alkenylamino, C₃₋₇ cycloalkylamino, aryl(C₁₋₆)alkylamino (optionally substituted by C₁₋₆ alkoxy) and C₂₋₆ alkoxycarbonyl.

Representative values of R¹ include phenyl, fluorophenyl, chlorophenyl, difluorophenyl, (chloro)(fluoro)phenyl, trifluorophenyl, (fluoro)(methyl)phenyl, (fluoro)(triffuoromethyl)phenyl, methoxyphenyl, (fluoro)(methoxy)phenyl, cyanophenyl, (cyano)(fluoro)phenyl, oxo-pyrrolidinyl, dihydropyrrolyl, pyridinyl, fluoro-pyridinyl, difluoro-pyridinyl, oxo-pyridinyl, pyrazinyl, furyl, thienyl, thiazolyl, triazolyl, methoxy, ethoxy, allyloxy, benzyloxy, triflyloxy, ethylthio, tert-butylthio, methylamino, ethylamino, propylamino, isopropylamino, allylamino, cyclopropylamino, cyclohexylamino, benzylamino, methoxybenzyl-amino and ethoxycarbonyl.

Particular values of R¹ include phenyl, fluorophenyl, chlorophenyl, difluorophenyl, (chloro)(fluoro)phenyl, (fluoro)(methyl)phenyl, (fluoro)(trifluoromethyl)phenyl, methoxyphenyl, (fluoro)(methoxy)phenyl, cyanophenyl, (cyano)(fluoro)phenyl, dihydropyrrolyl, pyridinyl, fluoro-pyridinyl, difluoro-pyridinyl, pyrazinyl, furyl, thienyl, thiazolyl, triazolyl, methoxy, ethoxy, allyloxy, benzyloxy, triflyloxy, ethylthio, tert-butylthio, methylamino, ethylamino, propylamino, isopropylamino, allylamino, cyclopropylamino, cyclohexylamino, benzylamino, methoxybenzyl-amino and ethoxycarbonyl.

Individual values of R¹ include phenyl, fluorophenyl, chlorophenyl, difluorophenyl, methoxyphenyl, cyanophenyl, (cyano)(fluoro)phenyl, dihydropyrrolyl, pyridinyl, fluoro-pyridinyl, pyrazinyl, furyl, thienyl, thiazolyl, triazolyl, methoxy, ethoxy, allyloxy, benzyloxy, methylamino, ethylamino, propylamino, isopropylamino, allylamino, cyclopropylamino, cyclohexylamino, benzylamino, methoxybenzyl-amino and ethoxycarbonyl.

In one embodiment, R¹ represents fluorophenyl (especially 2-fluorophenyl).

In another embodiment, R¹ represents fluoro-pyridinyl (especially 3-fluoropyridin-2-yl).

In a further embodiment, R¹ represents difluoro-pyridinyl. In one aspect of this embodiment, R¹ represents 3,5-diffuoropyridin-4-yl. In another aspect of this embodiment, R¹ represents 3,5-difluoropyridin-2-yl.

In an additional embodiment, R¹ represents trifluorophenyl (especially 2,4,6-trifluorophenyl).

Suitably, R² represents hydrogen, methoxycarbonyl or ethoxycarbonyl. In a particular embodiment, R² represents hydrogen.

A particular sub-class of compounds according to the invention is represented by the compounds of formula IIA, and N-oxides thereof and pharmaceutically acceptable salts thereof: 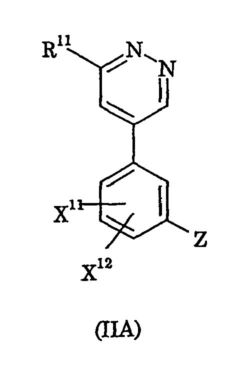 wherein
Z is as defined above;
X¹¹ represents hydrogen, fluoro, chloro, methyl, trifluoromethyl or methoxy;
X¹² represents hydrogen or fluoro; and
R¹¹ represents phenyl, halophenyl, dihalophenyl, trihalophenyl, (C₁₋₆ alkyl)(halo)phenyl, (trifluoromethyl)(halo)phenyl, C₁₋₆ alkoxyphenyl, (C₁₋₆ alkoxy)(halo)phenyl, cyanophenyl, (cyano)(halo)phenyl, C₃₋₇ heterocycloalkyl (optionally substituted by oxo), C₃₋₇ heterocycloalkenyl, heteroaryl (optionally substituted by one or more halogen atoms, and/or by oxo), C₁₋₆ alkoxy, C₂₋₆ alkenyloxy, aryl(C₁₋₆)alkoxy, triflyloxy, C₁₋₆ alkylthio, C₁₋₆ alkylamino, C₂₋₆ alkenylamino, C₃₋₇ cycloalkylamino, aryl(C₁₋₆)alkylamino (optionally substituted by C₁₋₆ alkoxy) or C₂₋₆ alkoxycarbonyl.

The present invention also provides a compound of formula IIA as depicted above, or an N-oxide thereof or a pharmaceutically acceptable salt thereof, wherein
R¹¹ represents phenyl, halophenyl, dihalophenyl, (C₁₋₆ alkyl)-(halo)phenyl, (trifluoromethyl)(halo)phenyl, C₁₋₆ alkoxyphenyl, (C₁₋₆ alkoxy)(halo)phenyl, cyanophenyl, (cyano)(halo)phenyl, C₃₋₇ heterocycloalkenyl, heteroaryl (optionally substituted by one or more halogen atoms), C₁₋₆ alkoxy, C₂₋₆ alkenyloxy, aryl(C₁₋₆)alkoxy, triflyloxy, C₁₋₆ alkylthio, C₁₋₆ alkylamino, C₂₋₆ alkenylamino, C₃₋₇ cycloalkylamino, aryl(C₁₋₆)alkylamino (optionally substituted by C₁₋₆ alkoxy) or C₂₋₆ alkoxycarbonyl; and
Z, X¹¹ and X¹² are as defined above.

The present invention further provides a compound of formula IIA as depicted above, or an N-oxide thereof or a pharmaceutically acceptable salt thereof, wherein
R¹¹ represents phenyl, halophenyl, dihalophenyl, C₁₋₆ alkoxyphenyl, cyanophenyl, (cyano)(halo)phenyl, C₃₋₇ heterocycloalkenyl, heteroaryl (optionally substituted by halo), C₁₋₆ alkoxy, C₂₋₆ alkenyloxy, aryl(C₁₋₆)alkoxy, C₁₋₆ alkylamino, C₂₋₆ alkenylamino, C₃₋₇ cycloalkylamino, aryl(C₁₋₆)alkylamino (optionally substituted by C₁₋₆ alkoxy) or C₂₋₆ alkoxycarbonyl; and
Z, X¹¹ and X¹² are as defined above.

Suitable values of X¹¹ include hydrogen and fluoro, especially fluoro.

In a favoured embodiment, X¹² represents hydrogen. In another embodiment, X¹² represents fluoro.

Where R¹¹ represents heteroaryl, this group is suitably pyridinyl, pyrazinyl, furyl, thienyl, thiazolyl or triazolyl, especially pyridinyl.

Representative values of R¹¹ include phenyl, fluorophenyl, chlorophenyl, difluorophenyl, (chloro)(fluoro)phenyl, trifluorophenyl, (fluoro)(methyl)phenyl, (fluoro)(tritluoromethyl)phenyl, methoxyphenyl, (fluoro)(methoxy)phenyl, cyanophenyl, (cyano)(fluoro)phenyl, oxo-pyrrolidinyl, dihydropyrrolyl, pyridinyl, fluoro-pyridinyl, difluoro-pyridinyl, oxo-pyridinyl, pyrazinyl, furyl, thienyl, thiazolyl, triazolyl, methoxy, ethoxy, allyloxy, benzyloxy, triflyloxy, ethylthio, tert-butylthio, methylamino, ethylamino, propylamino, isopropylamino, allylamino, cyclopropylamino, cyclohexylamino, benzylamino, methoxybenzyl-amino and ethoxycarbonyl.

Particular values of R¹¹ include phenyl, fluorophenyl, chlorophenyl, difluorophenyl, (chloro)(fluoro)phenyl, (fluoro)(methyl)phenyl, (fluoro)(tritluoromethyl)phenyl, methoxyphenyl, (fluoro)(methoxy)phenyl, cyanophenyl, (cyano)(fluoro)phenyl, dihydropyrrolyl, pyridinyl, fluoro-pyridinyl, difluoro-pyridinyl, pyrazinyl, furyl, thienyl, thiazolyl, triazolyl, methoxy, ethoxy, allyloxy, benzyloxy, triflyloxy, ethylthio, tert-butylthio, methylamino, ethylamino, propylamino, isopropylamino, allylamino, cyclopropylamino, cyclohexylamino, benzylamino, methoxybenzyl-amino and ethoxycarbonyl.

Individual values of R¹¹ include phenyl, fluorophenyl, chlorophenyl, difluorophenyl, methoxyphenyl, cyanophenyl, (cyano)(fluoro)phenyl, dihydropyrrolyl, pyridinyl, fluoro-pyridinyl, pyrazinyl, furyl, thienyl, thiazolyl, triazolyl, methoxy, ethoxy, allyloxy, benzyloxy, methylamino, ethylamino, propylamino, isopropylamino, allylamino, cyclopropylamino, cyclohexylamino, benzylamino, methoxybenzyl-amino and ethoxycarbonyl.

In one embodiment, R¹¹ represents fluorophenyl (especially 2-fluorophenyl).

In another embodiment, R¹¹ represents fluoro-pyridinyl (especially 3-fluoropyridin-2-yl).

In a further embodiment, R¹¹ represents difluoro-pyridinyl. In one aspect of this embodiment, R¹¹ represents 3,5-difluoropyridin-4-yl. In another aspect of this embodiment, R¹¹ represents 3,5-diffuoropyridin-2-yl.

In an additional embodiment, R¹¹ represents trifluorophenyl (especially 2,4,6-trifluorophenyl).

One representative subset of the compounds of formula IIA above is represented by the compounds of formula IIB, and *N*-oxides thereof and pharmaceutically acceptable salts thereof: 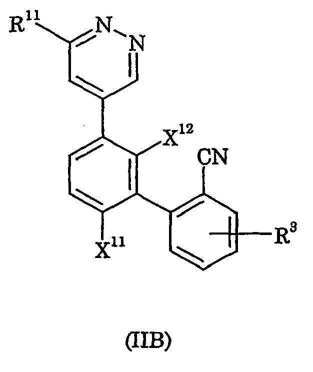 wherein X¹¹, X¹² and R¹¹ are as defined above; and
R³ represents hydrogen or fluoro.
In one embodiment, R³ is hydrogen.

In another embodiment, R³ is fluoro, in which case the fluorine atom R³ is favourably attached to the phenyl ring at the 4-, 5- or 6-position (relative to the cyano group at position 2).

Another representative subset of the compounds of formula IIA above is represented by the compounds of formula IIC, and N oxide thereof and pharmaceutically acceptable salts thereof: 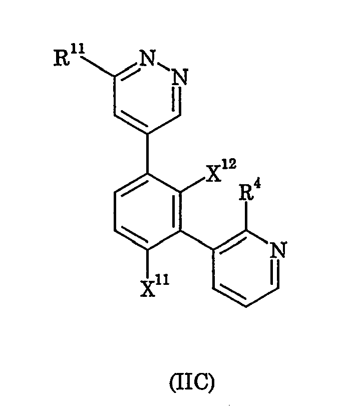 wherein X¹¹, X¹² and R¹¹ are as defined above; and
R⁴ represents hydrogen, fluoro, cyano or methyl.

In one embodiment, R⁴ is hydrogen.

In an additional embodiment, R⁴ is fluoro.

In another embodiment, R⁴ is cyano.

In a further embodiment, R⁴ is methyl.

A further representative subset of the compounds of formula IIA above is represented by the compounds of formula IID, and N-oxides thereof and pharmaceutically acceptable salts thereof: 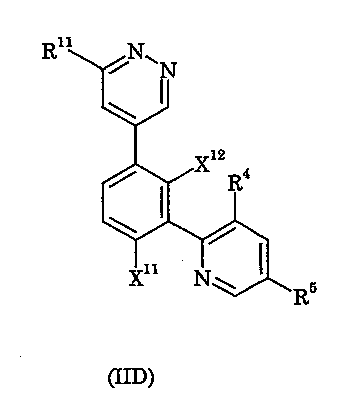 wherein X¹¹, X¹², R⁴ and R¹¹ are as defined above; and
R⁵ represents hydrogen or fluoro.

Suitably, R⁵ represents hydrogen.

In another embodiment, R⁵ represents fluoro.

A favoured subset of the compounds of formula IID above is represented by the compounds of formula IIE, and *N*-oxides thereof and pharmaceutically acceptable salts thereof: 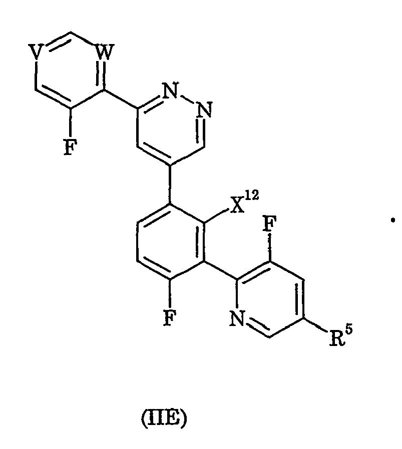 wherein
V represents N and W represents CF; or
V represents CF and W represents N; or
V and W both represent CF; and
X¹² and R⁵ are as defined above.

The present invention also provides a compound of formula IIE as depicted above, or an N-oxide thereof or a pharmaceutically acceptable salt thereof, wherein
V represents N and W represents CF; or
V represents CF and W represents N; and
X¹² and R⁵ are as defined above.

In one embodiment, V is N and W is CF.

In another embodiment, V is CF and W is N.

In a further embodiment, V and W are both CF.

Specific compounds within the scope of the present invention include:
5-[2-fluoro-3-(pyridin-3-yl)phenyl]3-phenylpyridazine;
4,2'-difluoro-5'-(6-phenylpyridazin-4yl)biphenyl-2-carbonitrile;
3-phenyl-5-[3-(pyridin-3-yl)phenyl]pyridazine;
3-phenyl-5-(3-[1,2,4]triazol-4-ylphenyl)pyridazine;
5-[2,4-difluoro-3-(pyridin-4-yl)phenyl]-3-phenylpyridazine;
5-[3-(2-methyl-2*H-*[1,2,4]triazol-3-ylmethoxy)phenyl]-3-phenylpyridazine;
6,2'-difluoro-5'-(6-phenylpyridazin-4-yl)biphenyl-2-carbonitrile;
5-[4-fluoro-3-(pyridin-4-yl)phenyl]-3-phenylpyridazine;
5-[4-fluoro-3-(3-fluoropyridin-2-yl)phenyl]-3-phenylpyridazine;
3-phenyl-5-[3-(pyridin-2-ylmethoxy)phenyl]pyridazine;
5-[4-fluoro-3-(3-fluoropyridin-4-yl)phenyl]-3-phenylpyridazine;
5-[2-fluoro-3-(pyridin-4-yl)phenyl]-3-phenylpyridazine;
5- [3-(3, 5-difluoropyridin- 2-yl)-4- fluorophenyl]-3-phenylpyridazine;
5-[4-fluoro-3-(pyridin-3-yl)phenyl]-3-phenylpyridazine;
5,2'-difluoro-5'-(6-phenylpyridazin-4-yl)biphenyl-2-carbonitrile;
3,2'-difluoro-5'-(6-phenylpyridazin-4-yl)biphenyl-2-carbonitrile;
6,2'-difluoro-5'-[6-(4-fluorophenyl)pyridazin-4-yl]biphenyl-2-carbonitrile;
4-fluoro-3'-(6-phenylpyridazin-4-yl)biphenyl-2-carbonitrile;
6,2'-difluoro-5'-[6-(thien-2-yl)pyridazin-4-yl]biphenyl-2-carbonitrile;
6,2'-difluoro-5'-[6-(4-methoxyphenyl)pyridazin-4-yl]biphenyl-2-carbonitrile;
5'-[6-(3-chlorophenyl)pyridazin-4-yl]-6,2'-difluorobiphenyl-2-carbonitrile;
6,2'-difluoro-5'- [6-(pyridin-3-yl)pyridazin-4-yl]biphenyl-2-carbonitrile;
5'-[6-(4-chlorophenyl)pyridazin-4-yl]-6,2'-difluorobiphenyl-2-carbonitrile;
6,2'-difluoro-5'-[6-(pyridin-4-yl)pyridazin-4-yl]biphenyl-2-carbonitrile;
5-[3-(3,5-difluoropyridin-2-yl)-4-fluorophenyl]-3-(4-fluorophenyl)-pyridazine;
5-[4-fluoro-3-(3-fluoropyridin-2-yl)phenyl]-3-(2-fluorophenyl)pyridazine;
5-[3-(3,5-difluoropyridin-2-yl)-4-fluorophenyl]-3-(2-fluorophenyl)-pyridazine;
5-[4-fluoro-3-(3-fluoropyridin-2-yl)phenyl]-3-(pyridin-3-yl)pyridazine;
5-[3-(3,5-difluoropyridin-2-yl)-4-fluorophenyl]-3-(3-fluorophenyl)-pyridazine;
3-(2,4-difluorophenyl)-5-[3-(3,5-difluoropyridin-2-yl)-4-fluorophenyl]-pyridazine;
5-[3-(3, 5-difluoropyridin-2-yl)-4-fluorophenyl]-3-(3-methoxyphenyl)-pyridazine;
6,2'-difluoro-5'-[6-(2-fluorophenyl)pyridazin-4-yl]biphenyl-2-carbonitrile;
6,2'-difluoro-5'-[6-(3-fluorophenyl)pyridazin-4-yl]biphenyl-2-carbonitrile;
5'-[6-(2-chlorophenyl)pyridazin-4-yl]-6,2'-difluorobiphenyl-2-carbonitrile;
4-fluoro-3'-[6-(4-methoxyphenyl)pyridazin-4-yl]biphenyl-2-carbonitrile;
5-[3-(3,5-difluoropyridin-2-yl)-4-fluorophenyl]-3-(4-methoxyphenyl)-pyridazine;
3-(4-chlorophenyl)-5-[3-(3,5-difluoropyridin-2-yl)-4-fluorophenyl]-pyridazine;
2-{5-[3-(3,5-difluoropyridin-2-yl)-4-fluorophenyl]pyridazin-3-yl}-5-fluorobenzonitrile;
3-(4-chlorophenyl)-5-[4-fluoro-3-(3-fluoropyridin-2-yl)phenyl]pyridazine;
5-[4-fluoro-3-(3-fluoropyridin-2-yl)phenyl]-3-(furan-3-yl)pyridazine;
5-[4-fluoro-3-(3-fluoropyridin-2-yl)phenyl]-3-(furan-2-yl)pyridazine;
3-(2,3-difluorophenyl)-5-[4-fluoro-3-(3-fluoropyridin-2-yl)phenyl]-pyridazine;
5-[4-fluoro-3-(3-fluoropyridin-2-yl)phenyl]-3-(thien-3-yl)pyridazine;
5-[4-fluoro-3-(3-fluoropyridin-2-yl)phenyl]-3-(thien-2-yl)pyridazine;
3-(2,5-difluorophenyl).5-[4-fluoro-3.(3.fluoropyridin.2-yl)phenyl]-pyridazine;
3-(3,4-difluorophenyl)-5-[4-fluoro-3-(3-fluoropyridin-2-yl)phenyl]-pyridazine;
4-{5-[4-fluoro-3-(3-fluoropyridin-2-yl)phenyl]pyridazin-3-yl}benzonitrile;
3'-(6-ethylaminopyridazin-4-yl)-4-fluorobiphenyl-2-carbonitrile;
4-fluoro-3'-(6-isopropylaminopyridazin-4-yl)biphenyl-2-carbonitrile;
4-fluoro-3'-(6-propylaminopyridazin-4-yl)biphenyl-2-carbonitrile;
3'-(6-cyclopropylaminopyridazin-4-yl)-4-fluorobiphenyl-2-carbonitrile;
3'-(6-allylaminopyridazin-4-yl)-4-fluorobiphenyl-2-carbonitrile;
3'-(6-benzylaminopyridazin-4-yl)-4-fluorobiphenyl-2-carbonitrile;
4-fluoro-3'-(6-methylaminopyridazin-4-yl)biphenyl-2-carbonitrile;
4-fluoro-3'-(6-methoxypyridazin-4-yl)biphenyl-2-carbonitrile;
3'-(6-ethoxypyridazin-4-yl)-4-fluorobiphenyl-2-carbonitrile;
3'-(6-benzyloxypyridazin-4-yl)-4-fluorobiphenyl-2-carbonitrile;
5-[4-fluoro-3-(2-methyl-2*H*-[1,2,4]triazol-3-ylmethoxy)phenyl]-3-phenylpyridazine;
5- [4-fluoro-3-(1-methyl-3-trifluoromethyl-1*H*-pyrazol-4-ylmethoxy)phenyl]-3-phenylpyridazine;
5-[4-fluoro-3-(pyridin-4-ylmethoxy)phenyl]-3-phenylpyridazine;
5-[4-fluoro-3-(pyridin-3-ylmethoxy)phenyl]-3-phenylpyridazine;
5-[4-fluoro-3-(3-fluoropyridin-2-yl)phenyl]-3-(pyridin-4-yl)pyridazine;
5-[4-fluoro-3-(3-ffuoropyridin-2-yl)phenyl]-3-(pyrazin-2-yl)pyridazine;
5-[4-fluoro-3-(3-fluoropyridin-2-yl)phenyl]-3-(thiazol-2-yl)pyridazine;
5-[4-fluoro-3-(3-fluoropyridin-2-yl)phenyl]-3-(pyridin-2-yl)pyridazine;
5-[4-fluoro-3-(3-fluoropyridin-2-yl)phenyl]-3-(3-fluoropyridin-4-yl)pyridazine;
5-[4-fluoro-3-(3-fluoropyridin-2-yl)phenyl]-3-(1*H*-[1,2,3]triazol-4-yl)pyridazine;
5-[4-fluoro-3-(3-fluoropyridin-2-yl)phenyl]pyridazine-3-carboxylic acid ethyl ester;
5-[3-(3,5-difluoropyridin-2-yl)-4-fluorophenyl]-3-(2-fluorophenyl)-pyridazine-1-oxide;
3-(2,6-difluorophenyl)-5-[4-fluoro-3-(3-fluoropyridin-2-yl)phenyl]-pyridazine;
3-(4-chloro-2-fluorophenyl)-5-[3-(3,5-difluoropyridin-2-yl)-4-fluorophenyl]pyridazine;
5-[3-(3,5-difluoropyridin-2-yl)-4-fluorophenyl]-3-(2-fluoro-4-trifluoromethylphenyl)pyridazine;
5-[3-(3,5-difluoropyridin-2-yl)-4-fluorophenyl]-3-(2-fluoro-4-methylphenyl)-pyridazine;
3-(3,5-difluoropyridin-2-yl)-5-[3-(3,5-difluoropyridin-2-yl)-4-fluorophenyl]pyridazine;
trifluoromethanesulfonic acid 5-[4-fluoro-3-(3-fluoropyridin-2-yl)phenyl]pyridazin-3-yl ester;
3-ethylsulfanyl-5-[4-fluoro-3-(3-ffuoropyridin-2-yl)phenyl]pyridazine;
3-tert-butylsulfanyl-5-[4-fluoro-3-(3-fluoropyridin-2-yl)phenyl]pyridazine;
5-[3-(3,5-difluoropyridin-2-yl)-4-fluorophenyl]-3-(3-fluoropyridin-4-yl)-pyridazine;
5-[3-(3,5-difluoropyridin-2-yl)-4-fluorophenyl]-3-(3-fluoropyridin-2-yl)-pyridazine;
5-[4-fluoro-3-(3-fluoropyridin-2-yl)phenyl]-3-(3-fluoropyridin-2-yl)-pyridazine;
5-[4-fluoro-3-(3-ffuoropyridin-2-yl)phenyl]-3-(3-tluoropyridin-4-yl)-pyridazine 1-oxide;
5-[4-fluoro-3-(3-fluoropyridin-2-yl)phenyl]-3-(3-fluoro-1-oxypyridin-4-yl)-pyridazine;
5-[2,4-difluoro-3-(3,5-difluoropyridin-2-yl)phenyl]-3-(3,5-difluoropyridin-4-yl)pyridazine;
5-[3-(3,5-diffuoropyridin-2-yl)-4-fluorophenyl]-3-(2-fluoro-4-methoxyphenyl)pyridazine;
5-[4-fluoro-3-(3-fluoropyridin-2-yl)phenyl]-3-(2-fluoro-4-methoxyphenyl)-pyridazine;
3-(3,5-difluoropyridin-4-yl)-5-[4-fluoro-3-(3-fluoropyridin-2-yl)phenyl]pyridazine;
3-(3,5-difluoropyridin-2-yl)-5-[4-fluoro-3-(3-fluoropyridin-2-yl)phenyl]pyridazine;
3-(3,5-difluoropyridin-4-yl)-5-[3-(3,5-difluoropyridin-2-yl)-4-fluorophenyl]pyridazine;
3-(3,5-difluoro-1-oxypyridin-4-yl)-5-[3-(3,5-difluoropyridin-2-yl)-4-fluorophenyl]pyridazine;
5'-[6-(3,5-difluoropyridin-2-yl)pyridazin-4-yl]-2'-fluorobiphenyl-2-carbonitrile;
5'-[6-(3,5-difluoropyridin-4-yl)pyridazin-4-yl]-2'-fluorobiphenyl-2-carbonitrile;
4,2'-difluoro-5'-[6-(3,5-difluoropyridin-4-yl)pyridazin-4-yl]biphenyl-2-carbonitrile;
4,2'-difluoro-5'-[6-(3,5-difluoropyridin-2-yl)pyridazin-4-yl]biphenyl-2-carbonitrile;
2-{5-[6-(3,5-difluoropyridin-4-yl)pyridazin-4-yl]-2-fluorophenyl}-nicotinonitrile;
2-{5-[6-(3,5-difluoropyridin-2-yl)pyridazin-4-yl]-2-fluorophenyl}-nicotinonitrile;
2'-fluoro-5'-[6-(2-oxopyrrolidin-1-yl)pyridazin-4-yl]biphenyl-2-carbonitrile;
2'-fluoro-5'-[6-(2-oxo-2*H*-pyridin-1-yl)pyridazin-4-yl]biphenyl-2-carbonitrile;
6,2'-difluoro-5'-[6-(3,5-difluoropyridin-2-yl)pyridazin-4-yl]biphenyl-2-carbonitrile;
3-(3,5-difluoropyridin-2-yl)-5-(4-fluoro-3-trifluoromethylphenyl)pyridazine;
3-(3,5-difluoropyridin-2-yl)-5-(6-fluoro-2'-trifluoromethylbiphenyl-3-yl)-pyridazine;
5-(6,2'-difluorobiphenyl-3-yl)-3-(3,5-difluoropyridin-2-yl)pyridazine;
3-(3,5-difluoropyridin-2-yl)-5-(6,2',4'-trifluorobiphenyl-3-yl)pyridazine;
5-[3-(3,5-difluoropyridin-2-yl)-4-fluorophenyl]-3-(2,4,6-trifluorophenyl)-pyridazine;
and pharmaceutically acceptable salts thereof.

The binding affinity (Kᵢ) of the compounds according to the present invention for the α3 subunit of the human GABA_{A} receptor is conveniently as measured in the assay described hereinbelow. The α3 subunit binding affinity (Kᵢ) of the anxiolytic compounds of the invention is ideally 50 nM or less, preferably 10 nM or less, and more preferably 5 nM or less.

The anxiolytic compounds according to the present invention will ideally elicit at least a 40%, preferably at least a 50%, and more preferably at least a 60%, potentiation of the GABA EC₂₀ response in stably transfected recombinant cell lines expressing the α3 subunit of the human GABA_{A} receptor. Moreover, the compounds of the invention will ideally elicit at most a 30%, preferably at most a 20%, and more preferably at most a 10%, potentiation of the GABA EC₂₀ response in stably transfected recombinant cell lines expressing the α1 subunit of the human GABA_{A} receptor.

The potentiation of the GABA EC₂₀ response in stably transfected cell lines expressing the α3 and α1 subunits of the human GABA_{A} receptor can conveniently be measured by procedures analogous to the protocol described in Wafford et al., Mol. Pharmacol., 1996, 50, 670-678. The procedure will suitably be carried out utilising cultures of stably transfected eukaryotic cells, typically of stably transfected mouse Ltk-fibroblast cells.

The compounds according to the present invention may exhibit anxiolytic activity, as may be demonstrated by a positive response in the elevated plus maze and conditioned suppression of drinking tests (cf. Dawson et al., Psychopharmacology, 1995, 121, 109-117). Moreover, the compounds of the invention are likely to be substantially non-sedating, as may be confirmed by an appropriate result obtained from the response sensitivity (chain-pulling) test (cf. Bayley et al., J. Psychopharmacol., 1996, 10, 206-213).

The compounds according to the present invention may also exhibit anticonvulsant activity. This can be demonstrated by the ability to block pentylenetetrazole-induced seizures in rats and mice, following a protocol analogous to that described by Bristol et al. in J. Pharmacol. Exp. Ther., 1996, 279, 492-501.

Cognition enhancement can be shown by testing the compounds in the Morris watermaze as reported by McNamara and Skelton, Psychobiology, 1993, 21, 101-108. Further details of relevant methodology are described in WO 96/25948.

Cognitive disorders for which the compounds of the present invention may be of benefit include delirium, dementia, amnestic disorders, and cognition deficits, including age-related memory deficits, due to traumatic injury, stroke, Parkinson's disease and Down Syndrome. Any of these conditions may be attributable to substance abuse or withdrawal. Examples of dementia include dementia of the Alzheimer's type with early or late onset, and vascular dementia, any of which may be uncomplicated or accompanied by delirium, delusions or depressed mood; and dementia due to HIV disease, head trauma, Parkinson's disease or Creutzfeld-Jakob disease.

In order to elicit their behavioural effects, the compounds of the invention will ideally be brain-penetrant; in other words, these compounds will be capable of crossing the so-called "blood-brain barrier". Preferably, the compounds of the invention will be capable of exerting their beneficial therapeutic action following administration by the oral route.

The invention also provides pharmaceutical compositions comprising one or more compounds of this invention in association with a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

In the treatment of neurological disorders, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds in accordance with the present invention may be prepared by a process which comprises reacting a compound of formula III with a compound of formula IV: 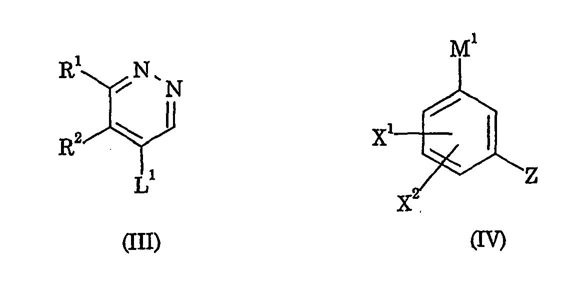 wherein X¹, X², Z, R¹ and R² are as defined above, L¹ represents a suitable leaving group, and M¹ represents a boronic acid moiety -B(OH)₂ or a cyclic ester thereof formed with an organic diol, e.g. pinacol, 1,3-propanediol or neopentyl glycol, or M¹ represents -Sn(Alk)₃ in which Alk represents a C₁₋₆ alkyl group, typically *n*-butyl or methyl, or M¹ represents -ZnHal in which Hal represents a halogen atom, e.g. chloro; in the presence of a transition metal catalyst.

The leaving group L¹ is typically a halogen atom, e.g. iodo, bromo or chloro.

The transition metal catalyst of use in the reaction between compounds III and IV is suitably tetrakis(triphenylphosphine)-palladium(0). The reaction is conveniently carried out at an elevated temperature in a solvent such as *N,N-*dimethylacetamide, 1,4-dioxane or tetrahydrofuran, advantageously in the presence of potassium phosphate, copper(I) iodide, sodium carbonate or cesium carbonate. Alternatively, the transition metal catalyst employed may be dichloro[1,1'-bis(diphenyl-phosphino)ferrocene]palladium(II), in which case the reaction is conveniently effected at an elevated temperature in a solvent such as *N,N-*dimethylformamide, advantageously in the presence of potassium phosphate.

In an alternative procedure, the compounds according to the present invention may be prepared by a process which comprises reacting a compound of formula V with a compound of formula VI: 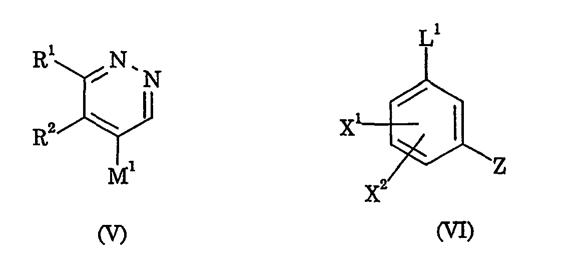 wherein X¹, X², Z, R¹, R², L¹ and M¹ are as defined above; in the presence of a transition metal catalyst; under conditions analogous to those described above for the reaction between compounds III and IV.

In another procedure, the compounds according to the present invention may be prepared by a process which comprises reacting a compound of formula VII with a compound of formula VIII: 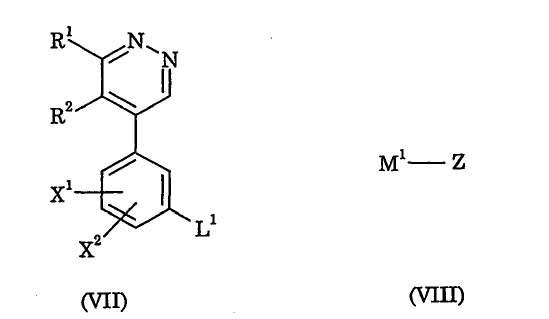 wherein X¹, X², Z, R¹, R², L¹ and M¹ are as defined above; in the presence of a transition metal catalyst; under conditions analogous to those described above for the reaction between compounds III and IV.

In the compounds of formula VI and VII above, the leaving group L¹ is typically trifluoromethanesulfonyloxy (triflyloxy); or a halogen atom, e.g. bromo.

Alternatively, the compounds according to the present invention may be prepared by a process which comprises reacting a compound of formula IX with a compound of formula X: 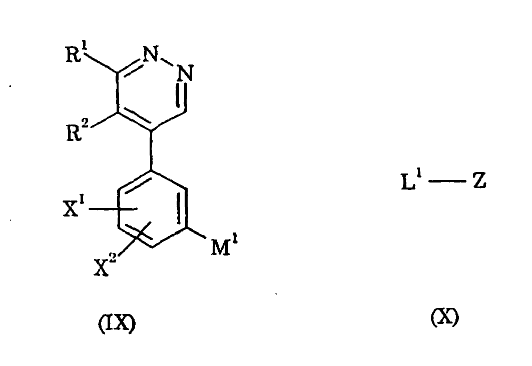 wherein X¹, X², Z, R¹, R², L¹ and M¹ are as defined above; in the presence of a transition metal catalyst; under conditions analogous to those described above for the reaction between compounds III and IV.

Where M¹ in the intermediates of formula IV and IX above represents a boronic acid moiety -B(OH)₂ or a cyclic ester thereof formed with pinacol or neopentyl glycol, the relevant compound IV or IX may be prepared by reacting bis(pinacolato)diboron or bis(neopentyl glycolato)diborane respectively with a compound of formula VI or VII as defined above; in the presence of a transition metal catalyst.

The transition metal catalyst of use in the reaction between bis(pinacolato)diboron or bis(neopentyl glycolato)diborane and compound VI or VII is suitably dichloro[1,1'-bis(diphenylphosphino)ferrocene]-palladium(II). The reaction is conveniently carried out at an elevated temperature in a solvent such as 1,4-dioxane, optionally in admixture with dimethylsulfoxide, typically in the presence of 1,1'-bis(diphenylphosphino)ferrocene and/or potassium acetate.

Where L¹ in the intermediates of formula VII above represents triflyloxy, the relevant compound VII may be prepared by reacting the appropriate compound of formula XI: 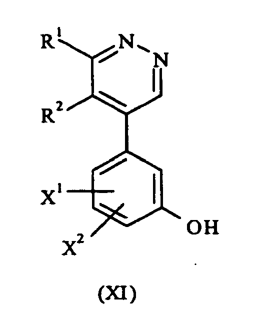 with triflic anhydride, typically in the presence of pyridine. Analogous conditions may be utilised for preparing a compound of formula VI wherein L¹ represents triflyloxy from the corresponding hydroxy precursor.

The intermediates of formula XI above may suitably be prepared from the appropriate methoxy-substituted precursor of formula XIII: 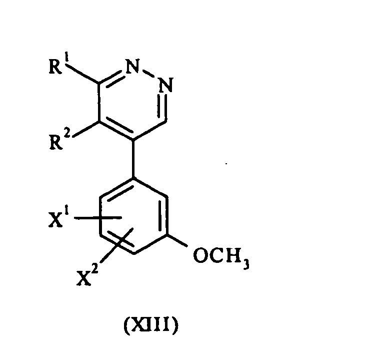 wherein X¹, X², R¹ and R² are as defined above; by treatment with boron tribromide, typically in chloroform or dichloromethane; or with hydrogen bromide, typically in acetic acid at reflux.

Where M¹ in the intermediates of formula V above represents -Sn(Alk)₃ and Alk is as defined above, this compound may be prepared by reacting a compound of formula III as defined above with a reagent of formula (Alk)₃Sn-Hal, in which Hal represents a halogen atom, typically chloro. The reaction is conveniently effected by treating compound III with isopropylmagnesium chloride, typically in a solvent such as tetrahydrofuran, with subsequent addition of the stannyl reagent (Alk)₃Sn-Hal.

In a further procedure, the compounds according to the present invention wherein R¹ represents an aryl or heteroaryl moiety may be prepared by a process which comprises reacting a compound of formula XIV with a compound of formula XV: 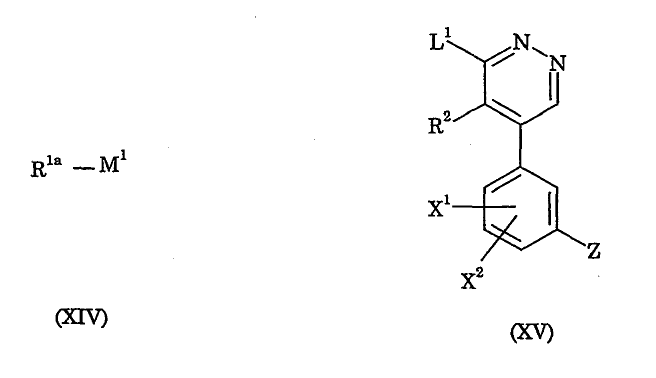 wherein X¹, X², Z, R², L¹ and M¹ are as defined above, and R^{1a} represents an aryl or heteroaryl moiety; in the presence of a transition metal catalyst; under conditions analogous to those described above for the reaction between compounds III and IV.

In the compounds of formula XV above, the leaving group L¹ is typically triflyloxy; or a halogen atom, e.g. chloro.

The transition metal catalyst of use in the reaction between compounds XIV and XV is suitably tetrakis(triphenylphosphine)-palladium(O), in which case the reaction is conveniently effected at an elevated temperature in a solvent such as tetrahydrofuran, 1,4-dioxane or *N,N-*dimethylformamide, typically in the presence of sodium carbonate. Alternatively, the transition metal catalyst may suitably be palladium bis(diphenylphosphinylbutane)dichloride, in which case the reaction is conveniently effected at an elevated temperature in a solvent such as tetrahydrofuran, typically in the presence of sodium carbonate.

In a still further procedure, the compounds according to the present invention wherein R¹ represents an aryl or heteroaryl moiety may be prepared by a process which comprises reacting a compound of formula XVI with a compound of formula XVII: 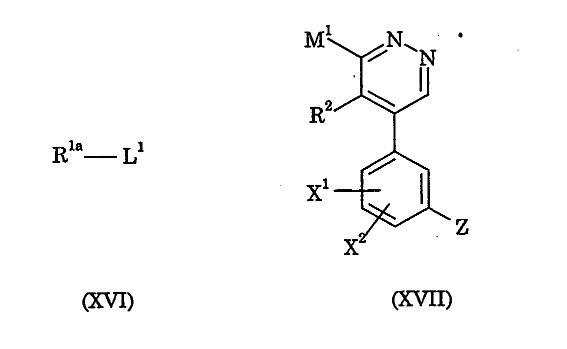 wherein X¹, X², Z, R^{1a}, R², L¹ and M¹ are as defined above; in the presence of a transition metal catalyst; under conditions analogous to those described above for the reaction between compounds III and IV.

The intermediates of formula XVII wherein M¹ represents -Sn(Alk)3 and Alk represents C₁₋₆ alkyl, e.g. methyl, may be prepared by reacting a compound of formula XV as defined above with a reagent of formula (Alk)₃Sn-Sn(Alk)₃. The reaction is conveniently effected in the presence of a transition metal catalyst, e.g. tetrakis(triphenylphosphine)palladium(O), with heating in a solvent such as 1,4-dioxane, typically in the presence of lithium chloride.

In a yet further procedure, the compounds according to the present invention wherein R¹ represents 1*H*-[1,2,3]triazol-4-yl may be prepared by a process which comprises reacting a compound of formula XVIII: 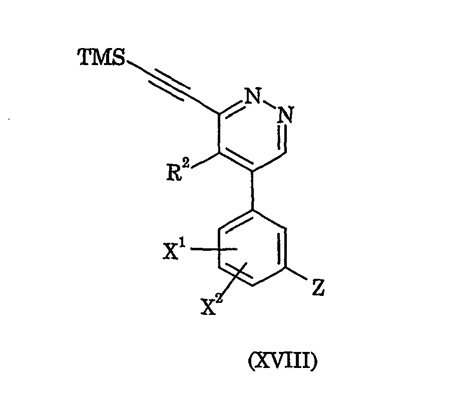 wherein X¹, X², Z and R² are as defined above, and TMS is an abbreviation for trimethylsilanyl; with sodium azide.

The reaction is conveniently effected by stirring the reactants in a solvent such as *N,N-*dimethylformamide.

The intermediates of formula XVIII may be prepared by reacting a compound of formula XV with TMS-acetylene, in the presence of a transition metal catalyst such as bis(triphenylphosphine)palladium(II) chloride. The reaction is conveniently effected by stirring in a solvent such as tetrahydrofuran, typically in the presence of triethylamine, triphenylphosphine and copper(I) chloride.

The compounds according to the present invention wherein R¹ represents -OR^{a} may be prepared by a process which comprises reacting a compound of formula XV as defined above with a compound of formula R^{a}-OH, wherein R^{a} is as defined above. The reaction is conveniently carried out in the presence of a base such as sodium hydride or sodium ethoxide.

The compounds according to the present invention wherein R¹ represents -SR^{a} may be prepared by a process which comprises reacting a compound of formula XV as defined above with a salt of formula R^{a}S-Na⁺, wherein R^{a} is as defined above. The reaction is conveniently carried out in the presence of a solvent such as *N,N*-dimethylformamide.

The compounds according to the present invention wherein R¹ represents -NR^{a}R^{b} may be prepared by a process which comprises reacting a compound of formula XV as defined above with a compound of formula H-NR^{a}R^{b}, wherein R^{a} and R^{b} are as defined above. The reaction is conveniently effected by stirring at an elevated temperature, typically in a solvent such as tetrahydrofuran.

The compounds according to the present invention wherein R¹ represents -CO₂R^{a} may be prepared by a process which comprises reacting a compound of formula XV as defined above with carbon monoxide and a compound of formula R^{a}-OH, wherein R^{a} is as defined above; in the presence of a transition metal catalyst.

The transition metal catalyst of use in the foregoing reaction is ideally [1,1'-bis(diphenylphosphino)ferrocene]palladium(II) chloride, in which case the reaction is conveniently carried out at an elevated temperature in a solvent such as *N,N-*dimethylformamide, optionally in admixture with dichloromethane, typically in the presence of sodium acetate.

The intermediates of formula XV wherein L¹ represents triflyloxy may be prepared by reacting a compound of formula XIX: 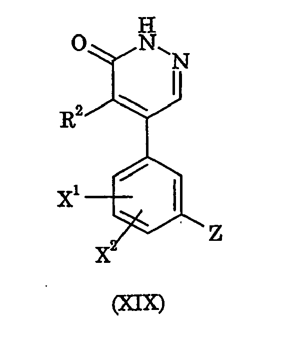 wherein X¹, X², Z and R² are as defined above; with *N-*phenyltriflylimide, typically in the presence of triethylamine, in a solvent such as dichloromethane; or with triflic anhydride, typically in the presence of pyridine.

Moreover, the intermediates of formula XV wherein L¹ represents chloro may be prepared by treating the requisite compound of formula XIX with phosphorus oxychloride at an elevated temperature.

The intermediates of formula XIX may be prepared by reacting a compound of formula IV as defined above with a compound of formula XX: 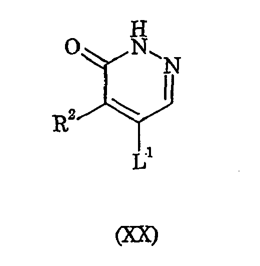 wherein R² and L¹ are as defined above; in the presence of a transition metal catalyst; under conditions analogous to those described above for the reaction between compounds III and IV.

Alternatively, the intermediates of formula XIX may be prepared by reacting a compound of formula XXI: 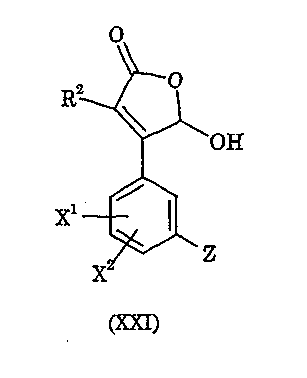 wherein X¹, X², Z and R² are as defined above; with hydrazine hydrate, typically in ethanol at reflux.

The compounds according to the present invention wherein R² represents methoxycarbonyl may be prepared by a process which comprises reacting a compound of formula XXII: 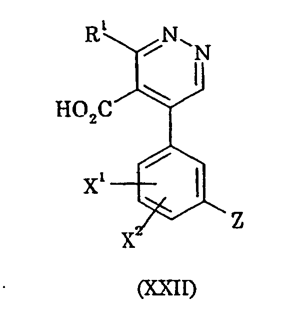 wherein X¹, X², Z and R¹ are as defined above; with diazomethane.

The reaction is conveniently accomplished by stirring in a solvent such as diethyl ether.

The intermediates of formula XXII may be prepared by saponifying a compound of formula I wherein R² represents C₂₋₆ alkoxycarbonyl, typically by treatment with potassium hydroxide in refluxing aqueous methanol.

The compounds according to the present invention wherein R² represents C₂₋₆ alkoxycarbonyl may be prepared by a process which comprises reacting a compound of formula XXIII: 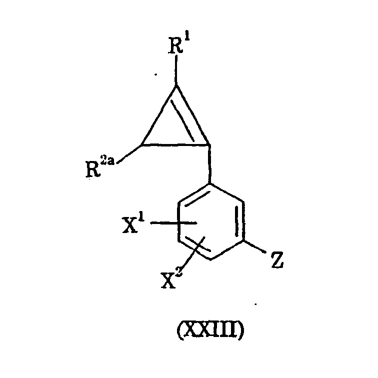 wherein X¹, X², Z and R¹ are as defined above, and R^{2a} represents C₂₋₆ alkoxycarbonyl; with diazomethane.

The reaction is conveniently effected by stirring in a solvent such as diethyl ether.

The compounds of formula XI and XIII may therefore be prepared by any of the methods described above for the preparation of the compounds according to the invention.

Where they are not commercially available, the starting materials of formula III, VIII, X, XIV, XVI, XX, XXI and XXIII may be prepared by methods analogous to those described in the accompanying Examples, or by standard methods well known from the art.

It will be understood that any compound of formula I initially obtained from any of the above processes may, where appropriate, subsequently be elaborated into a further compound of formula I by techniques known from the art. For example, a compound of formula I initially obtained may be converted into the *N-*oxide derivative thereof by treatment with m-chloroperbenzoic acid.

A compound of formula I wherein R¹ represents C₂₋₆ alkoxycarbonyl may be reduced with lithium aluminium hydride to the corresponding compound of formula I wherein R¹ represents hydroxymethyl. A compound wherein R¹ represents formyl may be reacted with a Grignard reagent of formula R^{a}MgBr to afford a compound of formula I wherein R¹ represents -CH(OH)R^{a}. A compound of formula I wherein R¹ represents -CH(OH)R^{a} may be converted into the corresponding compound of formula I wherein R¹ represents -CHFR^{a} by treatment with (diethylamino)sulfur trifluoride (DAST). Similarly, a compound wherein R¹ represents -COR^{a} may be converted into the corresponding compound of formula I wherein R¹ represents -CF₂R^{a} by treatment with DAST. A compound wherein R¹ represents -COCH₃ may be treated with thioacetamide in the presence of pyridinium tribromide to furnish the corresponding compound of formula I wherein R¹ represents 2-methylthiazol-5-yl. Moreover, a compound wherein R¹ is formyl may be treated with (p-tolylsulfonyl)methyl isocyanide (TosMIC) in the presence of potassium carbonate to afford the corresponding compound of formula I wherein R¹ represents oxazol-5-yl. A compound of formula I wherein R¹ represents hydroxymethyl may be treated with carbon tetrabromide and triphenylphosphine to afford the corresponding compound of formula I wherein R¹ represents bromomethyl. A compound wherein R¹ represents chloro may be converted into the corresponding compound wherein R¹ represents 2-oxopyrrolidin-1-yl by treatment with pyrrolidin-2-one in the presence of sodium hydride. A compound wherein R¹ represents chloro may be converted into the corresponding compound wherein R¹ represents 2-oxopyridin-1-yl by treatment with 2-hydroxypyridine in the presence of copper(I) iodide. A compound of formula I wherein Z is substituted with methoxy may be converted to the corresponding compound wherein Z is substituted with hydroxy by treatment with boron tribromide.

Where a mixture of products is obtained from any of the processes described above for the preparation of compounds according to the invention, the desired product can be separated therefrom at an appropriate stage by conventional methods such as preparative HPLC; or column chromatography utilising, for example, silica and/or alumina in conjunction with an appropriate solvent system.

Where the above-described processes for the preparation of the compounds according to the invention give rise to mixtures of stereoisomers, these isomers may be separated by conventional techniques such as preparative chromatography. The novel compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as (-)-di-p-toluoyl-d-tartaric acid and/or (+)-di-*p*-toluoyl-1-tartaric acid, followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 3rd edition, 1999. The protecting groups may be removed at a convenient subsequent stage using methods known from the art.

The following Examples illustrate the preparation of compounds according to the invention.

The compounds in accordance with this invention potently inhibit the binding of [³H]-flumazenil to the benzodiazepine binding site of human GABA_{A} receptors containing the α2 and/or α3 and/or α5 subunit stably expressed in Ltk- cells.

### Reagents

- Phosphate buffered saline (PBS).
- Assay buffer: 10 mM KH₂PO₄, 100 mM KCl, pH 7.4 at room temperature.
- [³H]-Flumazenil (18 nM for α1β3γ2 cells; 18 nM for α2β3γ2 cells; 10 nM for α3β3γ2 cells; 10 nM for α5βp3γ2 cells) in assay buffer.
- Flunitrazepam 100 µM in assay buffer.
- Cells resuspended in assay buffer (1 tray to 10 ml).

### Harvesting Cells

Supernatant is removed from cells. PBS (approximately 20 ml) is added. The cells are scraped and placed in a 50 ml centrifuge tube. The procedure is repeated with a further 10 ml of PBS to ensure that most of the cells are removed. The cells are pelleted by centrifuging for 20 min at 3000 rpm in a benchtop centrifuge, and then frozen if desired. The pellets are resuspended in 10 ml of buffer per tray (25 cm x 25 cm) of cells.

### Assay

Can be carried out in deep 96-well plates or in tubes. Each tube contains:
- 300 µl of assay buffer.
- 50 µl of [³H]-flumazenil (final concentration for α1β3γ2: 1.8 nM; for α2β3γ2: 1.8 nM; for α3β3γ2: 1.0 nM; for α5β3γ2: 1.0 nM).
- 50 µl of buffer or solvent carrier (e.g. 10% DMSO) if compounds are dissolved in 10% DMSO (total); test compound or flunitrazepam (to determine non-specific binding), 10 µM final concentration.
- 100 µl of cells.

Assays are incubated for 1 hour at 40°C, then filtered using either a Tomtec or Brandel cell harvester onto GF/B filters followed by 3 x 3 ml washes with ice cold assay buffer. Filters are dried and counted by liquid scintillation counting. Expected values for total binding are 3000-4000 dpm for total counts and less than 200 dpm for non-specific binding if using liquid scintillation counting, or 1500-2000 dpm for total counts and less than 200 dpm for non-specific binding if counting with meltilex solid scintillant. Binding parameters are determined by non-linear least squares regression analysis, from which the inhibition constant Kᵢ can be calculated for each test compound.

The compounds of the accompanying Examples were tested in the above assay, and all were found to possess a Kᵢ value for displacement of [³H]-flumazenil from the α2 and/or α3 and/or α5 subunit of the human GABA_{A} receptor of 100 nM or less.

### Reference EXAMPLE 1

### 3,5-Diphenylpyridazine

3-Phenyl-5-(tri-*n*-butylstannyl)pyridazine (100 mg, 0.22 mmol), bromobenzene (32 µl, 0.27 mmol) and tetrakis(triphenylphosphine)-palladium(0) (5 mg) in tetrahydrofuran, (2 ml) were combined and heated to 150°C for 600 s in a Smith Synthesiser microwave reactor (Personal Chemistry, Uppsala, Sweden). The reaction was diluted with CH₂Cl₂ (6 ml) and H₂O (2 ml) then poured into a PTFE (5 µM) fritted syringe barrel. The organic phase was collected and concentrated to leave 100 mg of crude product. Part of the sample was purified by HPLC with mass triggered fraction collection to give the *title compound* as a gum (3.1 mg). δ_{H} (400 MHz, d⁶ DMSO) 7.55-7.64 (6H, m), 8.06 (2H, dd, *J* 8.0, 1.6), 8.28 (2H, dd, *J* 8.0, 1.6), 8.46-8.47 (1H, d, *J* 4.0), 9.62 (1H, d, *J* 4.0); *m*/*z* (ES⁺) 233 (MH⁺).

### EXAMPLE 1

### 5-[2-Fluoro-3-(pyridin-3-yl)phenyl]3-phenylpyridazine

5-Iodo-3-phenylpyridazine (60 mg, 0.14 mmol), 2-fluoro-3-(pyridin-3-yl)phenylboronic acid (32 mg), tetrakis(triphenylphosphine)palladium(O) (5 mg) and 2N Na₂CO₃ (1 ml) in tetrahydrofuran (4 ml) were combined and heated to 150°C for 600 s in a Smith Synthesiser microwave reactor. The reaction was diluted with CH₂Cl₂ (6 ml) and H₂O (2 ml) then poured into a PTFE (5 µM) fritted syringe barrel. The organic phase was collected, concentrated and recrystallised from methanol-CH₂Cl₂-isopropanol to give the title compound as a colourless solid (36 mg). δ_{H} (400 MHz, d⁶ DMSO) 7.55-7.66 (5H, m), 7.77-7.81 (1H, m), 7.91-7.95 (1H, m), 8.08-8.11 (1H, m), 8.25-8.27 (2H, m), 8.48 (1H, m), 8.66 (1H, dd, *J* 1.6, 4.7), 8.87 (1H, d, *J* 2.0), 9.53 (1H, t, *J* 2.2); *m*/*z* (ES⁺) 328 (MH⁺).

### EXAMPLES 2 TO 16

The compounds in Table 1 were prepared, using the aryl halide, boronic acid or ester starting materials shown, in an analogous manner to that described in Reference Example 1 or Example 1. Samples were purified as described in Example 3 or by recrystallisation from methanol-ethyl acetate, CH₂Cl₂ or CH₂Cl₂-isopropanol.

**Table 1**

| **EX. NO.** | **STARTING MATERIAL** | **PRODUCT** | δ_{H} (400 MHz, d⁶ DMSO) | *m*/*z* (ES⁺) |
|---|---|---|---|---|
| 2 | 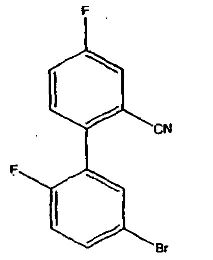 | 4,2'-Difluoro-5'-(6-phenyl-pyridazin-4-yl)biphenyl-2-carbonitrile | 7:56-7.67 (4H, m), 7.79-7.86 (2H, m), 8.05-8.08 (1H, m), 8.26-8.32 (4H, m), 8.55 (1H, d, *J* 4), 9.69 (1H, d, *J* 4) | 370 |
| 3 | 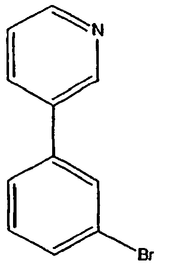 | 3-Phenyl-5-[3-(pyridin-3-yl)-phenyl]-pyridazine | 7.48-7.66 (4H, m), 7.73 (1H, t, *J* 7.8), 7.92-7.94 (1H, m), 8.12-8.13 (1H, m), 8.26-8.33 (3H, m), 8.41 (1H, t, *J* 1.8), 8.62-8.64 (2H, m), 9.10 (1H, dd, *J* 2.0, 0.8), 9.75 (1H, d, *J* 2.3). | 310 |
| 4 | 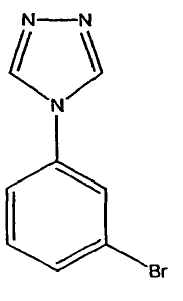 | 3-Phenyl-5-(3-[1,2,4]triazol-4-ylphenyl)pyrid-azine | 7.56-7.66 (4H, m), 7.79 (1H, t, *J* 7.8), 7.90-7.93 (1H, m), 8.14-8.16 (1H, m), 8.29-8.31 (1H, dd, *J* 0.8, 0.8), 8.42 (1H, t, *J* 2.0), 8.61 (1H, d, *J* 2.0), 9.29 (2H, s), 9.76 (1H, d, *J* 2.3). | 300 |
| 5 | 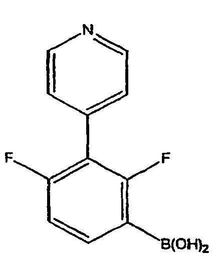 | 5-[2,4-Difluoro-3-(pyridin-4-yl)-phenyl]-3-phenyl-pyridazine | 7.53-7.64 (6H, m), 8.04-8.10 (1H, m), 8.26 (2H, dd, *J* 1.8, 8.0), 8.46 (1H, d, *J* 0.8), 8.77 (2H, dd, *J* 1.4, 4.5), 9.50 (1H, t, *J* 1.8). | 346 |
| 6 | 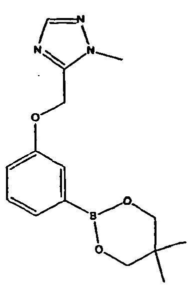 | 5-[3-(2-Methyl-2H-[1,2,4]triazol-3-ylmethoxy)-phenyl]-3-phenyl-pyridazine | 3.94 (3H, s), 5.46 (2H, s), 7.25 (1H, dd, *J* 2.0, 7.6), 7.52-7.62 (4H, m), 7.70 (1H, d, *J* 8.4), 7.79-7.80 (1H, m), 7.96 (1H, s), 8.28-8.30 (2H, m), 8.48 (1H, d, *J* 4.0), 9.63 (1H, s). | 344 |
| 7 | 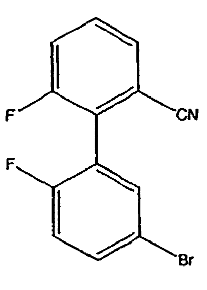 | 6,2'-Difluoro-5'-(6-phenyl-pyridazin-4-yl)-biphenyl-2-carbonitrile | 7.54-7.87 (6H, m), 7.98 (1H, dd, *J* 1.2, 7.4), 8.28-8.31 (2H, m), 8.34-8.38 (1H, m), 8.48 (1H, dd, *J* 2.5, 6.8), 8.55 (1H, d, *J* 2.0), 9.69 (1H, d, *J* 2.0). | 312 |
| 8 | 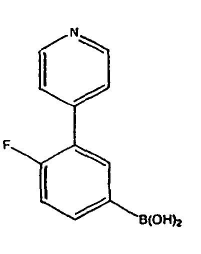 | 5-[4-Fluoro-3-(pyridin-4-yl)-phenyl]-3-phenyl-pyridazine | 7.54-7.68 (3H, m), 7.75-7.77 (2H, m), 8.21-8.25 (1H, m), 8.29-8.36 (3H, m), 8.60 (1H, d, *J* 2.3), 8.73-8.74 (2H, m), 9.73 (1H, d, *J* 2.3). | 328 |
| 9 | 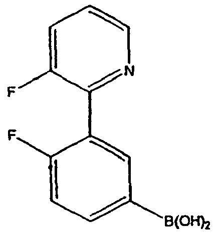 | 5-[4-Fluoro-3-(3-fluoro-pyridin-2-yl)-phenyl]-3-phenyl-pyridazine | 7.53-7.68 (5H, m), 7.92-7.97 (1H, m), 8.26-8.35 (4H, m), 8.55 (1H, d, *J* 2.3), 8.63-8.65 (1H, m), 9.67 (1H, d, *J* 2.3). | 346 |
| 10 | 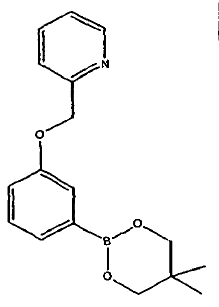 | 3-Phenyl-5-[3-(pyridin-2-ylmethoxy)-phenyl]-pyridazine | 5.33 (2H, s), 7.21-7.24 (1H, m), 7.30-7.40 (1H, m), 7.50-7.67 (6H, m), 7.76 (1H, t, *J* 2.0), 7.85-7.89 (1H, m), 8.29 (2H, d, *J* 1.6), 8.48 (1H, d, *J* 2.3), 8.60-8.65 (1H, m), 9.63 (1H, d, *J* 2.3). | 340 |
| 11 | 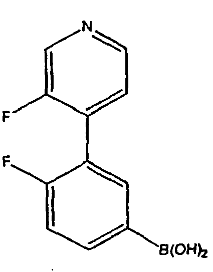 | 5-[4-Fluoro-3-(3-fluoro-pyridin-4-yl)phenyl]-3-phenyl-pyridazine | 7.54-7.68 (4H, m), 7.77 (1H, dd, *J* 5.1, 6.3), 8.29-8.36 (4H, m), 8.57 (1H, d, *J* 2.3), 8.63 (1H, dd, *J* 1.2, 5.1), 8.77 (1H, d, *J* 2.0), 9.71 (1H, d, *J* 2.3). | 346 |
| 2 | 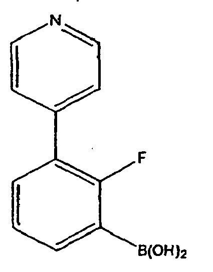 | 5-[2-Fluoro-3-(pyridin-4-yl)-phenyl]-3-phenyl-pyridazine | 7.50-7.71 (6H, m), 7.79-7.83 (1H, m), 7.94-7.99 (1H, m), 8.25-8.27 (2H, m), 8.48 (1H, dd, *J* 0.8, 1.2), 8.72-8.73 (2H, m), 9.53 (1H, t, *J* 2.0). | 328 |
| 13 | 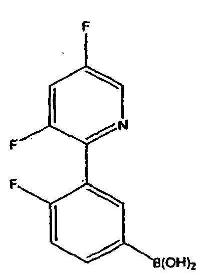 | 5-[3-(3,5-Difluoro-pyridin-2-yl)-4-fluorophenyl]-3-phenyl-pyridazine | 7.54-7.68 (4H, m), 8.16-8.22 (1H, m), 8.27-8.33 (4H, m), 8.54 (1H, d, *J* 2.0), 8.75 (1H, d, *J* 2.3), 9.66 (1H, d, *J* 2.3). | 364 |
| 14 | 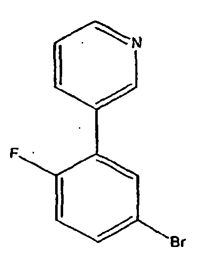 | 5-[4-Fluoro-3-(pyridin-3-yl)-phenyl]-3-phenyl-pyridazine pyridazine | 7.54-7.64 (5H, m), 8.13-8.22 (2H, m), 8.30-8.33 (3H, m), 8.60 (1H, d, *J* 2.5), 8.68 (1H, dd, *J* 1.6, 4.7), 8.94 (1H, s), 9.73 (1H, d, *J* 2.5). | 328 |
| 15 | 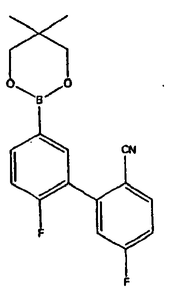 | 5,2'-Difluoro-5'-(6-phenyl-pyridazin-4-yl)-biphenyl-2-carbonitrile | 7.54-7.80 (6H, m), 8.15-8.20 (1H, m), 8.29-8.36 (4H, m), 8.58 (1H, d, *J* 2.3), 9.72 (1H, d, *J* 2.3). | 370 |
| 16 | 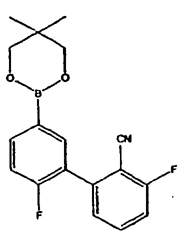 | 3,2'-Difluoro-5'-(6-phenyl-pyridazin-4-yl)-biphenyl-2-carbonitrile | 7.54-7.62 (3H, m), 7.65-7.71 (3H, m), 7.95-8.00 (1H, m), 8.29-8.38 (4H, m), 8.60-8.65 (1H, m), 9.70 (1H, d, *J* 2.4). | 370 |

### EXAMPLE 17

### 6,2'-Difluoro-5'-[6-(4-fluorophenyl]pyridazin-4-yl]biphenyl-2-carbonitrile

5-Chloropyridazin-3-one (22 mg, 0.169 mmol), palladium bis-(diphenylphosphinylbutane)dichloride (10 mg), 6,2'-difluoro-5'-(5,5-dimethyl-[1,3,2]dioxaborinan-2-yl)biphenyl-2-carbonitrile (50 mg, 0.15 mmol) and 2N Na₂CO₃ (0.3 ml) in toluene (3 ml) were combined and heated to 150°C for 600 s in a Smith Synthesiser microwave reactor. The reaction was diluted with CH₂Cl₂ (6 ml) and H₂O (2 ml) then poured into PTFE (5 µM) fritted syringe barrels. The organic phase was collected, concentrated and recrystallised from isopropanol to give 29 mg (62%) 6,2'-difluoro-5'-(6-oxo-1,6-dihydropyridazin-4-yl)biphenyl-2-carbonitrile.

The product from above was stirred with triethylamine (20 µl) and *N-*phenyltrifluoromethanesulfonimide (40 mg) in CH₂Cl₂ (5 ml) for 18 h. Further reagents were added and stirring continued for an additional 18 h. The reaction was concentrated and purified by column chromatography using 1:1 ethyl acetate/hexanes as eluent. Trifluoromethanesulfonic acid 5-(2'-cyano-6,6'-difluorobiphenyl-3-yl)pyridazin-3-yl ester was obtained as a yellow oil. *m*/*z* (ES⁺) 442.

The product from above, 4-fluorophenylboronic acid (14 mg), palladium bis(diphenylphosphinylbutane)dichloride (4 mg) and 2N Na₂CO₃ (0.5 ml) in tetrahydrofuran (2 ml) were combined and heated to 150°C for 600 s in a Smith Synthesiser microwave reactor. The reaction was diluted with CH₂Cl₂ (6 ml) and H₂O (2 ml) then poured into a PTFE (5 µM) fritted syringe barrel. The organic phase was collected, concentrated and purified by column chromatography using 1:1 ethyl acetate/hexanes as eluent. The *title compound* was recrystallised from isopropanol to give a colourless solid (3.9 mg). δ_{H} (400 MHz, d⁶ DMSO) 7.42 (2H, t, *J* 8.8), 7.70 (1H, t, *J* 9.2), 7.76-7.87 (2H, m), 7.98 (1H, dd, *J* 1.0, 7.6), 8.34-8.39 (3H, m), 8.35-8.40 (1H, m), 8.45-8.48 (1H, m), 9.69 (1H, d, *J* 2.0); *m*/*z* (ES⁺) 388.

### EXAMPLE 18

### 4-Fluoro-3'-(6-phenylpyridazin-4-yl)biphenyl-2-carbonitrile

4-Fluoro-3'-(6-oxo-1,6-dihydropyridazin-4-yl)biphenyl-2-carbonitrile (0.35g, 1.20 mmol) was suspended in phosphorus oxychloride (10 ml) and heated to 70°C for 1 h. The reaction was cooled to room temperature, poured onto ice water and stirred vigorously for 1 h. The beige solid was collected by filtration and azeotroped with methanol to give 3'-(6-chloropyridazin-4-yl)-4-fluorobiphenyl-2-carbonitrile (0.24 g). δ_{H} (400 MHz, d⁶ DMSO) 7.73-7.82 (3H, m), 7.83-7.87 (1H, m), 8.04 (1H, dd, *J* 2.7, 9.0), 8.12 (1H, dt, *J* 1.6, 7.8), 8.22 (1H, t, *J* 1.6), 8.40 (1H, d, *J* 2.0), 9.77 (1H, d, *J* 2.0); *mlz* (ES⁺) 310.

The product from above (40 mg, 0.129 mmol), benzeneboronic acid (24 mg, 0.197 mmol), tetrakis(triphenylphosphine)palladium(0) and 2N Na₂CO₃ (0.5 ml) in tetrahydrofuran (1.5 ml) were combined and heated to 150°C for 10 min in a Smith Synthesiser microwave reactor. The reaction was diluted with CH₂Cl₂ (6 ml) and NH₄Cl (2 ml) then poured into a PTFE (5 µM) fritted syringe barrel. The organic phase was concentrated while loading onto silica. Column chromatography using 40-50% ethyl acetate/hexanes gave the *title compound* as a white solid (20 mg). δ_{H} (400 MHz, d⁶ DMSO) 7.56-7.62 (3H, m), 7.74-7.80 (3H, m), 7.85-7.89 (1H, m), 8.03-8.06 (1H, m), 8.19 (1H, dt, *J* 1.8, 7.0), 8.29-8.32 (3H, m), 8.58 (1H, d, *J* 2.0), 9.71 (1H, d, *J* 2.0); *m*/*z* (ES⁺) 352.

### EXAMPLES 19 TO 47

The compounds in Table 2 were prepared using the requisite boronic acids in an analogous manner to that described in Examples 17 and 18.

**Table 2**

| **EX. NO.** | **PRODUCT** | δ_{H} (400 MHz, d⁶ DMSO unless specified otherwise) | *m*/*z* (ES⁺) |
|---|---|---|---|
| 19 | 6,2'-Difluoro-5'-[6-(thien-2-yl)pyridazin-4-yl]biphenyl-2-carbonitrile | 7.27 (1H, dd, *J* 4.7, 3.9), 7.71 (1H, t, *J* 9.2), 7.79-7.87 (3H, m), 7.97 (1H, d, *J* 7.0), 8.13 (1H, d, *J* 3.1), 8.33-8.35 (1H, m), 8.41 (1H, dd, *J* 1.2, 4.7), 8.59 (1H, d, *J* 1.2), 9.58 (1H, d, *J* 1.2). | 376 |
| 20 | 6,2'-Difluoro-5'-[6-(4-methoxyphenyl)-pyridazin-4-yl]biphenyl-2-carbonitrile | 3.86 (3H, s), 7.13 (2H, d, *J* 9.0), 7.69 (1H, t, *J* 9.2), 7.76-7.87 (2H, m), 7.97 (1H, dd, *J* 1.4, 7.6), 8.27 (2H, d, *J* 9.0), 8.32-8.36 (1H, m), 8.45 (1H, dd, *J* 2.3, 6.7), 8.48 (1H, d, *J* 2.0), 9.61 (1H, d, *J* 2.3). | 400 |
| 21 | 5'-[6-(3-Chlorophenyl)-pyridazin-4-yl]-6, 2'-difluorobiphenyl-2-carbonitrile | 7.62-7.63 (2H, m), 7.69-7.73 (1H, m), 7.76-7.87 (2H, m), 7.96 (1H, d, *J* 1.6), 8.28-8.31 (1H, m), 8.36-8.40 (2H, m), 8.48 (1H, dd, *J* 2.5, 6.8), 8.62 (1H, d, *J* 2.0), 9.73 (1H, d, *J* 2.3). | 404 |
| 22 | 6,2'-Diffuoro-5'-[6-(pyridin-3-yl)pyridazin-4-yl]biphenyl-2-carbonitrile | 7.63 (1H, dd, *J* 4.7, 7.8), 7.70-7.88 (3H, m), 7.98 (1H, dd, *J* 1.2, 7.4), 8.36-8.40 (1H, m), 8.49 (1H, dd, *J* 2.3, 6.7), 8.64-8.68 (2H, m), 8.76 (1H, dd, *J* 1.2, 4.7), 9.45 (1H, d, *J* 1.6), 9.76 (1H, d, *J* 2.3). | 371 |
| 23 | 5'-[6-(4-Chlorophenyl)-pyridazin-4-yl]-6,2'-difluorobiphenyl-2-carbonitrile | 7.56-7.92 (5H, m), 7.98 (1H, dd, *J* 1.2, 7.4), 8.28-8.50 (4H, m), 8.59 (1H, d, *J* 2.3), 9.71 (1H, d, *J* 2.3). | 404 |
| 24 | 6,2'-Difluoro-5'-[6-(pyridin-4-yl)pyridazin-4-yl]biphenyl-2-carbonitrile | 7.70-7.88 (3H, m), 7.97 (1H, d, *J* 7.4), 8.28 (2H, dd, *J* 1.4, 4.5), 8.37-8.41 (1H, m), 8.49 (1H, dd, *J* 2.5, 6.8), 8.70 (1H, d, *J* 2.3), 8.81-8.84 (2H, m), 9.80 (1H, d, *J* 2.3). | 371 |
| 25 | 5-[3-(3,5-Diffuoropyridin-2-yl)-4-fluorophenyl]-3-(4-fluorophenyl)pyridazine | 7.42 (2H, t, *J* 9.0), 7.62 (1H, dd, *J* 8.6, 0.8), 8.17-8.22 (1H, m), 8.27-8.39 (4H, m), 8.55 (1H, d, *J* 2.3), 8.75 (1H, d, *J* 2.3), 9.66 (1H, d, *J* 2.3). | 382 |
| 26 | 5-[4-Fluoro-3-(3-fluoro-pyridin-2-yl)phenyl]-3-(2-fluorophenyl)pyridazine | 7.41-7.46 (2H, m), 7.53-7.66 (3H, m), 7.91-8.02 (2H, m), 8.20-8.27 (2H, m), 8.37 (1H, t, *J* 2.0), 8.63-8.64 (1H, m), 9.72 (1H, d, *J* 2.3). | 364 |
| 27 | 5-[3-(3,5-Difluoropyridin-2-yl)-4-fluorophenyl]-3-(2-fluorophenyl)pyridazine | (CDCl₃): 7.20-7.24 (1H, m), 7.34-7.41 (3H, m), 7.47-7.53 (1H, m), 7.80-7.84 (1H, m), 7.96 (1H, dd, *J* 2.3, 6.7), 8.13 (1H, t, *J* 2.0), 8.19-8.24 (1H, m), 8.52 (1H, d, *J* 2.3), 9.44 (1H, d, *J* 2.0). | 382 |
| 28 | 5-[4-Fluoro-3-(3-fluoro-pyridin-2-yl)phenyl]-3-(pyridin-3-yl)pyridazine | 7.60-7.67 (3H, m), 7.92-7.97 (1H, m), 8.28-8.38 (2H, m), 8.64-8.68 (3H, m), 8.76 (1H, dd, *J* 1.6, 4.7), 9.46 (1H, d, *J* 2.0), 9.74 (1H, d, *J* 2.0). | 347 |
| 29 | 5-[3-(3,5-Difluoropyridin-2-yl)-4-ffuorophenyl]-3-(3-fluorophenyl)pyridazine | (CDCl₃): 7.20-7.25 (1H, m), 7.36-7.43 (2H, m), 7.50-7.55 (1H, m), 7.81-8.00 (5H, m), 8.52 (1H, d, *J* 2.3), 9.45 (1H, d, *J* 2.0). | 382 |
| 30 | 3-(2,4-Difluorophenyl)-5-[3-(3,5-difluoropyridin-2-yl)-4-fluorophenyl]-pyridazine | (CDCl₃): 6.96-7.02 (1H, m), 7.08-7.13 (1H, m), 7.36-7.42 (2H, m), 7.79-7.83 (1H, m), 7.95 (1H, dd, *J* 2.3, 6.3), 8.09 (1H, t, *J* 2.2), 8.23-8.29 (1H, m), 8.52 (1H, d, *J* 2.3), 9.44 (1H, d, *J* 2.3). | 400 |
| 31 | 5-[3-(3,5-Difluoropyridin-2-yl)-4-fluorophenyl]-3-(3-methoxyphenyl)-pyridazine | (CDCl₃): 3.93 (3H, s), 7.06-7.09 (1H, m), 7.36-7.47 (3H, m), 7.64 (1H, d, *J* 1.6), 7.79-7.84 (2H, m), 7.97 (1H, dd, *J* 2.3, 6.7), 8.01 (1H, d, *J* 2.3), 8.52 (1H, d, *J* 2.3), 9.43 (1H, d, *J* 2.3). | 394 |
| 32 | 6,2'-Difluoro-5'-[6-(2-fluorophenyl)pyridazin-4-yl]biphenyl-2-carbonitrile | 7.41-7.46 (2H, m), 7.59-7.72 (2H, m), 7.75-7.86 (2H, m), 7.94-8.01 (2H, m), 8.27-8.31 (1H, m), 8.39 (2H, q, *J* 2.1), 9.73 (1H, d, *J* 2.3). | 388 |
| 33 | 6,2'-Difluoro-5'-[6-(3-fluorophenyl)pyridazin-4-yl]biphenyl-2-carbonitrile | 7.38-7.43 (1H, m), 7.61-7.88 (4H, m), 7.96-8.05 (1H, m), 8.13-8.19 (2H, m), 8.35-8.40 (1H, m), 8.48 (1H, dd, *J* 2.5, 6.7), 8.61 (1H, d, *J* 2.1), 9.73 (1H, d, *J* 1.8). | 388 |
| 34 | 5'-[6-(2-Chlorophenyl)-pyridazin-4-yl]-6,2'-difluorobiphenyl-2-carbonitrile | 7.60-7.65 (2H, m), 7.73 (1H, t, *J* 9.1), 7.78-7.90 (2H, m), 7.98-8.05 (1H, m), 8.33 (2H, dd, *J* 1.9, 7.5), 8.36-8.41 (1H, m), 8.50 (1H, dd; *J* 2.3, 6.8), 8.58 (1H, d, *J* 2.1), 9.72 (1H, d, *J* 2.1). | |
| 35 | 4-Fluoro-3'-[6-(4-methoxy-phenyl)pyridazin-4-yl]-biphenyl-2-carbonitrile | 3.86 (3H, s), 7.14 (2H, d, *J* 8.8), 7.73-7.79 (3H, m), 7.85-7.89 (1H, m), 8.03-8.06 (1H, m), 8.17 (1H; dt, *J* 2.0, 7.0), 8.27-8.28 (1H, m), 8.28 (2H, d, *J* 8.8), 8.51 (1H, d, *J* 2.3), 9.63 (1H, d, *J* 2.3). | 382 |
| 36 | 5-[3-(3,5-Difluoropyridin-2-yl)-4-fluorophenyl]-3-(4-methoxyphenyl)-pyridazine | (CDCl₃): 3.90 (3H, s), 7.07 (2H, d, *J* 9.0), 7.36-7.41 (2H, m), 7.79-7.82 (1H, m), 7.94-7.96 (2H, m), 8.13 (1H, q, *J* 5.0), 8.12 (1H, d, *J* 9.0), 8.52 (1H, d, *J* 2.3), 9.36 (1H, d, *J* 2.3). | 394 |
| 37 | 3-(4-Chlorophenyl)-5-[3- (3,5-difluoropyridin-2-yl)-4-fluorophenyl]-pyridazine | 7.62-7.67 (3H, m), 8.17-8.22 (1H, m), 8.27-8.36 (4H, m), 8.58 (1H, d, *J* 2.0), 8.75 (1H, d, *J* 2.3), 9.68 (1H, d, *J* 2.3). | 398 |
| 38 | 2-{5-[3-(3,5-Difluoro-pyridin-2-yl)-4-fluoro-phenyl]pyridazin-3-yl}-5-fluorobenzonitrile | (CDCl₃): 7.36-7.45 (2H, m), 7.50-7.59 (2H, m), 7.84-7.87 (1H, m), 7.99 (1H, dd, *J* 2.3, 6.3), 8.15 (1H, dd, *J* 5.5, 8.6), 8.18 (1H, d, *J* 2.0), 8.51 (1H, d, *J* 2.3), 9.54 (1H, d, *J* 2.3). | 398 |
| 39 | 3-(4-Chlorophenyl)-5-[4-fluoro-3-(3-fluoropyridin-2-yl)phenyl]pyridazine | (CDCl₃): 7.38-7.46 (2H, m), 7.52-7.61 (3H, m), 7.80-7.84 (1H, m), 8.00 (1H, d, *J* 1.6), 8.01 (1H, d, *J* 2.3), 8.11 (2H, d, *J* 8.2), 8.60 (1H, d, *J* 4.3), 9.44 (1H, d, *J* 2.0). | 380 |
| 40 | 5-[4-Fluoro-3-(3-fluoro-pyridin-2-yl)phenyl]-3-(furan-3-yl)pyridazine | (CDCl₃): 7.08-7.10 (1H, m), 7.36-7.46 (2H, m), 7.55-7.61 (2H, m), 7.76-7.81 (2H, m), 7.98 (1H, dd, *J* 2.5, 6.5), 8.23 (1H, s), 8.59-8.61 (1H, m), 9.34 (1H, d, *J* 2.3). | |
| 41 | 5-[4-Fluoro-3-(3-fluoro-pyridin-2-yl)phenyl]-3-(furan-2-yl)pyridazine | (CDCl₃): 6.63 (1H, q, *J* 1.8), 7.36-7.45 (3H, m), 7.55-7.60 (1H, m), 7.63-7.64 (1H, m), 7.80-7.84 (1H, m), 8.01 (1H, dd, *J* 2.5, 6.5), 8.04 (1H, d, *J* 2.0), 8.59-8.61 (1H, m), 9.34 (1H, d, *J* 2.3). | 336 |
| 42 | 3-(2,3-Difluorophenyl)-5-[4-fluoro-3-(3-fluoro-pyridin-2-yl)phenyl]-pyridazine | (CDCl₃): 7.24-7.45 (4H, m), 7.52-7.60 (1H, m), 7.80-7.84 (1H, m), 7.94-8.02 (2H, m), 8.13 (1H, t, *J* 2.2), 8.59-8.61 (1H, m), 9.48 (1H, d, *J* 2.0). | 382 |
| 43 | 5-[4-Fluoro-3-(3-fluoro-pyridin-2-yl)phenyl]-3-(thien-3-yl)pyridazine | (CDCl₃): 7.37-7.52 (3H, m), 7.55-7.61 (1H, m), 7.79-7.83 (1H, m), 7.86 (1H, dd, *J* 1.2, 5.1), 7.92 (1H, d, *J* 2.3), 7.99 (1H, dd, J 2.3, 6.3), 8.14 (1H, q, J 1.4), 8.59-8.61 (1H, m), 9.37 (1H, d, *J* 2.0). | 352 |
| 44 | 5-[4-Fluoro-3-(3-fluoro-pyridin-2-yl)phenyl]-3-(thien-2-yl)pyridazine | (CDCl₃): 7.19 (1H, dd, *J* 3.7, 4.9), 7.37-7.46 (2H, m), 7.52-7.61 (2H, m), 7.77-7.82 (2H, m), 7.94 (1H, d, *J* 2.3), 7.99 (1H, dd, *J* 2.5, 6.5), 8.59-8.61 (1H, m), 9.33 (1H, d, *J* 2.0). | 352 |
| 45 | 3-(2,5-Difluorophenyl)-5-[4-fluoro-3-(3-fluoro-pyridin-2-yl)phenyl]-pyridazine | (CDCl₃): 7.14-7.23 (2H, m), 7.37-7.45 (2H, m), 7.52-7.60 (1H, m), 7.80-7.84 (1H, m), 7.96-8.01 (2H, m), 8.16 (1H, t, *J* 2.2), 8.59-8.61 (1H, m), 9.47 (1H, d, *J* 2.3). | 382 |
| 46 | 3-(3,4-Difluorophenyl)-5-[4-fluoro-3-(3-fluoro-pyridin-2-yl)phenyl]-pyridazine | (CDCl₃): 7.31-7.46 (3H, m), 7.56-7.61 (1H, m), 7.80-7.84 (1H, m), 7.87-7.91 (1H, m), 7.97-8.09 (3H, m), 8.59-8.61 (1H, m), 9.45 (1H, d, *J* 2.3). | 382 |
| 47 | 4-{5-[4- Fluoro-3-(3-fluoro-pyridin-2-yl)phenyl]-pyridazin-3-yl}-benzonitrile | (CDCl₃): 7.39-7.47 (2H, m), 7.57-7.61 (1H, m), 7.82-7.87 (3H, m), 8.03 (1H, dd, *J* 2.5, 6.5), 8.06 (1H, d, *J* 2.0), 8.28 (1H, s), 8.30 (1H, t, *J* 1.8), 8.59-8.61 (1H, m), 9.51 (1H, d, *J* 2.3). | 371 |

### EXAMPLE 48

### 3'-(6-Ethylaminopyridazin-4-yl)-4-fluorobiphenyl-2-carbonitrile

*N*-[5-(3-Bromophenyl)pyridazin-3-yl]-*N-*ethylamine (0.1 g, 0.360 mmol), 2-(5,5-dimethyl-[1,3,2]dioxaborinan-2-yl)-5-fluorobenzonitrile (0.1 g, 0.429 mmol), 2N Na₂CO₃ (0.5 ml) and tetrakis(triphenylphosphine)-palladium(0) (10 mg) in tetrahydrofuran (1.5 ml) were combined and heated to 150°C for 10 min in a Smith Synthesiser microwave reactor. The reaction was diluted with CH₂Cl₂ (6 ml) and NH₄Cl (2 ml) then poured into a PTFE (5 µM) fritted syringe barrel. The organic phase was concentrated while loading onto silica and purified by column chromatography using 50% ethyl acetate/hexanes followed by ethyl acetate as eluent. The *title compound* was obtained as a white solid by recrystallising from ethyl acetate (46 mg). δ_{H} (400 MHz, d⁶ DMSO) 1.19 (3H, t, *J* 7.0), 3.41 (2H, qd, *J* 5.1, 7.0), 6.88 (1H, t, *J* 5.1), 7.05 (1H, d, *J* 1.6), 7.69-7.76 (3H, m), 7.79-7.83 (1H, m), 7.85-7.88 (1H, m), 7.94-7.95 (1H, m), 8.01-8.04 (1H, m), 8.85 (1H, d, *J* 1.6); *m*/*z* (ES⁺) 319.

### EXAMPLES 49 TO 51

The compounds in Table 3 were prepared in an analogous manner to that described in Example 48.

**Table 3**

| **EX. NO.** | **PRODUCT** | δ_{H} (400 MHz, d⁶ DMSO) | *m*/*z* (ES⁺) |
|---|---|---|---|
| 49 | 4-Fluoro-3'-(6-isopropylamino-pyridazin-4-yl)-biphenyl-2-carbonitrile | 1.21 (6H, d, *J* 6.3), 4.14-4.22 (1H, m), 6.74 (1H, d, *J* 7.8), 7.03 (1H, d, *J* 2.0), 7.69-7.76 (3H, m), 7.79-7.82 (1H, m), 7.54-7.86 (1H, m), 7.92-7.94 (1H, m), 8.01-8.04 (1H, m), 8.83 (1H, d, J 2.0). | 333 |
| 50 | 4-Fluoro-3'-(6-propylaminopyridazin-4-yl)biphenyl-2-carbonitrile | 0.95 (3H, d, *J* 7.2), 1.56-1.67 (2H, m), 3.33-3.37 (2H, m), 6.89-6.93 (1H, m), 7.06-7.07 (1H, m), 7.69-7.88 (5H, m), 7.93-7.95 (1H, m), 8.00-8.04 (1H, m), 8.84-8.85 (1H, m). | 333 |
| 51 | 3'-(6-Cyclopropyl-aminopyridazin-4-yl)-4-fluorobiphenyl-2-carbonitrile | 0.48-0.52 (2H, m), 0.75-0.80 (2H, m), 2.65-2.71 (1H, m), 7.16 (1H, d, *J* 2.0), 7.35-7.37 (1H, m), 7.70-7.76 (3H, m), 7.80-7.84 (1H, m), 7.88-7.91 (1H, m), 7.98-7.99 (1H, m), 8.01-8.04 (1H, m), 8.92 (1H, d, *J* 2.0). | 331 |
| 52 | 3'-(6-Allylamino-pyridazin-4-yl)-4-fluorobiphenyl-2-carbonitrile | 4.04-4.07 (2H, m), 5.09-5.13 (1H, m), 5.23-5.28 (1H, m), 5.93-6.02 (1H, m), 7.07-7.09 (1H, m), 7.10 (1H, d, *J* 1.6), 7.70-7.76 (3H, m), 7.79-7.83 (1H, m), 7.85- 7.88 (1H, m), 7.94-7.95 (1H, m), 8.01-8.04 (1H, m), 8.80 (1H, d, *J* 1.6). | 331 |
| 53 | 3'-(6-Benzylamino-pyridazin-4-yl)-4-fluorobiphenyl-2-carbonitrile | 4.64 (2H, d, *J* 5.9), 7.14 (1H, d, J 2.0), 7.22-7.26 (1H, m), 7.31-7.35 (2H, m), 7.38-7.41 (2H, m), 7.45 (1H, t, *J* 5.2), 7.69-7.76 (3H, m), 7.78-7.82 (1H, m), 7.84-7.87 (1H, m), 7.92-7.94 (1H, m), 8.01-8.04 (1H, m), 8.89 (1H, d, *J* 2.0). | 381 |
| 54 | 4-Fluoro-3'-(6-methylamino-pyridazin-4-yl)-biphenyl-2-carbonitrile | 2.92 (3H, d, *J* 5.1), 6.88 (1H, t, *J* 5.1), 7.06 (1H, d, *J* 2.0), 7.69-7.76 (3H, m), 7.79-7.83 (1H, m), 7.86-7.89 (1H, m), 7.95-7.96 (1H, m), 8.00-8.04 (1H, m), 8.87 (1H, d, *J* 2.0). | 305 |
| 55 | 4-Fluoro-3'-(6-methoxypyridazin-4-yl)biphenyl-2-carbonitrile | 4.09 (3H, s), 7.61 (1H, d, *J* 2.0), 7.70-7.77 (3H, m), 7.82-7.86 (1H, m), 8.01-8.04 (2H, m), 8.13 (1H, t, *J* 1.6), 9.39 (1H, d, *J* 2.0). | 306 |
| 56 | 3'-(6-Ethoxypyridazin-4-yl)-4-fluorobiphenyl-2-carbonitrile | 1.42 (3H, t, *J* 7.0), 4.55 (2H, q, *J* 7.0), 7.59 (1H, d, J 2.0), 7.70-7.77 (3H, m), 7.83-7.86 (1H, m), 8.01-8.04 (2H, m), 8.13-8.14 (1H, m), 9.37 (1H, d, *J* 2.0). | 320 |
| 51 | 3'-(6-Benzyloxy-pyridazin-4-yl)-4-fluorobiphenyl-2-carbonitrile | 5.59 (2H, s), 7.34-7.44 (3H, m), 7.52-7.55 (2H, m), 7.70 (1H, d, *J* 2.0), 7.71-7.77 (3H, m), 7.82-7.86 (1H, m), 8.01-8.06 (2H, m), 8.14-8.15 (1H, m), 9.41 (1H, d, J 2.0). | 382 |

### Reference EXAMPLE 2

### 5-(4-Fluoro-3-hydroxyphenyl)-3-phenylpyridazine

To a solution of 5-(4-fluoro-3-methoxyphenyl)-3-phenylpyridazine (0.6 g, 2.14 mmol) in dichloromethane cooled to -70°C was added boron tribromide (0.81 ml, 8.56 mmol). The reaction mixture was stirred at -70°C for 30 min then allowed to warm to room temperature and stirred for 2 h. The reaction was cooled to 0°C, then 1 ml of methanol was added. The reaction mixture was poured onto a solution of sodium hydrogencarbonate, the precipitate obtained was collected by filtration, washed with water and dried (MgSO₄) to give the *title compound* (400 mg). δ_{H} (400 MHz, CDCl₃) 7.37 (1H, dd, *J* 8.4, 11.2), 7.47-7.61 (5H, m), 8.26-8.28 (2H, m), 8.37 (1H, d, *J* 2.0), 9.50 (1H, d, *J*.2.0), 10.25-10.40 (1H, br s); *m*/*z* (ES⁺) 267.

### EXAMPLE 58

### 5-[4-Fluoro-3-(2-methyl-2H-[1,2,4]triazol-3-ylmethoxy)phenyl]-3-phenylpyridazine

To a solution of 5-(4-fluoro-3-hydroxyphenyl)-3-phenylpyridazine (80 mg, 0.3 mmol) in *N,N-*dimethylformamide (2 ml) was added sodium hydride (30 mg, 0.6 mmol). After stirring for 10 min (2-methyl-2*H-*[1,2,4]triazol-3-yl)methanol (50 mg, 0.3 mmol) was added. The reaction mixture was heated at 60°C for 30 min. The reaction mixture was poured into water, and the precipitate obtained was collected by filtration, washed with water and dried (MgSO₄). The solid was dissolved in ethyl acetate, adsorbed onto silica and purified by flash chromatography using ethyl acetate/hexane (20:1) as eluent. The appropriate fractions were combined and evaporated under reduced pressure to give the *title compound* as a solid. δ_{H} (400 MHz, CDCl₃) 4.05 (3H, s), 5.44 (2H, s), 7.27-7.37 (3H, m), 7.51-7.61 (3H, m), 7.92 (2H, dd, *J* 11.3, 2.3), 8.16 (2H, dd, *J* 6.3, 1.6), 9.34 (1H, d, *J* 1.6); *m*/*z* (ES⁺) 362.

### EXAMPLE 59

### 5-[4-Fluoro-3-(1-methyl-3-trifluoromethyl-1H-pyrazol-4-ylmethoxy)-phenyl] -3-phenylpyridazine

To a slurry of 5-(4-fluoro-3-hydroxyphenyl)-3-phenylpyridazine (80 mg, 0.3 mmol), (1-methyl-3-tritluoromethyl-1*H*-pyrazol-4-yl)methanol (81 mg, 0.45 mmol) and triphenylphosphine (118 mg, 0.45 mmol) in tetrahydrofuran (2 ml) was added diisopropyl azodicarboxylate (91 mg, 0.45 mmol). The reaction mixture was stirred at room temperature for 2 h. The reaction mixture was adsorbed onto silica and purified by flash chromatography using a gradient elution of isohexane/ethyl acetate 20:1 to 1:20 as eluent. The appropriate fractions were combined and evaporated under reduced pressure to give a solid which was recrystallized from acetonitrile to give the *title compound* (45 mg). δ_{H} (400 MHz, CDCl₃) 3.96 (3H, s), 5.21 (2H, s), 7.27-7.34 (3H, m), 7.51-7.61 (4H, m), 7.91 (1H, d, *J* 2.3), 8.13-8.14 (2H, m), 9.35 (1H, d, *J* 2.3); *m*/*z* (ES⁺) 429.

### EXAMPLES 60 AND 61

The compounds in Table 4 were prepared in an analogous manner to that described in Example 58.

**Table 4**

| **EX. NO.** | **PRODUCT** | δ_{H} (400 MHz, CDCL₃) | *m*/*z* (ES⁺) |
|---|---|---|---|
| 60 | 5-[4-Fluoro-3-(pyridin-4-ylmethoxy)phenyl]-3-phenylpyridazine | 5.27 (2H, s), 7.28-7.34 (3H, m), 7.42 (2H, d, *J* 6.3), 7.51-7.59 (3H, m), 7.88 (1H, d, *J* 2.0), 8.11-8.13 (2H, m), 8.66-8.68 (2H, m), 9.32 (1H, d, J 2.0). | 358 |
| 61 | 5-[4-Fluoro-3-(pyridin-3-ylmethoxy)phenyl]-3-phenylpyridazine | 5.26 (2H, s), 7.27-7.39 (4H, m), 7.51-7.59 (3H, m), 7.83-7.86 (1H, m), 7.90 (1H, d, *J* 2.3), 8.12-8.14 (2H, m), 8.63 (1H, d, *J* 3.9), 8.74 (1H, s), 9.33 (1H, d, *J* 2.0). | 358 |

### EXAMPLE 62

### 5-[4-Fluoro-3-(3-fluoropyridin-2-yl)phenyl]-3-(pyridin-4-yl)pyridazine

To 3-chloro-5-[4-fluoro-3-(3-fluoropyridin-2-yl)phenyl]pyridazine (0.2 g, 0.66 mmol) in 1,4-dioxane (2 ml) was added 4-tri-*n*-butylstannylpyridine (0.484 g, 1.31 mmol) followed by tetrakis(triphenylphosphine)palladium(0) (0.076 g, 0.065 mmol) and the mixtures heated at 150°C in a Smith Synthesiser microwave reactor for 12 min. The reaction was diluted with dichloromethane, filtered and evaporated to give a brown solid. The crude product was chromatographed on silica eluting with 1-5% methanol/CH₂Cl₂. Appropriate fractions were pooled and evaporated to give the *title compound* as a cream-coloured solid. δ_{H} (400 MHz, CDCl₃) 7.40-7.47 (2H, m), 7.57-7.61 (1H, m), 7.82-7.86 (1H, m), 8.02-8.09 (4H, m), 8.60-8.62 (1H, m), 8.82-8.84 (2H, m), 9.53 (1H, d, *J* 2.3); *m*/*z* (ES⁺) 347.

### EXAMPLES 63 TO 66

The compounds in Table 5 were prepared using the requisite heterocyclic stannanes in an analogous manner to that described in Example 62 above.

**Table 5**

| **EX. NO.** | **PRODUCT** | δ_{H} (400 MHz, CDCl₃) | *m*/*z* (ES⁺) |
|---|---|---|---|
| 63 | 5-[4-Fluoro-3-(3-fluoropyridin-2-yl)phenyl]-3-(pyrazin-2-yl)-pyridazine | 7.38-7.46 (2H, m), 7.56-7.61 (1H, m), 7.87-7.92 (1H, m), 8.09 (1H, dd, *J* 2.3, 6.7), 8.59 (1H, m), 8.68-8.75 (3H, m), 9.55 (1H, d, *J* 2.3), 9.97 (1H, d, *J* 1.2). | 348 |
| 64 | 5-[4-Fluoro-3-(3-fluoropyridin-2-yl)phenyl]-3-(thiazol-2-yl)pyridazine | 7.37-7.46 (2H, m), 7.55-761 (2H, m), 7.86-7.90 (1H, m), 8.02 (1H, d, *J* 3.5), 8.08 (1H, dd, *J* 2.3, 6.7), 8.56 (1H, d, *J* 2.3), 8.59-8.61 (1H, m), 9.49 (1H, d, *J* 2.3). | 353 |
| 65 | 5-[4-Fluoro-3-(3-fluoropyridin-2-yl)phenyl]-3-(pyridin-2-yl)pyridazine | 7.54-7.67 (3H, m), 7.92-7.97 (1H, m), 8.05-8.09 (1H, m), 8.24 (1H, s), 8.26 (1H, s), 8.61-8.66 (2H, m), 8.78 (1H, d, *J* 2.3), 8.80 (1H, dd, *J* 0.8, 4.3), 9.77 (1H, d, *J* 2.3). | 347 |
| 66 | 5-[4-Fluoro-3-(3-fluoropyridin-2-yl)-phenyl]-3-(3-fluoro-pyridin-4-yl)-pyridazine | 7.39-7.46 (2H, m), 7.56-7.61 (1H, m), 7.81-7.85 (1H, m), 8.02 (1H, dd, *J 2*.5, 6.5), 8.23 (2H, s), 8.60 (1H, d, *J* 4.3), 8.67 (2H, dd, J 0.8, 7.0), 9.54 (1H, d, *J* 1.6). | 365 |

### EXAMPLE 67

### 5-[4-Fluoro-3-(3-fluoropyridin-2-yl)phenyl]-3-(1H-[1,2,3]triazol-4-yl)pyridazine

A degassed solution of 3-chloro-5-[4-fluoro-3-(3-fluoropyridin-2-yl)-phenyl]pyridazine (200 mg, 0.66 mmol) was formed in tetrahydrofuran (3 ml). (Trimethylsilyl)acetylene (0.14 ml, 0.99 mmol), triethylamine (0.14 ml, 1 mmol), bis(triphenylphosphine)palladium(II) chloride (23 mg, 0.03 mmol) and triphenylphosphine (4 mg, 0.015 mmol) were added in that order and the mixture left to stir for 20 min at room temperature. Copper(I) iodide (2 mg, 0.01 mmol) was added and the mixture stirred at room temperature for 18 h. The solvent was removed and the residue purified by flash column chromatography over silica using 80% ethyl acetate:20% isohexane as eluent to give 5-[4-fluoro-3-(3-fluoropyridin-2-yl)phenyl]-3-trimethylsilanylethynylpyridazine as a brown solid (210 mg). δ_{H} (400 MHz, CDCl₃) 0.31 (9H, s), 7.34-7.45 (2H, m), 7.55-7.60 (1H, m), 7.74-7.78 (1H, m), 7.79 (1H, d, *J* 2.3), 7.95 (1H, dd, *J* 2.5, 6.5), 8.58-8.61 (1H, m), 9.39 (1H, d, *J* 2.3).

Sodium azide (19 mg, 0.29 mmol) was added to a solution of 5-[4-fluoro-3-(3-fluoropyridin-2-yl)phenyl]-3-trimethylsilanylethynylpyridazine (100 mg, 0.26 mmol) in *N,N*-dimethylformamide (1 ml) and the mixture stirred at room temperature for 16 h, then at 70°C for 4 h. The reaction mixture was allowed to cool to room temperature and was added to water (20 ml) then extracted with ethyl acetate (5 x 10 ml). The combined organics were dried over anhydrous magnesium sulphate, filtered and evaporated to a brown solid. Purification by preparative thin-layer chromatography using 10% methanol:90% dichloromethane as eluent gave the *title compound* as a tan solid. δ_{H} (400 MHz, d⁶-DMSO) 7.63 (1H, t, *J* 9.8), 8.16-8.27 (3H, m), 8.49 (1H, d, *J* 2.3), 8.69 (1H, s), 8.75 (1H, d, *J* 2.3), 9.66 (1H, d, *J* 2.0); m/z (ES⁺) 355.

### EXAMPLE 68

### 5-[4-Fluoro-3-(3-fluoropyridin-2-yl)phenyl]pyridazine-3-carboxylic acid ethyl ester

A solution of 3-chloro-5-[4-fluoro-3-(3-fluoropyridin-2-yl)phenyl]-pyridazine (100 mg, 0.33 mmol) was formed in ethanol (10 ml) with *N,N-*dimethylformamide (2 ml). Sodium acetate (54 mg, 0.66 mmol) was added and the mixture degassed with nitrogen. [1,1'-Bis(diphenylphosphino)-ferrocene]palladium(II) chloride (1:1 complex with dichloromethane) (20 mg, 0.03 mmol) was added and carbon monoxide was bubbled through the solution as the mixture was heated to 90°C and maintained at that temperature for 45 min. The mixture was allowed to cool to room temperature and the solvent was removed. The residue was purified by flash column chromatography over silica, using 80% ethyl acetate:20% isohexane as eluent, then recrystallisation from dichloromethane and isohexane, to give the *title compound* as a white solid (30 mg). δ_{H} (400 MHz, CDCl₃) 1.51 (3H, t, *J* 7.0), 4.59 (2H, q, *J* 7.2), 7.38-7.46 (2H, m), 7.55-7.61 (1H, m), 7.81-7.86 (1H, m), 8.03 (1H, dd, *J* 2.5, 6.5), 8.41 (1H, d, *J* 2.3), 8.59-8.61 (1H, m), 9.62 (1H, d, *J* 2.3); m/z (ES⁺) 342.

### EXAMPLE 69

### 5-[3-(3,5-Difluoropyridin-2-yl)-4-fluorophenyl]-3-(2-fluorophenyl)-pyridazine-1-oxide

To a mixture of 5-[3-(3,5-difluoropyridin-2-yl)-4-fluorophenyl]-3-(2-fluorophenyl)pyridazine (50 mg, 0.13 mmol) in CH₂Cl₂ (2 ml) was added *m-*chloroperbenzoic acid (50%; 68 mg, 0.20 mmol). The solution was left to stand at room temperature for 22 h. 10% Sodium carbonate solution (1 ml) was added and the solution poured into a PTFE (5 µM) fritted syringe barrel. The organic phase was evaporated and the crude product purified by preparative TLC using a solvent system of 5% methanol in CH₂Cl₂ to yield a white solid. The solid was further purified by prep HPLC using a solvent system of 30-80% acetonitrile in 0.1% triffuoroacetic acid in water. Appropriate fractions were combined to give the *title compound* as a white solid (3 mg). δ_{H} (400 MHz, CDCl₃) 7.19-7.25 (1H, m), 7.29-7.41 (3H, m), 7.47-7.53 (1H, m), 7.73-7.76 (1H; m), 7.78 (1H, t, *J* 1), 7.91 (1H, dd, *J* 3, 7), 8.06-8.10 (1H, m), 8.42 (1H, d, *J* 1), 8.51 (1H, d, *J* 2); *m*/*z* (ES⁺) 398.

### EXAMPLE 70

### 3-(2,6-Difluorophenyl)-5-[4-fluoro-3-(3-fluoropyridin-2-yl)phenyl]pyridazine

To a degassed mixture of 3-chloro-5-[4-fluoro-3-(3-fluoropyridin-2-yl)phenyl]pyridazine (0.25 g, 0.82 mmol), hexamethylditin (0.323 g, 0.98 mmol) and lithium chloride (0.1024 g, 2.46 mmol) in 1,4-dioxane (12 ml) was added tetrakis(triphenylphosphine)palladium(0) (0.048 g, 0.041 mmol) and the mixture heated at 100°C for 3 h. Mass spectroscopy showed formation of the intended stannane. 1-Bromo-2,6-difluorobenzene (0.3177 g, 0.187 ml, 1.65 mmol) was added, followed by tetrakis(triphenylphosphine)palladium(0) (0.01 g, 0.0082 mmol), and the mixture heated at 100°C for 6 h. The reaction was cooled to room temperature, filtered and evaporated and the crude product purified by preparative TLC (2 plates; 4% methanol-CH₂Cl₂). Appropriate bands were collected and processed to give the *title compound* as an off-white solid which was recrystallised from ethyl acetate/isohexane to give a white solid (42 mg). δ_{H} (400 MHz, CDCl₃) 7.05-7.12 (2H, m), 7.32-7.59 (4H, m), 7.79-7.85 (2H, m), 8.00 (1H, dd, *J* 2.5, 6.5), 8.58-8.60 (1H, m), 9.51 (1H,d,*J* 2.3).

### EXAMPLE 71

### 3-(4-Chloro-2-fluorophenyl)-5-[3-(3,5-difluoropyridin-2-yl)-4-fluorophenyl]pyridazine

3-Chloro-5-[3-(3,5-diffuoropyridin-2-yl)-4-fluorophenyl]pyridazine was coupled to 1-bromo-4-chloro-2-fluorobenzene by the method of Example 108 to give 3-(4-chloro-2-fluorophenyl)-5-[3-(3,5-difluoropyridin-2-yl)-4-fluorophenyl]pyridazine as a white solid: δ_{H} (500 MHz, DMSO) 7.54 (1H, m), 7.61-7.65 (1H, m), 7.70 (1H, dd, *J* 10.0, 0.5), 8.03-8.06 (1H, m), 8.16-8.24 (3H, m), 8.37 (1H, s), 8.74 (1H, s), 9.73 (1H, s); *m*/*z* (ES⁺) 416:418 (3:1).

### EXAMPLE 72

### 5-[3-(3,5-Difluoropyridin-2-yl)-4-fluorophenyl]-3-(2-fluoro-4-trifluoromethylphenyl)pyridazine

3-Chloro-5-[3-(3,5-difluoropyridin-2-yl)-4-fluorophenyl]pyridazine was coupled to 4-bromo-3-fluorobenzotrifluoride by the method of Example 108 to give 5-[3-(3,5-difluoropyridin-2-yl)-4-fluorophenyl]-3-(2-fluoro-4-trifluoromethylphenyl)pyridazine as a white solid: δ_{H} (500 MHz, DMSO) 7.64 (1H, t, *J* 9.0), 7.82 (1H, d, *J* 8.1), 7.95 (1H, d, *J* 10.5), 8.16-8.26 (3H, m), 8.24 (1H, s), 8.46 (1H, s), 8.74 (1H, s), 9.78 (1H, s); *m*/*z* (ES⁺) 450..

### EXAMPLE 73

### 5-[3-(3,5-Difluoropyridin-2-yl)-4-fluorophenyl]-3-(2-fluoro-4-methylphenyl)-pyridazine

3-Chloro-5-[3-(3,5-difluoropyridin-2-yl)-4-fluorophenyl]pyridazine was coupled to 4-bromo-3-fluorotoluene by the method of Example 108 to give 5-[3-(3,5-difluoropyridin-2-yl)-4-fluorophenyl]-3-(2-fluoro-4-methylphenyl)pyridazine as a white solid: δ_{H} (500 MHz, DMSO) 2.42 (3H, s), 7.25 (2H, s), 7.62 (1H, t, *J* 9.4), 7.93 (1H, m), 8.21 (3H, m), 8.30 (1H, s), 8.74 (1H, d, *J* 0.5), 9.68 (1H, s); *m*/*z* (ES⁺) 396.

### EXAMPLE 74

### 3-(3,5-Difluoropyridin-2-yl)-5-[3-(3,5-difluoropyridin-2-yl)-4-fluorophenyl]pyridazine

3-Chloro-5-[3-(3,5-difluoropyridin-2-yl)-4-fluorophenyl]pyridazine was coupled to 2-bromo-3,5-difluoropyridine by the method of Example 108 to give 3-(3,5-difluoropyridin-2-yl)-5-[3-(3,5-difluoropyridin-2-yl)-4-fluorophenyl]pyridazine as a white solid: δ_{H} (500 MHz, DMSO) 7.61-7.65 (1H, m), 8.16-8.24 (4H, m), 8.48 (1H, s), 8.75 (2H, d, *J* 12.2), 9.79 (1H, s); *m*/*z* (ES⁺) 401.

### EXAMPLE 75

### Trifluoromethanesulfonic acid 5-[4-fluoro-3-(3-fluoropyridin-2-yl)phenyl]pyridazin-3-yl ester

A suspension of 5-[4-fluoro-3-(3-fluoropyridin-2-yl)phenyl]-2*H* pyridazin-3-one (1.29 g, 4.52 mmol) was formed in dry pyridine (20 ml) and cooled to 0°C. Trifluoromethanesulfonic anhydride (1.14 ml, 6.78 mmol) was added dropwise. The mixture was then allowed to warm to room temperature and after 30 min formed a dark red solution. The mixture was poured into water (100 ml) and extracted with dichloromethane (2 x 100 ml). The combined organics were dried over anhydrous magnesium sulphate, filtered and evaporated to a brown solid. Purification by flash column chromatography over silica using 80% dichloromethane:20% ethyl acetate as eluent then recrystallization from dichloromethane using isohexane gave trifluoromethanesulfonic acid 5-[4-fluoro-3-(3-fluoro-pyridin-2-yl)phenyl]pyridazin-3-yl ester as a white solid: δ_{H} (400 MHz, CDCl₃) 7.40-7.47 (2H, m), 7.56-7.61 (2H, m), 7.78-7.82 (1H, m), 8.00 (1H, dd, *J* 2 and 6), 8.59-8.61 (1H, m), 9.55 (1H, d, *J* 2); *m*/*z* (ES⁺) 418.

### EXAMPLE 76

### 3-Ethylsulfanyl-5-[4-fluoro-3-(3-fluoropyridin-2-yl)phenyl]pyridazine

A solution of 3-chloro-5-[4-fluoro-3-(3-fluoropyridin-2-yl)phenyl]-pyridazine (50 mg, 0.16 mmol) was formed in *N,N-*dimethylformamide (1 ml). Sodium ethanethiolate (28 mg, 0.33 mmol) was added and the mixture stirred at room temperature for 8 h. The reaction was poured into water (20 ml) and extracted with ethyl acetate (2 x 20 ml). The combined organics were dried over anhydrous magnesium sulphate, filtered and evaporated to an orange solid. Purification by preparative thin layer chromatography using ethyl acetate as eluent gave 3-ethylsulfanyl-5-[4-fluoro-3-(3-fluoropyridin-2-yl)phenyl]pyridazine as a white solid (10 mg): δ_{H} (400 MHz, CDCl₃) 1.47 (3H, t, *J* 7), 3.43 (2H, q, *J* 7), 7.35 (1H, t, *J* 9), 7.40-7.44 (1H, m), 7.48 (1H, d, *J* 2), 7.54-7.59 (1H, m), 7.70-7.74 (1H, m), 7.92 (1H, dd, *J* 2 and 6), 8.57-8.60 (1H, m), 9.17 (1H, d, *J* 2); *m*/*z* (ES⁺) 330.

### EXAMPLE 77

### 3-tert-Butylsulfanyl-5-[4-fluoro-3-(3-fluoropyridin-2-yl)phenyl]pyridazine

3-Chloro-5-[4-fluoro-3-(3-fluoropyridin-2-yl)phenyl]pyridazine (50 mg, 0.16 mmol) and sodium 2-methyl-2-propanethiolate (37 mg, 0.33 mmol) were reacted as in Example 114 to give 3-tert-butylsulfanyl-5-[4-fluoro-3-(3-fluoropyridin-2-yl)phenyl]pyridazine as a tan solid (13 mg): δ_{H} (400 MHz, CDCl₃) 1.66 (9H, s), 7.35 (1H, t, *J* 9), 7.40-7.44 (1H, m), 7.53 (1H, d, *J* 2), 7.54-7.59 (1H, m), 7.70-7.74 (1H, m), 7.91 (1H, dd, *J* 3 and 7), 8.58-8.60 (1H, m), 9.21 (1H, d, *J* 2); *m*/*z* (ES⁺) 302 (M+H-^{t}Bu) and 358 (M+H).

### EXAMPLE 78

### 5-[3-(3,5-Difluoropyridin-2-yl)-4-fluorophenyl]-3-(3-fluoropyridin-4-yl)-pyridazine

3-Chloro-5-[3-(3,5-difluoropyridin-2-yl)-4-fluorophenyl]pyridazine (100 mg, 0.31 mmol) was coupled to 3-fluoro-4-tributylstannylpyridine (144 mg, 0.37 mmol) using the method in Example 100 to give 5-[3-(3,5-difluoropyridin-2-yl)-4-fluorophenyl]-3-(3-fluoropyridin-4-yl)pyridazine as a white solid (31 mg): δ_{H} (400 MHz, CDCl₃) 7.36-7.44 (2H, m), 7.81-7.85 (1H, m), 7.97 (1H, dd, *J* 2 and 7), 8.21-8.24 (2H, m), 8.52 (1H, d, *J* 2), 8.64-8.68 (2H, m), 9.53 (1H, d, *J* 2); *m*/*z* (ES⁺) 383.

### EXAMPLE 79

### 5-[3-(3,5-Difluoropyrdin-2-yl)-4-fluorophenyl]-3-(3-fluoropyridin-2-yl)-pyridazine

3-Chloro-5-[3-(3,5-diffuoropyridin-2-yl)-4-fluorophenyl]pyridazine (139 mg, 0.43 mmol) was coupled to 3-fluoro-2-tributylstannylpyridine (200 mg, 0.51 mmol) using the method in Example 100 to give 5-[3-(3,5-difluoropyridin-2-yl)-4-fluorophenyl]-3-(3-fluoropyridin-2-yl)pyridazine as a white solid (70 mg): δ_{H} (500 MHz, CDCl₃) 7.36-7.41 (2H, m), 7.46-7.49 (1H, m), 7.66 (1H, t, *J* 10), 7.85-7.89 (1H, m), 8.01 (1H, dd, *J* 2 and 7), 8.41 (1H, s), 8.52 (1H, s), 8.61 (1H, d, *J* 4), 9.53 (1H, s); m/z (ES⁺) 383.

### EXAMPLE 80

### 5-[4-Fluoro-3-(3-fluoropyridin-2-yl)phenyl]-3-(3-fluoropyridin-2-yl)-pyridazine

3-Chloro,5-[4-fluoro-3-(3-fluoropyridin-2-yl)phenyl]pyridazine (216 mg, 0.71 mmol) was coupled to 3-fluoro-2-tributylstannylpyridine (300 mg, 0.78 mmol) using the method in Example 100 to give 5-[4-fluoro-3-(3-fluoropyridin-2-yl)phenyl]-3-(3-fluoropyridin-2-yl)pyridazine as a white solid (106 mg): δ_{H} (400 MHz, CDCl₃) 7.35-7.48 (3H, m), 7.55-7.61 (1H,m), 7.63-7.68 (1H, m), 7.84-7.89 (1H, m), 8.05 (1H, dd, *J* 3 and 7), 8.41 (1H, d, *J* 2), 8.58-8.62 (2H, m), 9.54 (1H, d, *J* 2); *m*/*z* (ES⁺) 365.

### EXAMPLE 81

### 5-[4-Fluoro-3-(3-fluoropyridin-2-yl)phenyl]-3-(3-fluoropyridin-4-yl)-pyridazine 1-oxide

A solution of 5-[4-fluoro-3-(3-fluoropyridin-2-yl)phenyl]-3-(3-fluoropyridin-4-yl)pyridazine (100 mg, 0.27 mmol) was formed in dichloromethane (4 ml). A solution of *m*-chloroperoxybenzoic acid (74 mg, 77%, 0.33 mmol) in dichloromethane (1 ml) was added and the mixture stirred at room temperature overnight. The reaction was quenched into aqueous sodium carbonate (2M, 10 ml) and extracted with dichloromethane (10 ml). The organic phase was washed with aqueous sodium carbonate (1M, 2 x 10 ml) then dried over anhydrous magnesium sulphate, filtered and evaporated to a tan solid. Purification by preparative HPLC gave 5-[4-fluoro-3-(3-fluoropyridin-2-yl)phenyl]-3-(3-fluoropyridin-4-yl)pyridazine 1-oxide as a white solid (14 mg): δ_{H} (400 MHz, CDCl₃) 7.39-7.47 (2H, m), 7.56-7.61 (1H, m), 7.74-7.78 (1H, m), 7.86 (1H, s), 7.96 (1H, dd, *J* 3 and 7), 8.02 (1H, t, *J* 5), 8.49 (1H, d, *J* 1), 8.58-8.68 (3H, m); *m*/*z* (ES⁺) 381.

### EXAMPLE 82

### 5-[4-Fluoro-3-(3-fluoropyridin-2-yl)phenyl]-3-(3-fluoro-1-oxypyridin-4-yl)-pyridazine

A solution of 3-fluoro-4-tributylstannylpyridine (1.0 g, 2.6 mmol) was formed in dichloromethane (5 ml). A solution of *m*-chloroperoxybenzoic acid (696 mg, 77%, 3.1 mmol) in dichloromethane (3 ml) was added and the mixture stirred at room temperature for 2 h. A further 10 ml of dichloromethane was added and the mixture was washed with saturated aqueous sodium bicarbonate (2 x 20 ml) then dried over anhydrous magnesium sulphate, filtered and evaporated to a yellow oil. Purification by flash column chromatography over silica using ethyl acetate as eluent gave 3-fluoro-4-tributylstannylpyridine 1-oxide as a colourless oil (1.0 g).

3-Chloro-5-[4-fluoro-3-(3-fluoropyridin-2-yl)phenyl]pyridazine (80 mg, 0.26 mmol) was coupled to 3-fluoro-4-tributylstannylpyridine 1-oxide (117 mg, 0.29 mmol) using the method in Example 100 to give 5-[4-fluoro-3-(3-fluoropyridin-2-yl)phenyl]-3-(3-fluoro-1-oxypyridin-4-yl)pyridazine as a tan solid (31 mg): δ_{H} (400 MHz, CDCl₃) 7.39-7.47 (2H, m), 7.56-7.62 (1H, m), 7.80-7.84 (1H, m), 8.00 (1H, dd, *J* 3 and 7), 8.18-8.22 (2H, m), 8.29 (1H, dd, *J* 2 and 7), 8.39 (1H, dd, *J* 7 and 9), 8.59-8.61 (1H, m), 9.50 (1H, d, *J* 2); *m*/*z* (ES⁺) 381.

### EXAMPLE 83

### 5-[2,4-Difluoro-3-(3,5-difluoropyridin-2-yl)phenyl]-3-(3,5-difluoropyridin-4-yl)pyridazine

A degassed solution of 2-bromo-3,5-difluoropyridine (4.0 g, 20.6 mmol), 2,6-difluorophenylboronic acid (3.9 g, 24.7 mmol) and potassium fluoride (4.3 g, 74.2 mmol) was formed in tetrahydrofuran (40 ml) with water (4 ml). Tris(dibenzylideneacetone)dipalladium(0) (189 mg, 0.21 mmol) followed by tri-*tert*-butylphosphine (0.8 ml, 0.51M in hexane, 0.42 mmol) were added and the mixture heated to 60°C for 18 h. The solvent was removed and the residue purified by flash column chromatography over silica using dichloromethane 60%:isohexane 40% as eluent to give 3,5-difluoro-2-(2,6-difluorophenyl)pyridine as a white solid (2.2 g): δ_{H} (400 MHz, CDCl₃) 6.99-7.06 (2H, m), 7.32-7.46 (2H, m), 8.50 (1H, d, *J* 2.3).

A solution of 3,5-difluoro-2-(2,6-difluorophenyl)pyridine (1.65 g, 7.26 mmol) was formed in concentrated sulphuric acid (10 ml) and cooled to 0°C. 1,3-Dibromo-5,5-dimethylhydantoin (2.08 g, 7.26 mmol) was added portionwise and the mixture left to stir at 0°C for 2 h. The reaction was quenched into ice (250 ml) and extracted with dichloromethane (2 x 100 ml). The combined organics were dried over anhydrous magnesium sulphate, filtered and evaporated to a yellow oil. Purification by flash column chromatography over silica using 60% isohexane:40% dichloromethane as eluent gave 2-(3-bromo-2,6-difluorophenyl)-3,5-difluoropyridine as a colourless oil (1.95 g): δ_{H} (400 MHz, CDCl₃) 6.94-6.99 (1H, m), 7.34-7.39 (1H, m), 7.61-7.67 (1H, m), 8.50 (1H, d, *J*2.3).

A degassed solution of 2-(3-bromo-2,6-difluorophenyl)-3,5-difluoropyridine (1.95 g, 6.37 mmol), bis(neopentyl glycolato)diborane (1.58 g, 7.01 mmol) and potassium acetate (1.25 g, 12.7 mmol) was formed in 1,4-dioxane (20 ml) with dimethylsulphoxide (0.4 ml). [1,1'-Bis(diphenylphosphino)ferrocene]dichloropalladium(II) complex with dichloromethane (1:1) (100 mg) was added and the mixture heated at 80°C for 18 h. The solvent was removed and the residue stirred with aqueous sodium hydroxide (2N, 100 ml) for 30 min, then filtered. The filtrate was washed with diethyl ether (2 x 50 ml) then cooled to 0°C before being neutralised with concentrated aqueous hydrochloric acid, then extracted with dichloromethane (2 x 50 ml). The combined organics were dried over anhydrous magnesium sulphate, filtered and evaporated to give 3,5-difluoro-2-[2,6-difluoro-3-(5,5-dimethyl-[1,3,2]dioxaborinan-2-yl)phenyl]pyridine as a pale yellow oil: δ_{H} (400 MHz, CDCl₃) 1.03 (6H, s), 3.78 (4H, s), 5.30 (1H, s), 6.97-7.02 (1H, m), 7.29-7.35 (1H, m), 7.80-7.86 (1H, m), 8.48 (1H, d, *J* 2.3).

A degassed solution of 3-chloro-5-methoxypyridazine (473 mg, 3.27 mmol), 3,5-difluoro-4-trimethylstannanylpyridine (1.0 g, 3.6 mmol), copper(I) iodide (31 mg, 0.2 mmol) and lithium chloride (416 mg, 9.8 mmol) was formed in 1,4-dioxane (10 ml). Tetrakis(triphenylphosphine)-palladium(0) (100 mg) was added and the mixture heated to 80°C for 16 h. The solvent was removed and the residue was purified by flash column chromatography over silica using 40% isohexane:60% ethyl acetate to give 3-(3,5-diffuoropyridin-4-yl)-5-methoxypyridazine as a pale yellow solid (220 mg): δ_{H} (400 MHz, CDCl₃) 7.10-7.12 (1H, m), 8.54 (2H, s), 9.02 (1H, d, *J* 3.1); *m*/*z* (ES⁺) 224.

A solution of 3-(3,5-difluoropyridin-4-yl)-5-methoxypyridazine (210 mg, 0.94 mmol) was formed in aqueous hydrobromic acid (5 ml, 48%) and refluxed for 2 h. The mixture was cooled in an ice bath and neutralised with aqueous sodium hydroxide to ∼ pH 2 then with solid sodium hydrogencarbonate to neutral. The resulting white precipitate was filtered and dried under vacuum over phosphorus pentoxide to give 6-(3,5-difluoropyridin-4-yl)-1*H*-pyridazin-4-one as a white solid (110 mg): *m*/*z* (ES⁺) 210.

A suspension of 6-(3,5-difluoropyridin-4-yl)-1*H*-pyridazin-4-one (60 mg, 0.28 mmol) was formed in phosphorus oxychloride (0.8 ml) and heated to 80°C for 30 min to give a yellow solution. The phosphorus oxychloride was removed under reduced pressure and the residue diluted with dichloromethane (5 ml) and washed with water (3 x 10 ml) and saturated aqueous sodium hydrogencarbonate (10 ml) then dried over anhydrous magnesium sulphate, filtered and evaporated to give 5-chloro-3-(3,5-difluoropyridin-4-yl)pyridazine as a yellow solid (63 mg): δ_{H} (400 MHz, CDCl₃) 7.76-7.78 (1H, m), 8.58 (2H, s), 9.31 (1H, d, *J* 2.3).

A solution of 5-chloro-3-(3,5-difluoropyridin-4-yl)pyridazine (107 mg, 0.47 mmol) and 3,5-difluoro-2-[2,6-difluoro-3-(5,5-dimethyl-[1,3,2]dioxaborinan-2-yl)phenyl]pyridine (191 mg, 0.56 mmol) was formed in 1,4-dioxane (4 ml) with aqueous sodium carbonate (1 ml, 2 M). Tetrakis(triphenylphosphine)palladium(0) (50 mg) was added and the mixture heated to 150°C for 700 s in a Smith Synthesiser microwave reactor. The solvent was evaporated and the residue purified by flash column chromatography over silica using dichloromethane:ethyl acetate (1:1). Recrystallization from dichloromethane using isohexane gave 5-[2,4-difluoro-3-(3,5-difluoropyridin-2-yl)phenyl]-3-(3,5-difluoropyridin-4-yl)pyridazine as a white solid (106 mg): δ_{H} (400 MHz, CDCl₃) 7.25-7.29 (1H, m), 7.38-7.43 (1H, m), 7.66-7.72 (1H, m), 7.90-7.91 (1H, m), 8.54 (1H, d, *J* 2.3), 8.57 (2H, s), 9.50-9.51 (1H, m); *m*/*z* (ES⁺) 419.

### EXAMPLES 84 AND 85

The compounds in Table 2A were prepared using the requisite boronic acids in an analogous manner to that described in Examples 17 and 18.

**Table 2A**

| **EX. NO.** | **PRODUCT** | δ_{H} | *m*/*z* (ES⁺) |
|---|---|---|---|
| 84 | 5-[3-(3,5-difluoropyridin-2-yl)-4-fluorophenyl]-3-(2-fluoro-4-methoxyphenyl)-pyridazine | (500 MHz, DMSO): 3.98 (3H, s), 7.42 (1H, s), 7.65 (1H, dd, *J* 8.7, 9.2), 7.89-7.91 (1H, m), 8.05 (1H, d, *J* 8.1), 8.17-8.21 (1H, m), 8.29 (1H, s), 8.30 (1H, s), 8.55 (1H, s), 8.75 (1H, s), 9.65 (1H, s). | 412 |
| 85 | 5-[4-Fluoro-3-(3-fluoropyridin-2-yl)-phenyl]-3-(2-fluoro-4-methoxyphenyl)-pyridazine | (400 MHz, CDCl₃): 3.89 (3H, s), 6.77 (1H, dd, *J* 2.3, 13.3), 6.91 (1H, dd, *J* 2.3, 8.6), 7.36-7.44 (2H, m), 7.55-7.60 (1H, m), 7.79-7.82 (1H, m), 7.99 (1H, dd, *J* 2.3, 6.7), 8.10 (1H, t, *J* 2.0), 8.21 (1H, t, *J* 9.0), 8.58-8.60 (1H, m), 9.39 (1H, d, *J* 2.0). | 394 |

### EXAMPLES 86 TO 88

The compounds in Table 5A were prepared using the requisite heterocyclic stannanes in an analogous manner to that described in Example 62.

**Table 5A**

| **EX. NO.** | **PRODUCT** | **δ_{H}** | *m*/*z* (ES⁺) |
|---|---|---|---|
| 86 | 3-(3,5-Difluoropyridin-4-yl)-5-[4-fluoro-3-(3-fluoropyridin-2-yl)phenyl]pyridazine | (400 MHz, d₆DMSO) 7.63-7.68 (2H, m), 7.95 (1H, m), 8.23-8.29 (2H, m), 8.55 (1H, br s), 8.64 (1H, m), 8.81 (2H, s), 9.88 (1H, d, *J* 1.8). | |
| 87 | 3-(3,5-Difluoropyridin-2-yl)-5-[4-fluoro-3-(3-fluoropyridin-2-yl)phenyl]pyridazine | (400 MHz, d₆DMSO) 7.61-7.67 (2H, m), 7.95 (1H, m), 8.22-8.27 (3H, m), 8.49 (1H, d, *J* 1.8), 8.65 (1H, m), 8.77 (1H, d, *J* 1.8), 9.81 (1H, d, *J* 1.9). | 383 |
| 88 | 3-(3,5-Difluoropyridin-4-yl)-5-[3-(3,5-difluoropyridin-2-yl)-4-fluorophenyl]pyridazine | (400 MHz, CDCl₃): 7.36-7.43 (2H, m), 7.80-7.84 (1H, m), 7.89 (1H, t, *J* 1.0), 7.97 (1H, dd, *J* 2.3, 6.7), 8.51 (1H, d, *J* 2.3), 8.57 (2H, s), 9.57 (1H, d, *J* 2.3). | 401 |

### EXAMPLE 89

### 3-(3,5-Difluoro-1-oxypyridin-4-yl)-5-[3-(3,5-difluoropyridin-2-yl)-4-fluorophenyl]pyridazine

A solution of 3,5-difluoro-4-trimethylstannanylpyridine (2.0 g, 7.2 mmol) was formed in dichloromethane (10 ml). A solution of 3-chloroperoxybenzoic acid (1.94 g, approx. 8.6 mmol) in dichloromethane (10 ml) was added dropwise and the solution stirred overnight at room temperature. The reaction mixture was poured into aqueous sodium carbonate (2M, 50 ml) and extracted with dichloromethane (2 x 50 ml). The combined organic phases were washed with water (50 ml), dried over anhydrous magnesium sulphate, filtered and evaporated to give crude 3,5-difluoro-4-trimethylstannanylpyridine 1-oxide as a white solid (1.31 g). 3,5-Difluoro-4-trimethylstannanylpyridine 1-oxide (438 mg, 1.5 mmol) was coupled to 3-chloro-5-[3-(3,5-difluoropyridin-2-yl)-4-fluorophenyl]-pyridazine (400 mg, 1.24 mmol) using the method in Example 100 to give 3-(3,5-difluoro-1-oxypyridin-4-yl)-5-[3-(3,5-difluoropyridin-2-yl)-4-fluorophenyl]pyridazine as a white solid (50 mg): δ_{H} (400 MHz, CDCl₃) 7.36-7.44 (2H, m), 7.79-7.83 (1H, m), 7.86-7.87 (1H, m), 7.95 (1H, dd, *J* 2.5, 6.5), 8.18-8.21 (2H, m), 8.51 (1H, d, *J* 2.3), 9.55 (1H, d, *J* 2.3); *m*/*z* (ES⁺) 417.

### EXAMPLE 90

### 5'-[6-(3,5-Difluoropyridin-2-yl)pyridazin-4-yl]-2'-fluorobiphenyl-2-carbonitrile

2'-Fluoro-5'-(4,4,5,5-tetramethyl-[1,3,2]dioxaborolan-2-yl)biphenyl-2-carbonitrile (2.50 g, 7.8 mmol) was coupled to 5-chloro-2*H*-pyridazin-3-one (1.32 g, 10.0 mmol) using the method in Example 33 to give 2'-fluoro-5'-(6-oxo-1,6-dihydropyridazin-4-yl)biphenyl-2-carbonitrile (2.36 g): *m*/*z* (ES⁺) 292.

2'-Fluoro-5'-(6-oxo-1,6-dihydropyridazin-4-yl)biphenyl-2-carbonitrile (2.36 g) was reacted with phosphorus oxychloride (50 ml) using the method in Example 34 to give 5'-(6-chloropyridazin-4-yl)-2'-fluoro-biphenyl-2-carbonitrile (1.89 g): *m*/*z* (ES⁺) 310.

A solution of 3,5-difluoro-4-trimethylsilylpyridine (202 mg, 1.08 mmol) in dry diethyl ether (2 ml) was added dropwise to a pre-formed solution of butyllithium (0.7 ml, 1.6M in hexanes, 1.13 mmol) and dry *N,N,N',N*'-tetramethylethylenediamine (0.2 ml, 1.35 mmol) in dry diethyl ether (2 ml) at -78°C. The yellow solution was stirred at -78°C for 1 h before dropwise addition of zinc chloride (1.6 ml, 1M in ether, 1.6 mmol). The resulting white precipitate was stirred at -78°C for 30 min then allowed to warm to room temperature over 2 h to give a solution of 3,5-difluoro-4-trimethylsilylpyridin-2-ylzinc chloride. A degassed solution of 5'-(6-chloropyridazin-4-yl)-2'-fluorobiphenyl-2-carbonitrile (200 mg, 0.65 mmol) and tetrakis(triphenylphosphine)palladium(0) (20 mg) in *N,N-*dimethylformamide (5 ml) was added to this solution and after further degassing the mixture was stirred at 80°C for 18 h. The mixture was poured into water (50 ml) and extracted with dichloromethane (2 x 50 ml). The combined organics were dried over anhydrous magnesium sulphate, filtered and evaporated to a brown oil. Purification by flash column chromatography over silica, using a mixtures of 20% ethyl acetate:80% dichloromethane as eluent, gave 5'-[6-(3,5-difluoropyridin-2-yl)pyridazin-4-yl]-2'-fluorobiphenyl-2-carbonitrile as a tan solid (80 mg): δ_{H} (400 MHz, CDCl₃) 7.41-7.48 (2H, m), 7.54-7.60 (2H, m), 7.71-7.75 (1H, m), 7.81-7.87 (3H, m), 8.35 (1H, d, *J* 2.3), 8.52 (1H, d, *J* 2.3), 9.52 (1H, d, *J* 2.3); *m*/*z* (ES⁺) 389.

### EXAMPLE 91

### 5'-[6-(3,5-Difluoropyridin-4-yl)pyridazin-4-yl]-2'-fluorobiphenyl-2-carbonitrile

5'-(6-Chloropyridazin-4-yl)-2'-fluorobiphenyl-2-carbonitrile (500 mg, 1.6 mmol) was coupled to 3,5-difluoro-4-trimethylstannanylpyridine (538 mg, 1.9 mmol) using the method in Example 100 to give 5'-[6-(3,5-difluoro-pyridin-4-yl)pyridazin-4-yl]-2'-fluorobiphenyl-2-carbonitrile as a tan solid (327 mg): δ_{H} (400 MHz, CDCl₃) 7.43-7.48 (1H, m), 7.55-7.60 (2H, m), 7.71-7.75 (1H, m), 7.78-7.89 (4H, m), 8.58 (2H, s), 9.57 (1H, d, *J* 1.6); *m*/*z* (ES⁺) 389.

### EXAMPLE 92

### 4,2'-Difluoro-5'-[6-(3,5-difluoropyridin-4-yl)pyridazin-4-yl]biphenyl-2-carbonitrile

4,2'-Difluoro-5'-(4,4,5,5-tetramethyl-[1,3,2]dioxaborolan-2-yl)-biphenyl-2-carbonitrile (5 g, 14.7 mmol) was coupled to 5-chloro-2*H-*pyridazin-3-one (1.59 g, 12.2 mmol) using the method in Example 33 to give 4,2'-difluoro-5'-(6-oxo-1,6-dihydropyridazin-4-yl)biphenyl-2-carbonitrile: *m*/*z* (ES⁺) 310.

4,2'-Difluoro-5'-(6-oxo-1,6-dihydropyridazin-4-yl)biphenyl-2-carbonitrile (4 g) was reacted with phosphorus oxychloride (20 ml) using the method in Example 34 to give 5'-(6-chloropyridazin-4-yl)-4,2'-difluoro-biphenyl-2-carbonitrile: *m*/*z* (ES⁺) 328.

5'-(6-Chloropyridazin-4-yl)-4,2'-difluorobiphenyl-2-carbonitrile (200 mg, 0.61 mmol) was coupled to 3,5-difluoro-4-trimethylstannanylpyridine (204 mg, 0.73 mmol) using the method in Example 100 to give 4,2'-difluoro-5'-[6-(3,5-difluoropyridin-4-yl)pyridazin-4-yl]biphenyl-2-carbonitrile as a tan solid (75 mg): δ_{H} (400 MHz, d⁶-DMSO) 7.65-7.70 (1H, m), 7.77-7.85 (2H, m), 8.08 (1H, dd, *J* 2.3, 8.6), 8.22-8.25 (2H, m), 8.54-8.55 (1H, m); 8.82 (2H, s), 9.90 (1H, d, *J* 2.3); *m*/*z* (ES⁺) 407.

### EXAMPLE 93

### 4,2'-Difluoro-5'-[6-(3,5-difluoropyridin-2-yl)pyridazin-4-yl]biphenyl-2-carbonitrile

5'-(6-Chloropyridazin-4-yl)-4,2'-difluorobiphenyl-2-carbonitrile (300 mg, 0.92 mmol) was coupled to 3,5-difluoro-4-trimethylsilylpyridin-2-yl-zinc chloride (1.55 mmol) using the method in Example 128 to give 4,2'-difluoro-5'-[6-(3,5-difluoropyridin-2-yl)pyridazin-4-yl]biphenyl-2-carbonitrile as a pale pink solid (88 mg): δ_{H} (400 MHz, CDCl₃) 7.42-7.48 (3H, m), 7.53-7.60 (2H, m), 7.80 (1H, dd, *J* 2.5, 6.8), 7.83-7.88 (1H, m), 8.34 (1H, d, *J* 2.0), 8.52 (1H, d, *J* 2.3), 9.52 (1H, d, *J* 2.3); *m*/*z* (ES⁺) 407.

### EXAMPLE 94

### 2-{5-[6-(3,5-Difluoropyridin-4-yl)pyridazin-4-yl]-2-fluorophenyl}-nicotinonitrile

2-[2-Fluoro-5-(4,4,5,5-tetramethyl-[1,3,2]dioxaborolan-2-yl)phenyl]-nicotinonitrile (4.86 g, 15 mmol) was coupled to 5-chloro-2*H* pyridazin-3-one (1.78 g, 13.7 mmol) using the method in Example 33 to give 2-[2-fluoro-5-(6-oxo-1,6-dihydropyridazin-4-yl)phenyl]nicotinonitrile (2.4 g): *m*/*z* (ES⁺) 293.

2-[2-Fluoro-5-(6-oxo-1,6-dihydropyridazin-4-yl)phenyl]-nicotinonitrile (2.4 g, 8.2 mmol) was reacted with phosphorus oxychloride (20 ml) using the method in Example 34 to give 2-[5-(6-chloropyridazin-4-yl)-2-fluorophenyl]nicotinonitrile (1.39 g): *m*/*z* (ES⁺) 311.

2-[5-(6-Chloropyridazin-4-yl)-2-fluorophenyl]nicotinonitrile (300 mg, 0.97 mmol) was coupled to 3,5-difluoro-4-trimethylstannanylpyridine (322 mg, 1.16 mmol) using the method in Example 100 to give 2-{5-[6-(3,5-difluoropyridin-4-yl)pyridazin-4-yl]-2-fluorophenyl)nicotinonitrile as a tan solid (95 mg): δ_{H} (400 MHz, CDCl₃) 7.46-7.55 (2H, m), 7.86-7.90 (2H, m), 7.97 (1H, dd, *J* 2.3, 6.7), 8.15 (1H, dd, *J* 1.6, 7.8), 8.58 (2H, s), 8.96 (1H, dd, *J* 1.8, 4.9), 9.58 (1H, d, *J* 2.3); *m*/*z* (ES⁺) 390.

### EXAMPLES 95

### 2-{5-[6-(3,5-Difluoropyridin-2-yl)pyridazin-4-yl]-2-fluorophenyl} nicotinonitrile

2-[5-(6-Chloropyridazin-4-yl)-2-fluorophenyl]nicotinonitrile (300 mg, 0.97 mmol) was coupled to 3,5-difluoro-4-trimethylsilylpyridin-2-ylzinc chloride (1.93 mmol) using the method in Example 128 to give 2-{5-[6-(3,5-difluoropyridin-2-yl)pyridazin-4-yl]-2-fluorophenyl}nicotinonitrile as a white solid (70 mg): δ_{H} (400 MHz, CDCl₃) 7.43-7.54 (3H, m), 7.90-7.94 (1H, m), 8.01 (1H, dd, *J* 2.3, 6.7), 8.15 (1H, dd, *J* 2.0, 7.8), 8.37 (1H, d, *J* 2.0), 8.52 (1H, d, *J* 2.3), 8.96 (1H, dd, *J* 1.8, 4.9), 9.54 (1H, d, *J* 2.3); *mlz* (ES⁺) 390.

### EXAMPLES 96

### 2'-Fluoro-5'-[6-(2-oxopyrrolidin-1-yl)pyridazin-4-yl]biphenyl-2-carbonitrile

A solution of 2-pyrrolidinone (60 mg, 0.71 mmol) was formed in *N,N*-dimethylformamide (5 ml) and cooled to 0°C. Sodium hydride (60% in mineral oil, 28 mg, approx. 0.71 mmol) was added and the suspension stirred at room temperature for 30 min. 5'-(6-Chloropyridazin-4-yl)-2'-fluorobiphenyl-2-carbonitrile (200 mg, 0.64 mmol) was added and the mixture heated at 50°C overnight. The reaction was quenched into water (50 ml) and extracted with ethyl acetate (3 x 50 ml). The combined organics were dried over anhydrous magnesium sulphate, filtered and evaporated to a brown oil. Purification by flash column chromatography over silica using a mixture of 20% ethyl acetate:80% dichloromethane as eluent, then recrystallization from dichloromethane using isohexane, gave 2'-fluoro-5'-[6-(2-oxopyrrolidin-1-yl)pyridazin-4-yl]biphenyl-2-carbonitrile as a tan solid (18 mg): δ_{H} (400 MHz, CDCl₃) 2.21.2.27 (2H, m), 2.72 (2H, t, *J* 8.1), 4.33 (2H, t, *J* 7.2), 7.40 (1H, t, *J* 9.0), 7.53-7.58 (2H, m), 7.69-7.84 (4H, m), 8.94 (1H, d, *J* 2.0), 9.23 (1H, d, *J* 2.0); *m*/*z* (ES⁺) 359.

### EXAMPLE 97

### 2'-Fluoro-5'-[6-(2-oxo-2H-pyridin-1-yl)pyridazin-4-yl]biphenyl-2-carbonitrile

A mixture of 5'-(6-chloropyridazin-4-yl)-2'-fluorobiphenyl-2-carbonitrile (310 mg, 1.0 mmol), 2-hydroxypyridine (143 mg, 1.5 mmol), copper(I) iodide (19 mg, 0.1 mmol) and potassium carbonate (207 mg, 1.5 mmol) in *N,N*-dimethylformamide were heated to 150°C overnight. Mixture was allowed to cool to room temperature then aqueous ammonia (50 ml, ~5%) was added and the mixture extracted with ethyl acetate (2 x 50 ml). The combined organics were washed with brine (50 ml) then dried over anhydrous magnesium sulphate, filtered and evaporated to a brown solid. Purification by flash column chromatography over silica using ethyl acetate as eluent, then recrystallization from ethyl acetate, gave 2'-fluoro-5'-(6-(2-oxo-2*H*-pyridin-1-yl)pyridazin-4-yl]biphenyl-2-carbonitrile: δ_{H} (400 MHz, CDCl₃) 6.38-6.42 (1H, m), 6.68 (1H, d, *J* 8.8), 7.40-7.50 (2H, m), 7.54-7.58 (2H, m), 7.70-7.74 (1H, m), 7.78-7.85 (3H, m), 8.12-8.14 (1H, m), 8.45 (1H, d, *J* 2.0), 9.45 (1H, d, *J* 2.2); *m*/*z* (ES⁺) 369.

### EXAMPLES 98

### 6,2'-Difluoro-5'-[6-(3,5-difluoropyridin-2-yl)-pyridazin-4-yl]biphenyl-2-carbonitrile

6,2'-Diffuoro-5'-(4,4,5,5-tetramethyl-[1,3,2]dioxaborolan-2-yl)-biphenyl-2-carbonitrile (5.0 g, 14.7 mmol) was coupled to 5-chloro-2*H-*pyridazin-3-one (1.74 g, 13.3 mmol) using the method in Example 33 to give 6,2'-difluoro-5'-(6-oxo-1,6-dihydropyridazin-4-yl)biphenyl-2-carbonitrile (4.2 g): *m*/*z* (ES⁺) 310.

6,2'-Difluoro-5'-(6-oxo-1,6-dihydropyridazin-4-yl)biphenyl-2-carbonitrile (4.2-g, 13.6 mmol) was reacted with phosphorus oxychloride (30 ml) using the method in Example 34 to give 5'-(6-chloropyridazin-4-yl)-6,2'-difluorobiphenyl-2-carbonitrile (2.02 g): *mlz* (ES⁺) 328.

5'-(6-Chloropyridazin-4-yl)-6,2'-difluorobiphenyl-2-carbonitrile (500 mg, 1.53 mmol) was coupled to 3,5-difluoro-4-trimethylsilylpyridin-2-yl-zinc chloride (3.05 mmol) using the method in Example 128 to give 6,2'-difluoro-5'-[6-(3,5-difluoropyridin-2-yl)pyridazin-4-yl]biphenyl-2-carbonitrile as a tan solid (100 mg): δ_{H} (400 MHz, CDCl₃) 7.42-7.51 (3H, m), 7.55-7.60 (1H, m), 7.65-7.67 (1H, m), 7.82 (1H, dd, *J* 2.3, 6.3), 7.87-7.91 (1H, m), 8.34 (1H, d, *J* 2.0), 8.52 (1H, d, *J* 2.3), 9.52 (1H, d, *J* 2.3); *m*/*z* (ES⁺) 407.

### EXAMPLE 99

### 3-(3,5-Difluoropyridin-2-yl)-5-(4-fluoro-3-trifluoromethylphenyl)pyridazine

2-(4-Fluoro-3-trifluoromethylphenyl)-4,4,5,5-tetramethyl-[1,3,2]dioxaborolane (3.0 g, 10.3 mmol) was coupled to 5-chloro-2*H-*pyridazin-3-one (1.13 g, 8.62 mmol) using the method in Example 33 to give 5-(4-fluoro-3-trifluoromethylphenyl)-2*H* pyridazin-3-one (1.93 g): *m*/*z* (ES⁺) 259.

5-(4-Fluoro-3-trifluoromethylphenyl)-2*H*-pyridazin-3-one (1.93 g, 7.48 mmol) was reacted with phosphorus oxychloride (10 ml) using the method in Example 34 to give 3-chloro-5-(4-fluoro-3-trifluoromethyl-phenyl)pyridazine (1.42 g): *m*/*z* (ES⁺) 277.

3-Chloro-5-(4-fluoro-3-trifluoromethylphenyl)pyridazine (500 mg, 1.81 mmol) was coupled to 3,5-difluoro-4-trimethylsilylpyridin-2-ylzinc chloride (3.62 mmol) using the method in Example 128 to give 3-(3,5-difluoropyridin-2-yl)-5-(4-fluoro-3-trifluoromethylphenyl)pyridazine as a light brown solid (40 mg): δ_{H} (400 MHz, CDCl₃) 7.41-7.49 (2H, m), 7.93-8.01 (2H, m), 8.34 (1H, d, *J* 2.3), 8.54 (1H, d, *J* 2.3), 9.49 (1H, d, *J* 2.0); *m*/*z* (ES⁺) 356.

### EXAMPLE 100

### 3-(3,5-Difluoropyridin-2-yl)-5-(6-fluoro-2'-trifluoromethylbiphenyl-3-yl)-pyridazine

4-Fluorophenylboronic acid was coupled to 5-chloro-2*H*-pyridazin-3-one using the method in Example 33 to give 5-(4-fluorophenyl)-2*H-*pyridazin-3-one: *mlz* (ES⁺) 191.

5-(4-Fluorophenyl)-2*H*-pyridazin-3-one was reacted with phosphorus oxychloride using the method in Example 34 to give 3-chloro-5-(4-fluorophenyl)pyridazine: *m*/*z* (ES⁺) 209.

3-Chloro-5-(4-fluorophenyl)pyridazine was coupled to 3,5-difluoro-4-trimethylsilylpyridin-2-ylzinc chloride using the method in Example 128 to give 3-(3,5-difluoropylidin-2-yl)-5-(4-fluorophenyl)pyridazine: *m*/*z* (ES⁺) 288.

3-(3,5-Difluoropyridin-2-yl)-5-(4-fluorophenyl)pyridazine (0.314 g, 1.1 mmol) was dissolved in concentrated sulfuric acid (4 ml) and 1,3-dibromo-5,5-dimethylhydantoin (0.311 g, 1.1 mmol) added and the mixtures stirred for 20 min. The reaction mixture was poured onto ice (50 g) and adjusted to pH 11 with sodium hydroxide solution. The aqueous phase was extracted with dichloromethane (2 x 100 ml), and the combined organics dried over MgSO₄, filtered and evaporated to give a brown solid. The crude product was chromatographed on silica eluting with ethyl acetate to give 5-(3-bromo-4-fluorophenyl)-3-(3,5-difluoropyridin-2-yl)-pyridazine as a white solid (0.111 g): *mlz* (ES⁺) 366:368 (1:1).

5-(3-Bromo-4-fluorophenyl)-3-(3,5-difluoropyridin-2-yl)pyridazine was coupled to 2-trifluoromethylphenylboronic acid by the method of Example 34 to give 3,(3,5-difluoropyridin-2-yl)-5-(6-fluoro-2', trifluoromethylbiphenyl-3-yl)pyridazine: δ_{H} (500 MHz, CDCl₃) 7.35 (1H, t, *J* 8.7), 7.40-7.47 (2H, m), 7.58 (1H, t, *J* 7.8), 7.63-7.69 (2H, m), 7.79-7.84 (2H, m), 8.31 (1H, d, *J* 2.0), 8.51 (1H, d, *J* 1.5), 9.49 (1H, d, *J* 1.7); *m*/*z* (ES⁺) 432.

### EXAMPLE 101

### 5-(6,2'-Difluorobiphenyl-3-yl)-3-(3,5-difluoropyridin-2-yl)pyridazine

5-(3-Bromo-4-fluorophenyl)-3-(3,5-difluoropyridin-2-yl)pyridazine was coupled to 2-fluorophenylboronic acid by the method of Example 34 to give 5-(6,2'-difluorobiphenyl-3-yl)-3-(3,5-difluoropyridin-2-yl)pyridazine: δ_{H} (500 MHz, CDCl₃) 7.21-7.24 (1H, m), 7.28-7.29 (1H, m), 7.36-7.47 (4H, m), 7.75-7.83 (2H, m), 8.33 (1H, d, *J* 2.2), 8.52 (1H, d, *J* 2.2), 9.51 (1H, d, *J* 2.2); *m*/*z* (ES⁺) 382.

### EXAMPLE 102

### 3-(3,5-Difluoropyridin-2-yl)-5-(6,2',4'-trifluorobiphenyl-3-yl)pyridazine

5-(3-Bromo-4-fluorophenyl)-3-(3,5-difluoropyridin-2-yl)pyridazine was coupled to 2,4-difluorophenylboronic acid by the method of Example 34 to give 3-(3,5-difluoropyridin-2-yl)-5-(6,2',4'-trifluorobiphenyl-3-yl)-pyridazine: δ_{H} (500 MHz, CDCl₃) 6.96-7.05 (2H, m), 7.36-7.47 (3H, m), 7.75-7.80 (2H, m), 8.33 (1H, d, *J* 2.2), 8.52 (1H, d, *J* 2.2), 9.50 (1H, d, *J* 2.4); *m*/*z* (ES⁺) 400.

### EXAMPLE 103

### 5-[3-(3,5-Difluoropyridin-2-yl)-4-fluorophenyl]-3-(2,4,6-trifluorophenyl)-pyridazine

3-Chloro-5-[3-(3,5-difluoropyridin-2-yl)-4-fluorophenyl]pyridazine was coupled to 1-bromo-2,4,6-trifluorobenzene by the method of Example 108 to give 5-[3-(3,5-difluoropyridin-2-yl)-4-fluorophenyl]-3-(2,4,6-trifluorophenyl)pyridazine: δ_{H} (500 MHz, CDCl₃) 6.86 (2H, t, *J* 8.2), 7.34-7.41 (2H, m), 7.81 (2H, t, *J* 6.4), 7.94 (1H, dd, *J* 2.2, 6.4), 8.51 (1H, d, *J* 2.2), 9.47-9.57 (1H, m); *m*/*z* (ES⁺) 418.

### EXAMPLE 104

### 3-(3,5-Difluoropyridin-2-yl)-5-[4-fluoro-3-(3-fluoropyridin-2-yl)phenyl]pyridazine

3-Chloro-5-[4-fluoro-3-(3-fluoropyridin-2-yl)phenyl]pyridazine was prepared via the coupling of 5-chloro-2*H*-pyridazin-3-one with 3-(3-fluoropyridin-2-yl)-4-fluorophenylboronic acid (prepared according to WO 0238568), according to the method of Example 126.

3-chloro-5-[4-fluoro-3-(3-fluoropyridin-2-yl)phenyl]pyridazine (0.2g, 0.66 mmol), dry dioxane (5 ml), lithium chloride (0.22g), and Copper (I) iodide (0.020g), were combined and degassed under nitrogen. 3,5-difluoro-2-trimethylstannylpyridine (0.318g; 1.11memol) was added followed by tetrakis(triphenylphosphine)palladium (0) (0.100g), the mixtures thoroughly degassed under a nitrogen atmosphere, then heated at 85°C for 48 h. The reaction was cooled to ambient temperature, diluted with dichloromethane (30 ml), and washed with 25% aqueous ammonium hydroxide (30 ml). The organic phase was separated and evaporated at reduced pressure. The residue was subjected to chromatography on silica gel eluent 5% methanol in dichloromethane. The product was dissolved in warm methanol and applied to an SCX acidic ion exchange resin cartridge. Elution with methanol, followed by a solution of ammonia in methanol (2 molar), afforded clean product, which was recrystallised from hot aqueous methanol. (400 MHz, d₆DMSO) 7.61-7.67 (2H, m), 7.95 (1H, m), 8.22-8.27 (3H, m), 8.49 (1H, d, *J* 1.8), 8.65 (1H, m), 8.77 (1H, d, *J* 1.8), 9.81 (1H, d, *J* 1.9); *m*/*z* (ES⁺) 383.

### EXAMPLE 105

### 3-(3,5-Difluoropyridin-4-yl)-5-[3-(3,5-difluoro-pyridin-2-yl)-4-fluorophenyl]pyridazine

To a degassed suspension of 5-chloro-2*H*-pyridazin-3-one (7.1198g; 54.54mmol) and 3-(3,5-difluoro-pyridin-2-yl)-4-fluorophenylboronic acid (prepared according to WO 0238568) (11.5g; 45.45mmol) in dioxan (300 g ml) was added degassed sodium carbonate solution (60 ml, 2N) followed by dichloro[1,1'-bis(diphenylphosphino)ferrocene]palladium (1.85g; 2.27mmol) mixture heated at reflux for 18 h. The mixture was cooled in an ice bath with stirring for 30 min and the solid collected by filtration washing with water and dried under vacuum over P₂O₅ to give 5-[3-(3,5-difluoro-pyridin-2-yl)-4-fluoro-phenyl]-2H-pyridazin-3-one as a beige powdery solid. (9.5 g) *m*/*z* (ES⁺) 304.

5-[3-(3,5-Difluoro-pyridin-2-yl)-4-fluoro-phenyl]-2H-pyridazin-3-one (4.5g; 14.8397mmol) was added to phosphorus oxychloride (80 ml) and the mixture placed in an oil bath pre-heated at 80°C. After stirring for 40 minutes volatiles were removed under vacuum and the brown residue treated with ice (150 g), trituration of the brown oil gave a powdery solid. The mixture was neutralised by the addition of saturated sodium hydrogen carbonate solution. The aqueous phase was extracted with ethyl acetate (3x300 ml), and the combined organics washed with brine (200 ml), dried over MgSO4, filtered and evaporated to give 3-chloro-5-[3-(3,5-difluoro-pyridin-2-yl)-4-fluoro-phenyl]-pyridazine as a beige coloured powdery solid. *m*/*z* (ES⁺) 322:324 (2:1).

To a degassed suspension of 3-chloro-5-[3-(3,5-difluoro-pyridin-2-yl)-4-fluoro-phenyl]-pyridazine (3.045g; 9.46mmol), 3,5-difluoro-4-trimethylstannylpyridine (3.15g; 11.35mmol), copper (I) iodide (0.09g ; 0.473mmol) and lithium chloride (0.80g ; 18.93mmol) in dioxan (30ml) was added tetrakis(triphenylphosphine)palladium (0) (0.5469g; 0.473mmol) and the mixture heated at 85°C for 18 h. The reaction was cooled to ambient temperature, diluted with ethyl acetate (70 ml) and filtered through a catalyst filter. The filtrate was absorbed onto silica and chromatographed eluting with ethyl acetate:iso-hexane 1:1, to give the product as an off white solid. Recrystallisation from ethyl acetate isohexane gave a white solid (3.13g). (400 MHz, CDCl₃): 7.36-7.43 (2H, m), 7.80-7.84 (1H, m), 7.89 (1H, t, *J* 1.0), 7.97 (1H, dd, *J* 2.3, 6.7), 8.51 (1H, d, *J* 2.3), 8.57 (2H, s), 9.57 (1H, d, *J* 2.3); *m*/*z* (ES⁺) 401.

### EXAMPLE 106

### 3-(3,5-Difluoropyridin-2-yl)-5-[3-(3,5-difluoropiridin-2-yl)-4-fluorophenyl]pyridazine

To a stirred solution of the 2-bromo-3,5-difluoropyridine (0.5g; 2.57mmol) in ether (5 ml) at -78°C was added n-butyllithium (1.77ml; 2.83mmol; 1.6 M in hexanes) and the mixture stirred for 15 min, zinc chloride (3.866ml; 3.8664mmol; 1M soln in ether) was added and the mixture stirred at -78°C for 15 min then allowed to warm to room temperature. 3-Chloro-5-[3-(3,5-difluoropyridin-2-yl)-4-fluorophenyl]-pyridazine (0.33g; 1.03mmol) was dissolved in DMF (6 ml) and degassed for 15 minutes, tetrakis(triphenylphosphine)palladium (0) (0.148g; 0.128mmol) was added and the mixture added to the aryl zinc prepared above and heated at 80°C for 14 h. The reaction was partitioned between water (50 ml) and DCM (100 ml). The organic phase was separated dried (MgSO₄) filtered and evaporated to give a brown oil. The product was chromatographed on silica elututed with DCM to 40% ethyl acetate in DCM to give the product as a white solid (182 mg) δ_{H} (500 MHz, DMSO) 7.61-7.65 (1H, m), 8.16-8.24 (4H, m), 8.48 (1H, s), 8.75 (2H, d, *J* 12.2), 9.79 (1H, s); *m*/*z* (ES⁺) 401.

### EXAMPLE 107

### 5-[3-(3,5-Difluoropyridin-2-yl)-4-fluorophenyl]-3-(2,4,6-trifluorophenyl)-pyridazines

To a degassed mixture of 3-chloro-5-[3-(3,5-difluoropyridin-2-yl)-4-fluorophenyl]pyridazine (0.3 g, 0.95 mmol), hexamethylditin (0.366 g, 1.1 mmol) and lithium chloride (0.116 g, 2.7 mmol) in 1,4-dioxane (12 ml) was added tetrakis(triphenylphosphine)palladium(0) (0.053 g, 0.04 mmol) and the mixture heated at 100°C for 2 h. Mass spectroscopy showed formation of the intended stannane. 1-Bromo-2,4,6-trifluorobenzene (0.393 g, 1.8 mmol) was added, followed by tetrakis(triphenylphosphine)palladium(0) (0.01 g, 0.0082 mmol), and the mixture heated at 100°C for 18 h. The reaction was cooled to room temperature, filtered and evaporated. The crude product was chromatographed on silica eluted with 30% ethyl acetate in DCM to give a brown solid, which was triturated with diethyl ether and the solid collected by filtration to give the product as an off-white solid (12 mg). δ_{H} (500 MHz, CDCl₃) 6.86 (2H, t, *J* 8.2), 7.34-7.41 (2H, m), 7.81 (2H, t, *J* 6.4), 7.94 (1H, dd, *J* 2.2, 6.4), 8.51 (1H, d, *J* 2.2), 9.47-9.57 (1H, m); *m*/*z* (ES⁺) 418.

## Claims

1. A compound of formula I, or an *N*-oxide thereof or a pharmaceutically acceptable salt thereof: 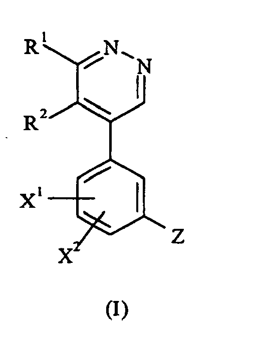 wherein
X¹ represents hydrogen, halogen, C₁₋₆ alkyl, trifluoromethyl or C₁₋₆ alkoxy;
X² represents hydrogen or halogen;
Z represents an optionally substituted aryl or heteroaryl group;
R¹ represents hydrocarbon, a heterocyclic group, -OR^{a}, -OSO₂CF₃, -SR^{a}, -NR^{a}R^{b} or -CO₂R^{a};
R² represents hydrogen or C₂₋₆ alkoxycarbonyl; and
R^{a} is C₁₋₆alkyl, C₂₋₆alkenyl, C₃₋₇cycloalkyl or aryl(C₁₋₆)alkyl (optionally substituted by C₁₋₆alkoxy);
R^{b} is hydrogen or C₁₋₆alkyl;
heteroaryl is pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl or tetrazolyl.

2. A compound as claimed in claim 1 represented by formula IIA, and *N*-oxides thereof and pharmaceutically acceptable salts thereof: 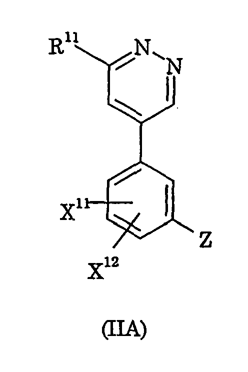 wherein
Z is as defined in claim 1;
X¹¹ represents hydrogen, fluoro, chloro, methyl, trifluoromethyl or methoxy;
X¹² represents hydrogen or fluoro; and
R¹¹ represents phenyl, halophenyl, dihalophenyl, trihalophenyl, (C₁₋₆ alkyl)(halo)phenyl, (trifluoromethyl)(halo)phenyl, C₁₋₆ alkoxyphenyl, (C₁₋₆ alkoxy)(halo)phenyl, cyanophenyl, (cyano)(halo)phenyl, C₃₋₇ heterocycloalkyl (optionally substituted by oxo), C₃₋₇ heterocycloalkenyl, heteroaryl (optionally substituted by one or more halogen atoms, and/or by oxo), C₁₋₆ alkoxy, C₂₋₆ alkenyloxy, axyl(C₁₋₆)alkoxy, triflyloxy, C₁₋₆ alkylthio, C₁₋₆ alkylamino, C₂₋₆ alkenylamino, C₃₋₇ cycloalkylamino, aryl(C₁₋₆)alkylamino (optionally substituted by C₁₋₆ alkoxy) or C₂₋₆ alkoxycarbonyl.

3. A compound as claimed in claim 2 represented by formula IIB, and *N*-oxides thereof and pharmaceutically acceptable salts thereof: 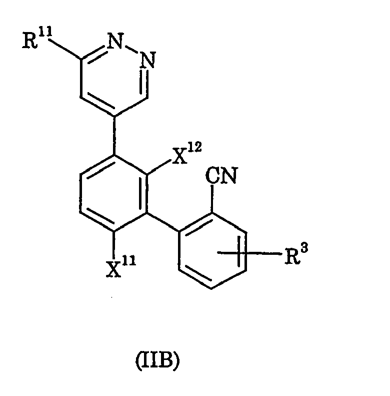 wherein X¹¹, X¹² and R¹¹ are as defined in claim 2; and
R³ represents hydrogen or fluoro.

4. A compound as claimed in claim 2 represented by formula IIC, and *N*-oxides thereof and pharmaceutically acceptable salts thereof: 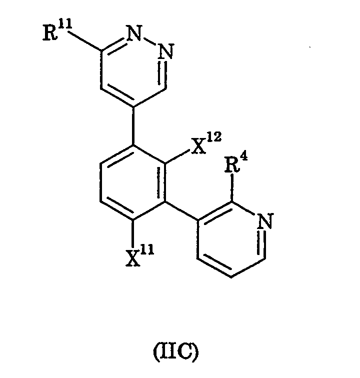 wherein X¹¹, X¹² and R¹¹ are as defined in claim 2; and
R⁴ represents hydrogen, fluoro, cyano or methyl.

5. A compound as claimed in claim 2 represented by formula IID, and *N*-oxides thereof and pharmaceutically acceptable salts thereof: 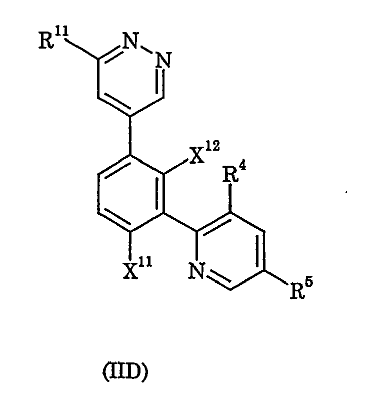 wherein X¹¹, X¹² and R¹¹ are as defined in claim 2;
R⁴ is as defined in claim 4; and
R⁵ represents hydrogen or fluoro.

6. A compound as claimed in claim 5 represented by formula IIE, and *N*-oxides thereof and pharmaceutically acceptable salts thereof: 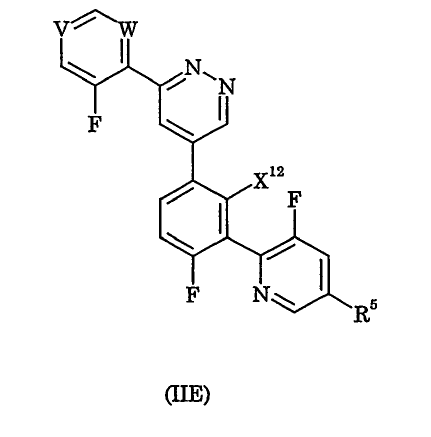 wherein
V represents N and W represents CF; or
V represents CF and W represents N; or
V and W both represent CF;
X¹² is as defined in claim 2; and
R⁵ is as defined in claim 5.

7. A compound according to any preceding claim selected from:
5-[2-fluoro-3-(pyridin-3-yl)phenyl]3-phenylpyridazine;
4,2'-difluoro-5'-(6-phenylpyridazin-4-yl)biphenyl-2-carbonitrile;
3-phenyl-5-[3-(pyridin-3-yl)phenyl]pyridazine;
3-phenyl-5-(3-[1,2,4]triazol-4-ylphenyl)pyridazine;
5-[2,4-difluoro-3-(pyridin-4-yl)phenyl]-3-phenylpyridazine;
5-[3-(2-methyl-2*H*-[1,2,4]triazol-3-ylmethoxy)phenyl]-3-phenylpyridazine;
6,2'-difluoro-5'-(6-phenylpyridazin-4-yl)biphenyl-2-carbonitrile;
5-[4-fluoro-3-(pyridin-4-yl)phenyl]-3-phenylpyridazine;
5-[4-fluoro-3-(3-fluoropyridin-2-yl)phenyl]-3-phenylpyridazine;
3-phenyl-5-[3-(pyridin-2-ylmethoxy)phenyl]pyridazine;
5-[4-fluoro-3-(3-fluoropyridin-4-yl)phenyl]-3-phenylpyridazine;
5-[2-fluoro-3-(pyridin-4-yl)phenyl]-3-phenylpyridazine;
5-[3-(3,5-difluoropyridin-2-yl)-4-fluorophenyl]-3-phenylpyridazine;
5-[4-fluoro-3-(pyridin-3-yl)phenyl]-3-phenylpyridazine;
5,2'-difluoro-5'-(6-phenylpyridazin-4-yl)biphenyl-2-carbonitrile;
3,2'-difluoro-5'-(6-phenylpyridazin-4-yl)biphenyl-2-carbonitrile;
6,2'-difluoro-5'-[6-(4-fluorophenyl)pyridazin-4-yl]biphenyl-2-carbonitrile;
4-fluoro-3'-(6-phenylpyridazin-4-yl)biphenyl-2-carbonitrile;
6,2'-difluoro-5'-[6-(thien-2-yl)pyridazin-4-yl]biphenyl-2-carbonitrile;
6,2'-difluoro-5'-[6-(4-methoxyphenyl)pyridazin-4-yl]biphenyl-2-carbonitrile;
5'-[6-(3-chlorophenyl)pyridazin-4-yl]-6,2'-difluorobiphenyl-2-carbonitrile;
6,2'-difluoro-5'-[6-(pyridin-3-yl)pyridazin-4-yl]biphenyl-2-carbonitrile;
5'-[6-(4-chlorophenyl)pyridazin-4-yl]-6,2'-difluorobiphenyl-2-carbonitrile;
6,2'-difluoro-5'-[6-(pyridin-4-yl)pyridazin-4-yl]biphenyl-2-carbonitrile;
5-[3-(3,5-difluoropyridin-2-yl)-4-fluorophenyl]-3-(4-fluorophenyl)-pyridazine;
5-[4-fluoro-3-(3-fluoropyridin-2-yl)phenyl]-3-(2-fluorophenyl)pyridazine;
5-[3-(3,5-difluoropyridin-2-yl)-4-fluorophenyl]-3-(2-fluorophenyl)-pyridazine;
5-[4-fluoro-3-(3-fluoropyridin-2-yl)phenyl]-3-(pyridin-3-yl)pyridazine;
5-[3-(3,5-difluoropyridin-2-yl),4-fluorophenyl]-3-(3-fluorophenyl)-pyridazine;
3-(2,4-difluorophenyl)-5-[3-(3,5-difluoropyridin-2-yl)-4-fluorophenyl]-pyridazine;
5-[3-(3,5-difluoropyridin-2-yl)-4-fluorophenyl]-3-(3-methoxyphenyl)-pyridazine;
6,2'-difluoro-5'-[6-(2-fluorophenyl)pyridazin-4-yl]biphenyl-2-carbonitrile;
6,2'-difluoro-5'-[6-(3-fluorophenyl)pyridazin-4-yl]biphenyl-2-carbonitrile;
5'-[6-(2-chlorophenyl)pyridazin-4-yl]-6,2'-difluorobiphenyl-2-carbonitrile;
4-fluoro-3'-[6-(4-methoxyphenyl)pyridazin-4-yl]biphenyl-2-carbonitrile;
5-[3-(3,5-difluoropyridin-2-yl)-4-fluorophenyl]-3-(4-methoxyphenyl)-pyridazine;
3-(4-chlorophenyl)-5-[3-(3,5-difluoropyridin-2-yl)-4-fluorophenyl]-pyridazine;
2-{5-[3-(3,5-difluoropyridin-2-yl)-4-fluorophenyl]pyridazin-3-yl}-5-fluorobenzonitrile;
3-(4-chlorophenyl)-5-[4-fluoro-3-(3-fluoropyridin-2-yl)phenyl]pyridazine;
5-[4-fluoro-3-(3-fluoropyridin-2-yl)phenyl]-3-(furan-3-yl)pyridazine;
5-[4-fluoro-3-(3-fluoropyridin-2-yl)phenyl]-3-(furan-2-yl)pyridazine;
3-(2,3-difluorophenyl)-5-[4-fluoro-3-(3-fluoropyridin-2-yl)phenyl]-pyridazine;
5-[4-fluoro-3-(3-fluoropyridin-2-yl)phenyl]-3-(thien-3-yl)pyridazine;
5-[4-fluoro-3-(3-fluoropyridin-2-yl)phenyl]-3-(thien-2-yl)pyridazine;
3-(2,5-difluorophenyl)-5-[4-fluoro-3-(3-fluoropyridin-2-yl)phenyl]-pyridazine;
3-(3,4-difluorophenyl)-5-[4-fluoro-3-(3-fluoropyridin-2-yl)phenyl]-pyridazine;
4-{5-[4-fluoro-3-(3-fluoropyridin-2-yl)phenyl]pyridazin-3-yl}benzonitrile;
3'-(6-ethylaminopyridazin-4-yl)-4-fluorobiphenyl-2-carbonitrile;
4-fluoro-3'-(6-isopropylaminopyridazin-4-yl)biphenyl-2-carbonitrile;
4-fluoro-3'-(6-propylaminopyridazin-4-yl)biphenyl-2-carbonitrile;
3'-(6-cyclopropylaminopyridazin-4-yl)-4-fluorobiphenyl-2-carbonitrile;
3'-(6-allylaminopyridazin-4-yl)-4-fluorobiphenyl-2-carbonitrile;
3'-(6-benzylaminopyridazin-4-yl)-4-fluorobiphenyl-2-carbonitrile;
4-fluoro-3'-(6-methylaminopyridazin-4-yl)biphenyl-2-carbonitrile;
4-fluoro-3'-(6-methoxypyridazin-4-yl)biphenyl-2-carbonitrile;
3'-(6-ethoxypyridazin-4-yl)-4-fluorobiphenyl-2-carbonitrile;
3'-(6-benzyloxypyridazin-4-yl)-4-fluorobiphenyl-2-carbonitrile;
5-[4-fluoro-3-(2-methyl-2*H*-[1,2,4]triazol-3-ylmethoxy)phenyl]-3-phenylpyridazine;
5-[4-fluoro-3-(1-methyl-3-trifluoromethyl-1*H*-pyrazol-4-ylmethoxy)phenyl]-3-phenylpyridazine;
5-[4-fluoro-3-(pyridin-4-ylmethoxy)phenyl]-3-phenylpyridazine;
5-[4-fluoro-3-(pyridin-3-ylmethoxy)phenyl]-3-phenylpyridazine;
5-[4-fluoro-3-(3-fluoropyridin-2-yl)phenyl]-3-(pyridin-4-yl)pyridazine;
5-[4-fluoro-3-(3-fluoropyridin-2-yl)phenyl]-3-(pyrazin-2-yl)pyridazine;
5-[4-fluoro-3-(3-fluoropyridin-2-yl)phenyl]-3-(thiazol-2-yl)pyridazine;
5-[4-fluoro-3-(3-fluoropyridin-2-yl)phenyl]-3-(pyridin-2-yl)pyridazine;
5-[4-fluoro-3-(3-fluoropyridin-2-yl)phenyl]-3-(3-fluoropyridin-4-yl)pyridazine;
5-[4-fluoro-3-(3-fluoropyridin-2-yl)phenyl]-3-(1*H*-[1,2,3]triazol-4-yl)pyridazine;
5-[4-fluoro-3-(3-fluoropyridin-2-yl)phenyl]pyridazine-3-carboxylic acid ethyl ester;
5-[3-(3,5-difluoropyridin-2-yl)-4-fluorophenyl]-3-(2-fluorophenyl)-pyridazine-1-oxide;
3-(2,6-difluorophenyl)-5-[4-fluoro-3-(3-fluoropyridin-2-yl)phenyl]-pyridazine;
and pharmaceutically acceptable salts thereof.

8. A compound according to any one of claims 1 to 6 selected from:
3-(4-chloro-2-fluorophenyl)-5-[3-(3,5-difluoropyridin-2-yl)-4-fluorophenyl]pyridazine;
5-[3-(3,5-difluoropyridin-2-yl)-4-fluorophenyl]-3-(2-fluoro-4-trifluoromethylphenyl)pyridazine;
5-[3-(3,5-difluoropyridin-2-yl)-4-fluorophenyl]-3-(2-fluoro-4-methylphenyl)-pyridazines;
3-(3,5-difluoropyridin-2-yl)-5-[3-(3,5-difluoropyridin-2-yl)-4-fluorophenyl]pyridazine;
trifluoromethanesulfonic acid 5-[4-fluoro-3-(3-fluoropyridin-2-yl)phenyl]pyridazin-3-yl ester;
3-ethylsulfanyl-5-[4-fluoro-3-(3-fluoropyridin-2-yl)phenyl]pyridazine;
3-*tert*-butylsulfanyl-5-[4-fluoro-3-(3-fluoropyridin-2-yl)phenyl]pyridazine;
5-[3-(3,5-difluoropyridin-2-yl)-4-fluorophenyl]-3-(3-fluoropyridin-4-yl)-pyridazine;
5-[3-(3,5-difluoropyridin-2-yl)-4-fluorophenyl]-3-(3-fluoropyridin-2-yl)-pyridazine;
5-[4-fluoro-3-(3-fluoropyridin-2-yl)phenyl]-3-(3-fluoropyridin-2-yl)-pyridazine;
5-[4-fluoro-3-(3-fluoropyridin-2-yl)phenyl]-3-(3-fluoropyridin-4-yl)-pyridazine 1-oxide;
5-[4-fluoro-3-(3-fluoropyridin-2-yl)phenyl]-3-(3-fluoro-1-oxypyridin-4-yl)-pyridazine;
5-[2,4-difluoro-3-(3,5-difluoropyridin-2-yl)phenyl]-3-(3,5-difluoropyridin-4-yl)pyridazine;
5-[3-(3,5-difluoropyridin-2-yl)-4-fluorophenyl]-3-(2-fluoro-4-methoxyphenyl)pyridazine;
5-[4-fluoro-3-(3-fluoropyridin-2-yl)phenyl]-3-(2-fluoro-4-methoxyphenyl)-pyridazine;
3-(3,5-difluoropyridin-4-yl)-5-[4-fluoro-3-(3-fluoropyridin-2-yl)phenyl]pyridazine;
3-(3,5-difluoropyridin-2-yl)-5-[4-fluoro-3-(3-fluoropyridin-2-yl)phenyl]pyridazine;
3-(3,5-difluoropyridin-4-yl)-5-[3-(3,5-difluoropyridin-2-yl)-4-fluorophenyl]pyridazine;
and pharmaceutically acceptable salts thereof.

9. A compound according to any one of claims 1 to 6 selected from:
3-(3,5-difluoro-1-oxypyridin-4-yl)-5-[3-(3,5-difluoropyridin-2-yl)-4-fluorophenyl]pyridazine;
5'-[6-(3,5-difluoropyridin-2-yl)pyridazin-4-yl]-2'-fluorobiphenyl-2-carbonitrile;
5'-[6-(3,5-difluoropyridin-4-yl)pyridazin-4-yl]-2'-fluorobiphenyl-2-carbonitrile;
4,2'-difluoro-5'-[6-(3,5-difluoropyridin-4-yl)pyridazin-4-yl]biphenyl-2-carbonitrile;
4,2'-difluoro-5'-[6-(3,5-difluoropyridin-2-yl)pyridazin-4-yl]biphenyl-2-carbonitrile;
2-{5-[6-(3,5-difluoropyridin-4-yl)pyridazin-4-yl]-2-fluorophenyl}-nicotinonitrile;
2-{5-[6-(3,5-difluoropyridin-2-yl)pyridazin-4-yl]-2-fluorophenyl}-nicotinonitrile;
2'-fluoro-5'-[6-(2-oxopyrrolidin-1-yl)pyridazin-4-yl]biphenyl-2-carbonitrile;
2'-fluoro-5'-[6-(2-oxo-2*H*-pyridin-1-yl)pyridazin-4-yl]biphenyl-2-carbonitrile;
6,2'-difluoro-5'-[6-(3,5-difluoropyridin-2-yl)pyridazin-4-yl]biphenyl-2-carbonitrile;
3-(3,5-difluoropyridin-2-yl)-5-(4-fluoro-3-trifluoromethylphenyl)pyridazine;
3-(3,5-difluoropyridin-2-yl)-5-(6-fluoro-2'-trifluoromethylbiphenyl-3-yl)-pyridazine;
5-(6,2'-difluorobiphenyl-3-yl)-3-(3,5-difluoropyridin-2-yl)pyridazine;
3-(3,5-difluoropyridin-2-yl)-5-(6,2',4'-trifluorobiphenyl-3-yl)pyridazine;
5-[3-(3,5-difluoropyridin-2-yl)-4-fluorophenyl]-3-(2,4,6-trifluorophenyl)-pyridazine;
and pharmaceutically acceptable salts thereof.

10. A pharmaceutical composition comprising a compound of formula I according to any preceding claim, or an *N*-oxide thereof or a pharmaceutically acceptable salt thereof, in association with a pharmaceutically acceptable carrier.

11. The use of a compound as defined in any one of claims 1 to 9, or an *N*-oxide thereof or a pharmaceutically acceptable salt thereof, for the manufacture of a medicament for the treatment and/or prevention of neurological disorders.

12. A process for the preparation of a compound as claimed in claim 1, which comprises:
(A) reacting a compound of formula III with a compound of formula IV: 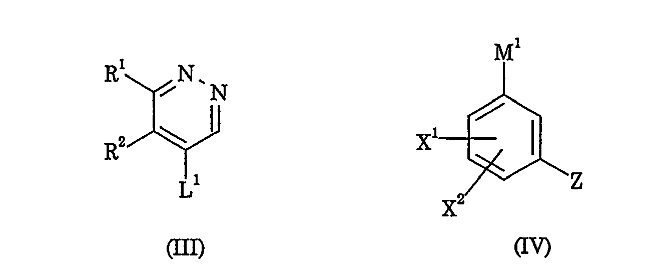 wherein X¹, X², Z, R¹ and R² are as defined in claim 1, L¹ represents a suitable leaving group, and M¹ represents a boronic acid moiety -B(OH)₂ or a cyclic ester thereof formed with an organic diol, or M¹ represents -Sn(Alk)₃ in which Alk represents C₁₋₆ alkyl, or M¹ represents -ZnHal in which Hal represents halogen; in the presence of a transition metal catalyst; or
(B) reacting a compound of formula V with a compound of formula VI: 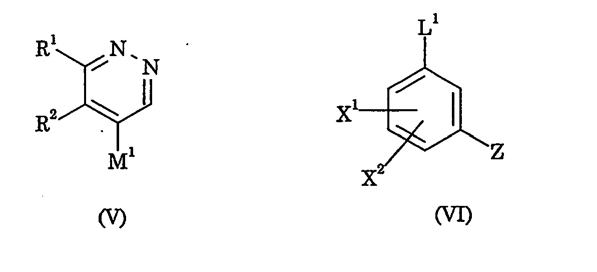 wherein X¹, X², Z, R¹ and R² are as defined in claim 1, and L¹ and M¹ are as defined above; in the presence of a transition metal catalyst; or
(C) reacting a compound of formula VII with a compound of formula VIII: 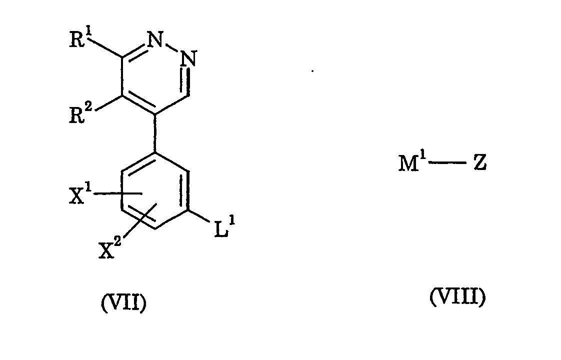 wherein X¹, X², Z, R¹ and R² are as defined in claim 1, and L¹ and M¹ are as defined above; in the presence of a transition metal catalyst; or
(D) reacting a compound of formula IX with a compound of formula X: 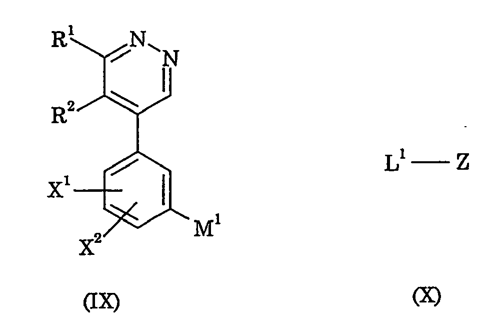 wherein X¹, X², Z, R¹ and R² are as defined in claim 1, and L¹ and M¹ are as defined above; in the presence of a transition metal catalyst; or
(E) reacting a compound of formula XI with a compound of formula XII: 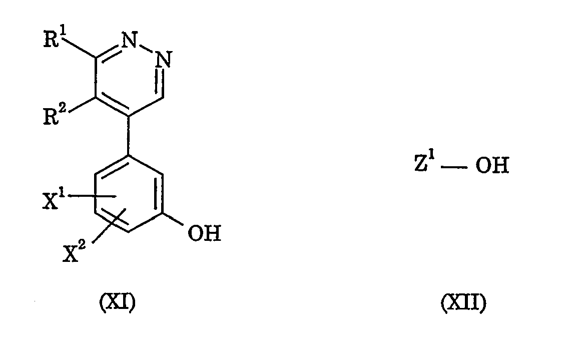 wherein X¹, X², R¹ and R² are as defined in claim 1, and Z¹ represents C₁₋₆ alkyl or optionally substituted heteroaryl(C₁₋₆)alkyl; in the presence of triphenylphosphine and a dialkyl azodicarboxylate; or
(F) reacting a compound of formula XIV with a compound of formula XV: 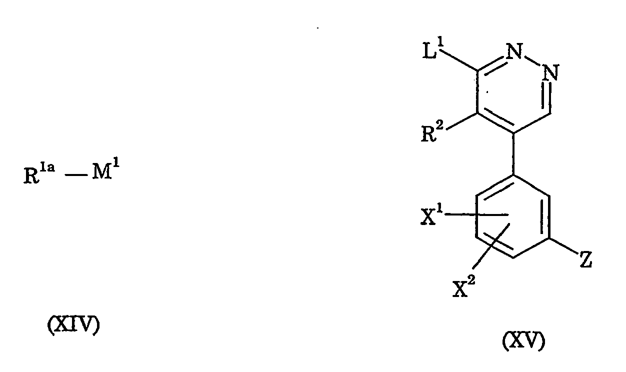 wherein X¹, X², Z and R² are as defined in claim 1, L¹ and M¹ are as defined above, and R^{1a} represents an aryl or heteroaryl moiety; in the presence of a transition metal catalyst; or
(G) reacting a compound of formula XVI with a compound of formula XVII: 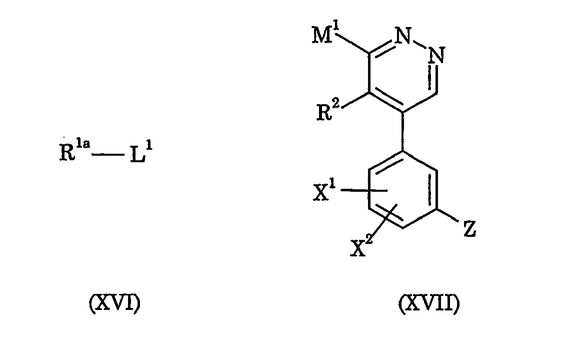 wherein X¹, X², Z and R² are as defined in claim 1, and R^{1a}, L¹ and M¹ are as defined above; in the presence of a transition metal catalyst; or
(H) reacting a compound of formula XVIII: 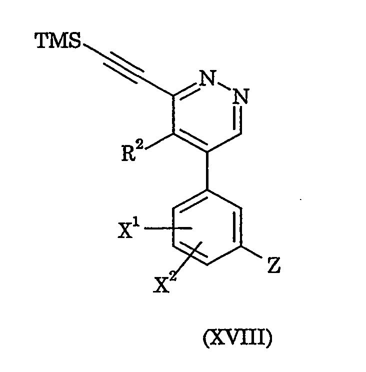 wherein X¹, X², Z and R² are as defined in claim 1, and TMS is an abbreviation for trimethylsilanyl; with sodium azide; or
(J) reacting a compound of formula XV as defined above with a compound of formula R^{a}-OH, wherein R^{a} is as defined in claim 1; or
(K) reacting a compound of formula XV as defined above with a salt of formula R^{a}S-Na⁺, wherein R^{a} is as defined in claim 1; or
(L) reacting a compound of formula XV as defined above with a compound of formula H-NR^{a}R^{b}, wherein R^{a} and R^{b} are as defined in claim 1; or
(M) reacting a compound of formula XV as defined above with carbon dioxide and a compound of formula R^{a}-OH, wherein R^{a} is as defined in claim 1; in the presence of a transition metal catalyst; or
(N) reacting a compound of formula VII above wherein L¹ represents a halogen atom with zinc cyanide; in the presence of a transition metal catalyst; or
(P) reacting a compound of formula XXII: 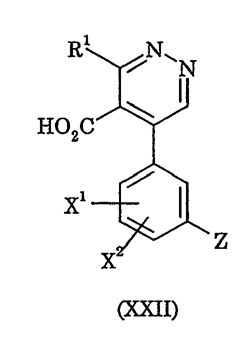 wherein X¹, X², Z and R¹ are as defined in claim 1; with diazomethane; or
(Q) reacting a compound of formula XXIII: 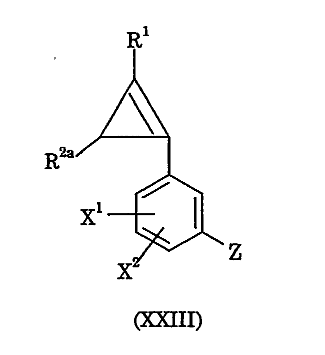 wherein X¹, X², Z and R¹ are as defined in claim 1, and R^{2a} represents C₂₋₆ alkoxycarbonyl; with diazomethane; and
(R) subsequently, if required, converting a compound of formula I initially obtained into a further compound of formula I by standard methods.

13. A compound as defined in any one of claims 1 to 9, or an *N*-oxide thereof or pharmaceutically acceptable salt thereof, for use in a method of treatment of the human body by therapy.

## Patentansprüche

1. Eine Verbindung der Formel I oder ein *N-*Oxid davon oder ein pharmazeutisch annehmbares Salz davon: 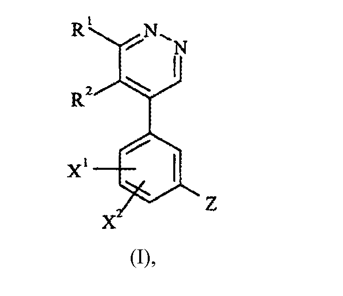 wobei
X¹ Wasserstoff, Halogen, C₁₋₆-Alkyl, Trifluormethyl oder C₁₋₆-Alkoxy bedeutet,
X² Wasserstoff oder Halogen bedeutet,
Z eine gegebenenfalls substituierte Aryl- oder Heteroarylgruppe bedeutet,
R¹ Kohlenwasserstoff, eine heterocyclische Gruppe, -OR^{a}, -OSO₂CF₃, -SR^{a}, -NR^{a}R^{b} oder -CO₂R^{a} bedeutet,
R² Wasserstoff oder C₂₋₆-Alkoxycarbonyl bedeutet und
R^{a} C₁₋₆-Alkyl, C₂₋₆-Alkenyl, C₃₋₇-Cycloalkyl oder Aryl(C₁₋₆)alkyl (gegebenenfalls substituiert durch C₁₋₆-Alkoxy) ist,
R^{b} Wasserstoff oder C₁₋₆-Alkyl ist,
Heteroaryl Pyridinyl, Chinolinyl, Isochinolinyl, Pyridazinyl, Pyrimidinyl, Pyrazinyl, Furyl, Benzofuryl, Dibenzofuryl, Thienyl, Benzthienyl, Pyrrolyl, Indolyl, Pyrazolyl, Indazolyl, Oxazolyl, Isoxazolyl, Thiazolyl, Isothiazolyl, Imidazolyl, Benzimidazolyl, Oxadiazolyl, Thiadiazolyl, Triazolyl oder Tetrazolyl ist.

2. Eine wie in Anspruch 1 beanspruchte Verbindung, dargestellt durch Formel IIA, und N Oxide davon und pharmazeutisch annehmbare Salze davon: 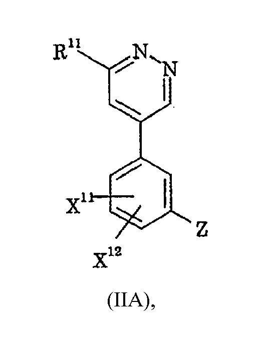 wobei
Z wie in Anspruch 1 definiert ist,
X¹¹ Wasserstoff, Fluor, Chlor, Methyl, Trifluormethyl oder Methoxy bedeutet,
X¹² Wasserstoff oder Fluor bedeutet und
R¹¹ Phenyl, Halogenphenyl, Dihalogenphenyl, Trihalogenphenyl, (C₁₋₆-Alkyl)(halogen)phenyl, (Trifluormethyl)(halogen)phenyl, C₁₋₆-Alkoxyphenyl, (C₁₋₆-Alkoxy)(halogen)phenyl, Cyanophenyl, (Cyano)(halogen)phenyl, C₃₋₇-Heterocycloalkyl (gegebenenfalls substituiert durch Oxo), C₃₋₇-Heterocycloalkenyl, Heteroaryl (gegebenenfalls substituiert durch ein oder mehrere Halogenatome und/oder durch Oxo), C₁₋₆-Alkoxy, C₂₋₆-Alkenyloxy, Aryl(C₁₋₆)alkoxy, Triflyloxy, C₁₋₆-Alkylthio, C₁₋₆-Alkylamino, C₂₋₆-Alkenylamino, C₃₋₇-Cycloalkylamino, Aryl(C₁₋₆₎alkylamino (gegebenenfalls substituiert durch C₁₋₆-Alkoxy) oder C₂₋₆-Alkoxycarbonyl bedeutet.

3. Eine wie in Anspruch 2 beanspruchte Verbindung, dargestellt durch Formel (IIB), und *N-*Oxide davon und pharmazeutisch annehmbare Salze davon: 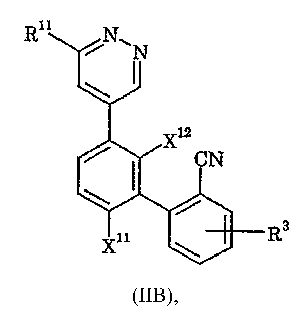 wobei X¹¹, X¹² und R¹¹ wie in Anspruch 2 definiert sind und
R³ Wasserstoff oder Fluor bedeutet.

4. Eine wie in Anspruch 2 beanspruchte Verbindung, dargestellt durch Formel IIC, und *N-*Oxide davon und pharmazeutisch annehmbare Salze davon: 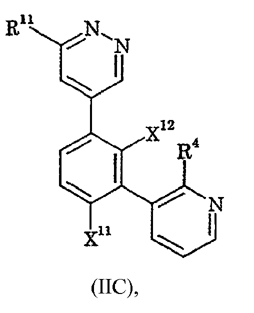 wobei X¹¹, X¹² und R¹¹ wie in Anspruch 2 definiert sind und
R⁴ Wasserstoff, Fluor, Cyano oder Methyl bedeutet.

5. Eine wie in Anspruch 2 beanspruchte Verbindung, dargestellt durch Formel IID, und *N-*Oxide davon und pharmazeutisch annehmbare Salze davon: 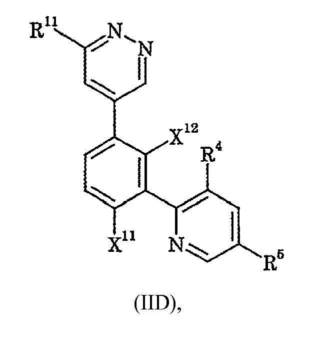 wobei X¹¹, X¹² und R¹¹ wie in Anspruch 2 definiert sind,
R⁴ wie in Anspruch 4 definiert ist und
R⁵ Wasserstoff oder Fluor bedeutet.

6. Eine wie in Anspruch 5 beanspruchte Verbindung, dargestellt durch Formel IIE, und *N-*Oxide davon und pharmazeutisch annehmbare Salze davon: 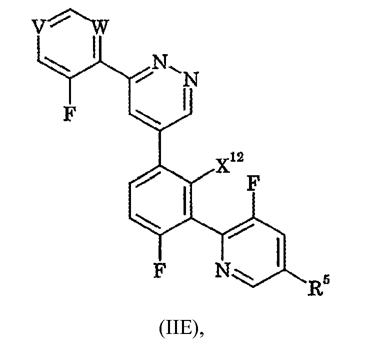 wobei
V N bedeutet und W CF bedeutet, oder
V CF bedeutet und W N bedeutet, oder
V und W beide CF bedeuten,
X¹² wie in Anspruch 2 definiert ist und
R⁵ wie in Anspruch 5 definiert ist.

7. Eine Verbindung gemäß irgendeinem vorhergehenden Anspruch, ausgewählt aus
5-[2-Fluor-3-(pyridin-3-yl)phenyl]-3-phenylpyridazin,
4,2'-Difluor-5'-(6-phenylpyridazin-4-yl)biphenyl-2-carbonitril,
3-Phenyl-5-[3-(pyridin-3-yl)phenyl]pyridazin,
3-Phenyl-5-(3-[1,2,4]triazol-4-ylphenyl)pyridazin,
5-[2,4-Difluor-3-(pyridin-4-yl)phenyl]-3-phenylpyridazin,
5-[3-(2-Methyl-2H-[1,2,4]triazol-3-ylmethoxy)phenyl]-3-phenylpyridazin,
6,2'-Difluor-5'-(6-phenylpyridazin-4-yl)biphenyl-2-carbonitril,
5-[4-Fluor-3-(pyridin-4-yl)phenyl]-3-phenylpyridazin,
5-[4-Fluor-3-(3-fluorpyridin-2-yl)phenyl]-3-phenylpyridazin,
3-Phenyl-5-[3-(pyridin-2-ylmethoxy)phenyl]pyridazin,
5-[4-Fluor-3-(3-fluorpyridin-4-yl)phenyl]-3-phenylpyridazin,
5-[2-Fluor-3-(pyridin-4-yl)phenyl]-3-phenylpyridazin,
5-[3-(3,5-Difluorpyridin-2-yl)-4-fluorphenyl]-3-phenylpyridazin,
5-[4-Fluor-3-(pyridin-3-yl)phenyl]-3-phenylpyridazin,
5,2'-Difluor-5'-(6-phenylpyridazin-4-yl)biphenyl-2-carbonitril,
3,2'-Difluor-5'-(6-phenylpyridazin-4-yl)biphenyl-2-carbonitril,
6,2'-Difluor-5'-[6-(4-fluorphenyl)pyridazin-4-yl]biphenyl-2-carbonitril,
4-Fluor-3'-(6-phenylpyridazin-4-yl)biphenyl-2-carbonitril,
6,2'-Difluor-5'-[6-(thien-2-yl)pyridazin-4-yl]biphenyl-2-carbonitril,
6,2'-Difluor-5'-[6-(4-methoxyphenyl)pyridazin-4-yl]biphenyl-2-carbonitril,
5'-[6-(3-Chlorphenyl)pyridazin-4-yl]-6,2'-difluorbiphenyl-2-carbonitril,
6,2'-Difluor-5'-[6-(pyridin-3-yl)pyridazin-4-yl]biphenyl-2-carbonitril,
5'-[6-(4-Chlorphenyl)pyridazin-4-yl]-6,2'-difluorbiphenyl-2-carbonitril,
6,2'-Difluor-5'-[6-(pyridin-4-yl)pyridazin-4-yl]biphenyl-2-carbonitril,
5-[3-(3,5-Difluorpyridin-2-yl)-4-fluorphenyl]-3-(4-fluorphenyl)pyridazin,
5-[4-Fluor-3-(3-fluorpyridin-2-yl)phenyl]-3-(2-fluorphenyl)pyridazin,
5-[3-(3,5-Difluorpyridin-2-yl)-4-fluorphenyl]-3-(2-fluorphenyl)pyridazin,
5-[4-Fluor-3-(3-fluorpyridin-2-yl)phenyl]-3-(pyridin-3-yl)pyridazin,
5-[3-(3,5-Difluorpyridin-2-yl)-4-fluorphenyl]-3-(3-fluorphenyl)pyridazin,
3-(2,4-Difluorphenyl)-5-[3-(3,5-difluorpyridin-2-yl)-4-fluorphenyl]pyridazin,
5-[3-(3,5-Difluorpyridin-2-yl)-4-fluorphenyl]-3-(3-methoxyphenyl)pyridazin,
6,2'-Difluor-5'-[6-(2-fluorphenyl)pyridazin-4-yl]biphenyl-2-carbonitril,
6,2'-Difluor-5'-[6-(3-fluorphenyl)pyridazin-4-yl]biphenyl-2-carbonitril,
5'-[6-(2-Chlorphenyl)pyridazin-4-yl]-6,2'-difluorbiphenyl-2-carbonitril,
4-Fluor-3'-[6-(4-methoxyphenyl)pyridazin-4-yl]biphenyl-2-carbonitril,
5-[3-(3,5-Difluorpyridin-2-yl)-4-fluorphenyl]-3-(4-methoxyphenyl)pyridazin,
3-(4-Chlorphenyl)-5-[3-(3,5-difluorpyridin-2-yl)-4-fluorphenyl]pyridazin,
2-{5-[3-(3,5-Difluorpyridin-2-yl)-4-fluorphenyl]pyridazin-3-yl}-5-fluorbenzonitril,
3-(4-Chlorphenyl)-5-[4-fluor-3-(3-fluorpyridin-2-yl)phenyl]pyridazin,
5-[4-Fluor-3-(3-fluorpyridin-2-yl)phenyl]-3-(furan-3-yl)pyridazin, 5-[4-Fluor-3-(3-fluorpyridin-2-yl)phenyl]-3-(furan-2-yl)pyridazin,
3-(2,3-Difluorphenyl)-5-[4-fluor-3-(3-fluorpyridin-2-yl)phenyl]pyridazin,
5-[4-Fluor-3-(3-fluorpyridin-2-yl)phenyl]-3-(thien-3-yl)pyridazin,
5-[4-Fluor-3-(3-fluorpyridin-2-yl)phenyl]-3-(thien-2-yl)pyridazin,
3-(2,5-Difluorphenyl)-5-[4-fluor-3-(3-fluorpyridin-2-yl)phenyl]pyridazin,
3-(3,4-Difluorphenyl)-5-[4-fluor-3-(3-fluorpyridin-2-yl)phenyl]pyridazin,
4-{5-[4-Fluor-3-(3-fluorpyridin-2-yl)phenyl]pyridazin-3-yl}benzonitril,
3'-(6-Ethylaminopyridazin-4-yl)-4-fluorbiphenyl-2-carbonitril,
4-Fluor-3'-(6-isopropylaminopyridazin-4-yl)biphenyl-2-carbonitril,
4-Fluor-3'-(6-propylaminopyridazin-4-yl)biphenyl-2-carbonitril,
3'-(6-Cyclopropylaminopyridazin-4-yl)-4-fluorbiphenyl-2-carbonitril,
3'-(6-Allylaminopyridazin-4-yl)-4-fluorbiphenyl-2-carbonitril,
3'-(6-Benzylaminopyridazin-4-yl)-4-fluorbiphenyl-2-carbonitril,
4-Fluor-3'-(6-methylaminopyridazin-4-yl)biphenyl-2-carbonitril,
4-Fluor-3'-(6-methoxypyridazin-4-yl)biphenyl-2-carbonitril,
3'-(6-Ethoxypyridazin-4-yl)-4-fluorbiphenyl-2-carbonitril,
3'-(6-Benzyloxypyridazin-4-yl)-4-fluorbiphenyl-2-carbonitril,
5-[4-Fluor-3-(2-methyl-2*H*-[1,2,4]triazol-3-ylmethoxy)phenyl]-3-phenylpyridazin,
5-[4-Fluor-3-(1-methyl-3-trifluormethyl-1*H-*pyrazol-4-ylmethoxy)phenyl]-3-phenylpyridazin,
5-[4-Fluor-3-(pyridin-4-ylmethoxy)phenyl]-3-phenylpyridazin,
5-[4-Fluor-3-(pyridin-3-ylmethoxy)phenyl]-3-phenylpyridazin,
5-[4-Fluor-3-(3-fluorpyridin-2-yl)phenyl]-3-(pyridin-4-yl)pyridazin,
5-[4-Fluor-3-(3-fluorpyridin-2-yl)phenyl]-3-(pyridazin-2-yl)pyridazin,
5-[4-Fluor-3-(3-fluorpyridin-2-yl)phenyl]-3-(thiazol-2-yl)pyridazin,
5-[4-Fluor-3-(3-fluorpyridin-2-yl)phenyl]-3-(pyridin-2-yl)pyridazin,
5-[4-Fluor-3-(3-fluorpyridin-2-yl)phenyl]-3-(3-fluorpyridin-4-yl)pyridazin,
5-[4-Fluor-3-(3-fluorpyridin-2-yl)phenyl]-3-(1*H-*[1,2,3]triazol-4-yl)pyridazin,
5-[4-Fluor-3-(3-fluorpyridin-2-yl)phenyl]pyridazin-3-carbonsäureethylester,
5-[3-(3,5-Difluorpyridin-2-yl)-4-fluorphenyl]-3-(2-fluorphenyl)pyridazin-1-oxid,
3-(2,6-Difluorphenyl)-5-[4-fluor-3-(3-fluorpyridin-2-yl)phenyl]pyridazin,
und pharmazeutisch annehmbare Salze davon.

8. Eine Verbindung gemäß irgendeinem der Ansprüche 1 bis 6, ausgewählt aus:
3-(4-Chlor-2-fluorphenyl)-5-[3-(3,5-difluorpyridin-2-yl)-4-fluorphenyl]pyridazin,
5-[3-(3,5-Difluorpyridin-2-yl)-4-fluorphenyl]-3-(2-fluor-4-trifluormethylphenyl)pyridazin,
5-[3-(3,5-Difluorpyridin-2-yl)-4-fluorphenyl]-3-(2-fluor-4-methylphenyl)pyridazin,
3-(3,5-Difluorpyridin-2-yl)-5-[3-(3,5-difluorpyridin-2-yl)-4-fluorphenyl]pyridazin,
Trifluormethansulfonsäure-5-[4-fluor-3-(3-fluorpyridin-2-yl)phenyl]pyridazin-3-ylester,
3-Ethylsulfanyl-5-[4-fluor-3-(3-fluorpyridin-2-yl)phenyl]pyridazin,
3-*tert*.-Butylsulfanyl-5-[4-fluor-3-(3-fluorpyridin-2-yl)phenyl]pyridazin,
5-[3-(3,5-Difluorpyridin-2-yl)-4-fluorphenyl]-3-(3-fluorpyridin-4-yl)pyridazin,
5-[3-(3,5-Difluorpyridin-2-yl)-4-fluorphenyl]-3-(3-fluorpyridin-2-yl)pyridazin,
5-[4-Fluor-3-(3-fluorpyridin-2-yl)phenyl]-3-(3-fluorpyridin-2-yl)pyridazin,
5-[4-Fluor-3-(3-fluorpyridin-2-yl)phenyl]-3-(3-fluorpyridin-4-yl)pyridazin-1-oxid,
5-[4-Fluor-3-(3-fluorpyridin-2-yl)phenyl]-3-(3-fluor-1-oxypyridin-4-yl)pyridazin,
5-[2,4-Difluor-3-(3,5-difluorpyridin-2-yl)phenyl]-3-(3,5-difluorpyridin-4-yl)pyridazin,
5-[3-(3,5-Difluorpyridin-2-yl)-4-fluorphenyl]-3-(2-fluor-4-methoxyphenyl)pyridazin,
5-[4-Fluor-3-(3-fluorpyridin-2-yl)phenyl]-3-(2-fluor-4-methoxyphenyl)pyridazin,
3-(3,5-Difluorpyridin-4-yl)-5-[4-fluor-3-(3-fluorpyridin-2-yl)phenyl]pyridazin,
3-(3,5-Difluorpyridin-2-yl)-5-[4-fluor-3-fluorpyridin-2-yl)phenyl]pyridazin,
3-(3,5-Difluorpyridin-4-yl)-5-[3-(3,5-difluorpyridin-2-yl)-4-fluorphenyl]pyridazin,
und pharmazeutisch annehmbare Salze davon.

9. Eine Verbindung gemäß irgendeinem der Ansprüche 1 bis 6, ausgewählt aus:
3-(3,5-Difluor-1-oxypyridin-4-yl)-5-[3-(3,5-difluorpyridin-2-yl)-4-fluorphenyl]pyridazin,
5'-[6-(3,5-Difluorpyridin-2-yl)pyridazin-4-yl]-2'-fluorbiphenyl-2-carbonitril,
5'-[6-(3,5-Difluorpyridin-4-yl)pyridazin-4-yl]-2'-fluorbiphenyl-2-carbonitril,
4,2'-Difluor-5'-[6-(3,5-difluorpyridin-4-yl)pyridazin-4-yl]biphenyl-2-carbonitril,
4,2'-Difluor-5'-[6-(3,5-difluorpyridin-2-yl)pyridazin-4-yl]biphenyl-2-carbonitril,
2-{5-[6-(3,5-Difluorpyridin-4-yl)pyridazin-4-yl]-2-fluorphenyl{nicotinonitril,
2-{5-[6-(3,5-Difluorpyridin-2-yl)pyridazin-4-yl]-2-fluorphenyl{nicotinonitril,
2'-Fluor-5'-[6-(2-oxopyrrolidin-1-yl)pyridazin-4-yl]biphenyl-2-carbonitril,
2'-Fluor-5'-[6-(2-oxo-2*H*-pyridin-1-yl)pyridazin-4-yl]biphenyl-2-carbonitril,
6,2'-Difluor-5'-[6-(3,5-difluorpyridin-2-yl)pyridazin-4-yl]biphenyl-2-carbonitril,
3-(3,5-Difluorpyridin-2-yl)-5-(4-fluor-3-trifluormethylphenyl)pyridazin,
3-(3,5-Difluorpyridin-2-yl)-5-(6-fluor-2'-trifluormethylbiphenyl-3-yl)pyridazin,
5-(6,2'-Difluorbiphenyl-3-yl)-3-(3,5-difluorpyridin-2-yl)pyridazin,
3-(3,5-Difluorpyridin-2-yl)-5-(6,2',4'-trifluorbiphenyl-3-yl)pyridazin,
5-[3-(3,5-Difluorpyridin-2-yl)-4-fluorphenyl]-3-(2,4,6-trifluorphenyl)pyridazin,
und pharmazeutisch annehmbare Salze davon.

10. Eine pharmazeutische Zusammensetzung, die eine Verbindung der Formel I gemäß irgendeinem vorhergehenden Anspruch oder ein *N*-Oxid davon oder ein pharmazeutisch annehmbares Salz davon enthält, in Verbindung mit einem pharmazeutisch annehmbaren Träger.

11. Die Verwendung einer wie in irgendeinem der Ansprüche 1 bis 9 definierten Verbindung oder eines *N*-Oxids davon oder eines pharmazeutisch annehmbaren Salzes davon zur Herstellung eines Medikaments zur Behandlung und/oder Prävention von neurologischen Störungen.

12. Ein Verfahren zur Herstellung einer wie in Anspruch 1 beanspruchten Verbindung, das umfasst:
(A) Umsetzung einer Verbindung der Formel III mit einer Verbindung der Formel IV: 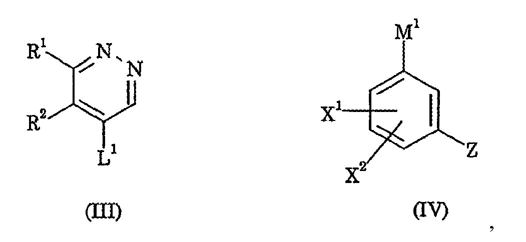 wobei X¹, X², Z, R¹ und R² wie in Anspruch 1 definiert sind, L¹ eine geeignete Abgangsgruppe bedeutet und M¹ einen Boronsäurerest -B(OH)₂ oder einen cyclischen Ester davon, gebildet mit einem organischen Diol, bedeutet oder M¹ -SN(Alk)₃ bedeutet, wobei Alk C₁₋₆-Alkyl bedeutet, oder M¹ -ZnHal bedeutet, wobei Hal Halogen bedeutet, in Gegenwart eines Übergangsmetallkatalysators, oder
(B) Umsetzung einer Verbindung der Formel V mit einer Verbindung der Formel VI: 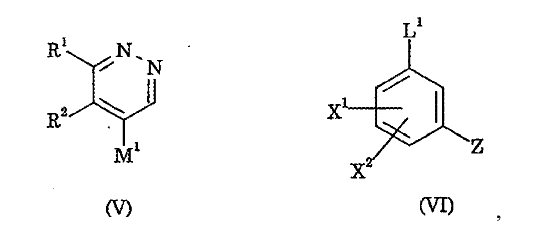 wobei X¹, X², Z, R¹ und R² wie in Anspruch 1 definiert sind und L¹ und M¹ wie oben definiert sind, in Gegenwart eines Übergangsmetallkatalysators, oder
(C) Umsetzung einer Verbindung der Formel VII mit einer Verbindung der Formel VIII: 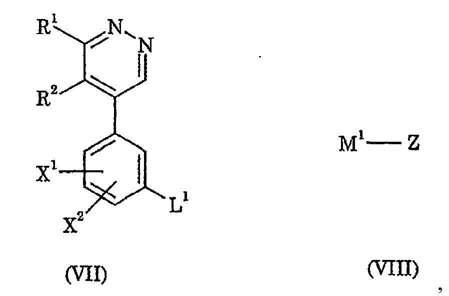 wobei X¹, X², Z, R¹ und R² wie in Anspruch 1 definiert sind und L¹ und M¹ wie oben definiert sind, in Gegenwart eines Übergangsmetallkatalysators, oder
(D) Umsetzung einer Verbindung der Formel IX mit einer Verbindung der Formel X: 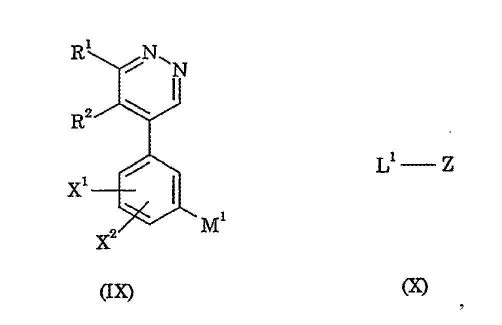 wobei X¹, X², Z, R¹ und R² wie in Anspruch 1 definiert sind und L¹ und M¹ wie oben definiert sind, in Gegenwart eines Übergangsmetallkatalysators, oder
(E) Umsetzung einer Verbindung der Formel XI mit einer Verbindung der Formel XII: 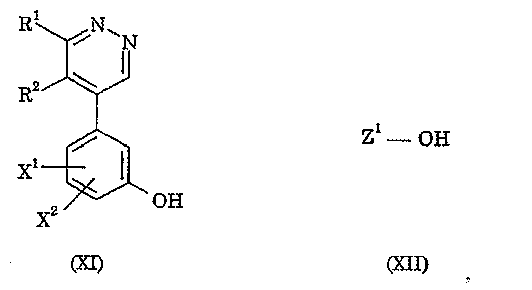 wobei X¹, X², R¹ und R² wie in Anspruch 1 definiert sind und Z¹ C₁₋₆-Alkyl oder gegebenenfalls substituiertes Heteroaryl(C₁₋₆)alkyl bedeutet, in Gegenwart von Triphenylphosphin und einem Dialkylazodicarboxylat, oder
(F) Umsetzung einer Verbindung der Formel XIV mit einer Verbindung der Formel XV: 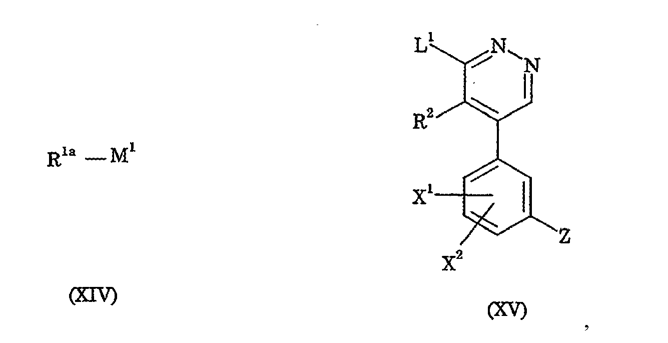 wobei X¹, X², Z und R² wie in Anspruch 1 definiert sind, L¹ und M¹ wie oben definiert sind und R^{1a} einen Aryl- oder Heteroarylrest bedeutet, in Gegenwart eines Übergangsmetallkatalysators, oder
(G) Umsetzung einer Verbindung der Formel XVI mit einer Verbindung der Formel XVII: 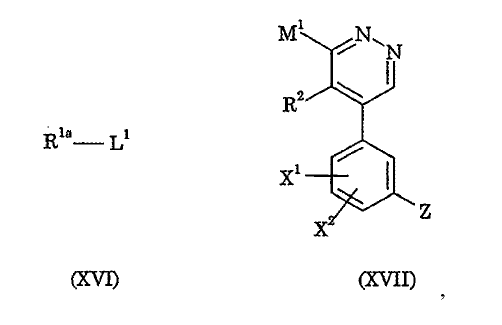 wobei X¹, X², Z und R² wie in Anspruch 1 definiert sind und R^{1a}, L¹ und M¹ wie oben definiert sind, in Gegenwart eines Übergangsmetallkatalysators, oder
(H) Umsetzung einer Verbindung der Formel XVIII: 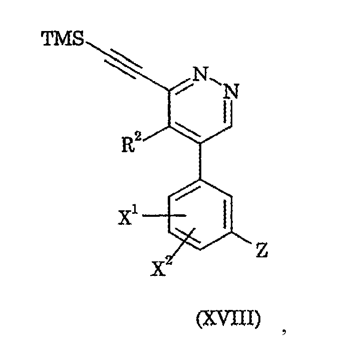 wobei X¹, X², Z und R² wie in Anspruch 1 definiert sind und TMS eine Abkürzung für Trimethylsilanyl ist, mit Natriumazid, oder
(J) Umsetzung einer wie oben definierten Verbindung der Formel XV mit einer Verbindung der Formel R^{a}-OH, wobei R^{a} wie in Anspruch 1 definiert ist, oder
(K) Umsetzung einer wie oben definierten Verbindung der Formel XV mit einem Salz der Formel R^{a}S⁻Na⁺, wobei R^{a} wie in Anspruch 1 definiert ist, oder
(L) Umsetzung einer wie oben definierten Verbindung der Formel XV mit einer Verbindung der Formel H-NR^{a}R^{b}, wobei R^{a} und R^{b} wie in Anspruch 1 definiert sind, oder
(M) Umsetzung einer wie oben definierten Verbindung der Formel XV mit Kohlendioxid und einer Verbindung der Formel R^{a}-OH, wobei R^{a} wie in Anspruch 1 definiert ist, in Gegenwart eines Übergangsmetallkatalysators, oder
(N) Umsetzung einer Verbindung der obigen Formel VII, wobei L¹ ein Halogenatom bedeutet, mit Zinkcyanid in Gegenwart eines Übergangsmetallkatalysators, oder
(P) Umsetzung einer Verbindung der Formel XXII: 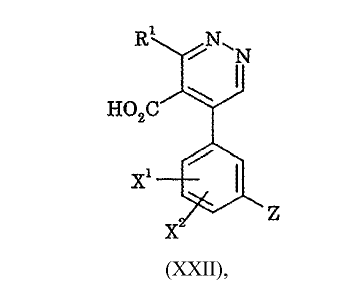 wobei X¹, X², Z und R¹ wie in Anspruch 1 definiert sind, mit Diazomethan, oder
(Q) Umsetzung einer Verbindung der Formel XXIII: 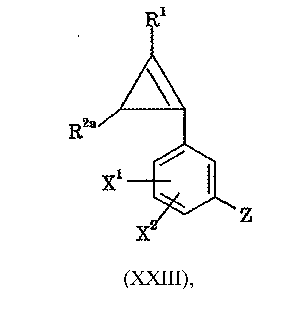 wobei X¹, X², Z und R¹ wie in Anspruch 1 definiert sind und R^{2a} C₂₋₆-Alkoxycarbonyl bedeutet, mit Diazomethan, und
(R) anschließend, falls erforderlich, Umwandlung einer ursprünglich erhaltenen Verbindung der Formel I durch Standardverfahren in eine weitere Verbindung der Formel I.

13. Eine wie in irgendeinem der Ansprüche 1 bis 9 definierte Verbindung oder ein N-Oxid davon oder ein pharmazeutisch annehmbares Salz davon zur Verwendung bei einem Verfahren zur Behandlung des menschlichen Körpers durch Therapie.

## Revendications

1. Composé de la formule I, ou N-oxyde de celui-ci ou sel pharmaceutiquement acceptable de celui-ci: 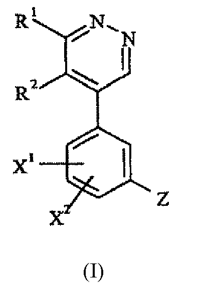 dans laquelle:
X¹ représente un hydrogène, un halogène, un groupe alkyle C₁₋₆, trifluorométhyle ou alcoxy C₁₋₆;
X² représente un hydrogène ou un halogène;
Z représente un groupe aryle ou hétéroaryle optionnellement substitué;
R¹ représente un hydrocarbure, un groupe hétérocyclique, -OR^{a}, -OSO₂CF₃, -SR^{a}, -NR^{a}R^{b} ou - CO₂R^{a};
R² représente un hydrogène ou un groupe alcoxycarbonyle C₂₋₆; et
R^{a} est un groupe alkyle C₁₋₆, alcényle C₂₋₆, cycloalkyle C₃₋₇ ou aryl-alkyle C₁₋₆ (optionnellement substitué par un groupe alcoxy C₁₋₆);
R^{b} est un hydrogène ou un groupe alkyle C₁₋₆ ;
un groupe hétéroaryle est un groupe pyridinyle, quinoléinyle, isoquinoléinyle, pyridazinyle, pyrimidinyle, pyrazinyle, furyle, benzofuryle, dibenzofuryle, thiényle, benzthiényle, pyrrolyle, indolyle, pyrazolyle, indazolyle, oxazolyle, isoxazolyle, thiazolyle, isothiazolyle, imidazolyle, benzimidazolyle, oxadiazolyle, thiadiazolyle, triazolyle ou tétrazolyle.

2. Composé selon la revendication 1, représenté par la formule IIA, et N-oxydes de celui-ci et sels pharmaceutiquement acceptables de celui-ci: 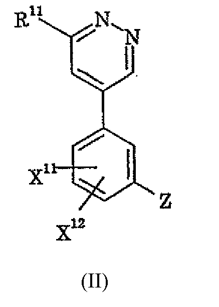 dans laquelle:
Z est tel que défini dans la revendication 1;
X¹¹ représente un hydrogène, un groupe fluoro, chloro, méthyle, trifluorométhyle ou méthoxy;
X¹² représente un hydrogène ou un groupe fluoro; et
R¹¹ représente un groupe phényle, halophényle, dihalophényle, trihalophényle, (alkyle C₁₋₆)-(halo)phényle, (trifluorométhyl)(halo)phényle, (alcoxy C₁₋₆)-phényle, (alcoxy C₁₋₆)-(halo)phényle, cyanophényle, (cyano)(halo)phényle, hétérocycloalkyle C₃₋₇ (optionnellement substitué par un groupe oxo), hétérocycloalcényle C₃₋₇, hétéroaryle (optionnellement substitué par un ou plusieurs atomes d'halogène et/ou par un groupe oxo), alcoxy C₁₋₆, alcényloxy C₂₋₆, aryl-alcoxy C₁₋₆, triflyloxy, alkylthio C_{1-6,} alkylamino C₁₋₆, alcénylamino C₂₋₆, cycloalkylamino C₃₋₇, aryl-alkylamino C₁₋₆ (optionnellement substitué par un groupe alcoxy C₁₋₆) ou alcoxycarbonyle C₂₋₆.

3. Composé selon la revendication 2, représenté par la formule IIB, et N-oxydes de celui-ci et sels pharmaceutiquement acceptables de celui-ci: 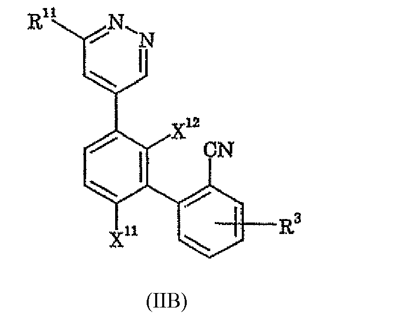 dans laquelle X¹¹, X¹² et R¹¹ sont tels que définis dans la revendication 2; et
R³ représente un hydrogène ou un groupe fluoro.

4. Composé selon la revendication 2, représenté par la formule IIC, et N-oxydes de celui-ci et sels pharmaceutiquement acceptables de celui-ci: 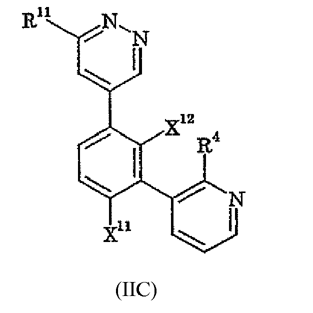 dans laquelle X¹¹, X¹² et R¹¹ sont tels que définis dans la revendication 2; et
R⁴ représente un hydrogène, un groupe fluoro, cyano ou méthyle.

5. Composé selon la revendication 2, représenté par la formule IID, et N-oxydes de celui-ci et sels pharmaceutiquement acceptables de celui-ci: 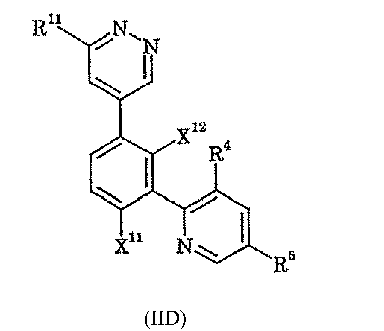 dans laquelle X¹¹, X¹² et R¹¹ sont tels que définis dans la revendication 2; et
R⁴ est tel que défini dans la revendication 4; et
R⁵ représente un hydrogène ou un groupe fluoro.

6. Composé selon la revendication 5, représenté par la formule IIE, et N-oxydes de celui-ci et sels pharmaceutiquement acceptables de celui-ci: 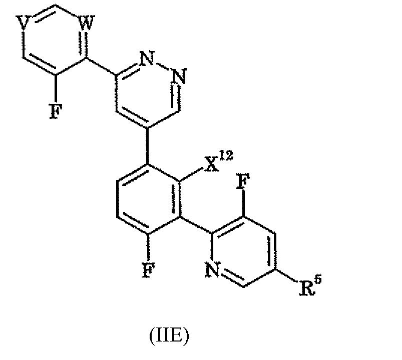 dans laquelle:
V représente N et W représente CF; ou
V représente CF et W représente N; ou
V et W représentent tous les deux CF;
X¹² est tel que défini dans la revendication 2; et
R⁵ est tel que défini dans la revendication 5.

7. Composé selon l'une quelconque des revendications précédentes, choisi parmi:
la 5-[2-fluoro-3-(pyridin-3-yl)phényl]-3-phénylpyridazine;
le 4,2'-difluoro-5'-(6-phénylpyridazin-4-yl)biphényl-2-carbonitrile;
la 3-phényl-5-[3-(pyridin-3-yl)phényl]pyridazine;
la 3-phényl-5-(3-[1,2,4]triazol-4-ylphényl)pyridazine;
la 5-[2,4-difluoro-3-(pyridin-4-yl)phényl]-3-phénylpyridazine;
la 5-[3-(2-méthyl-2H-[1,2,4]triazol-3ylméthoxy)phényl]-3-phénylpyridazine;
le 6,2'-difluoro-5'-(6-phénylpyridazin-4-yl)biphényl-2-carbonitrile;
la 5-[4-fluoro-3-(pyridin-4-yl)phényl]-3-phénylpyridazine;
la 5-[4-fluoro-3-(3-fluoropyridin-2-yl)phényl]-3-phénylpyridazine;
la 3-phényl-5-[3-(pyridin-2-ylméthoxy)phényl]pyridazine;
la 5-[4-fluoro-3-(3-fluoropyridin-4-yl)phényl]-3-phénylpyridazine;
la 5-[2-fluoro-3-(pyridin-4-yl)phényl]-3-phénylpyridazine;
la 5-[3-(3,5-difluoropyridin-2-yl)-4-fluorophényl]-3-phénylpyridazine;
la 5-[4-fluoro-3-(pyridin-3-yl)phényl]-3-phénylpyridazine;
le 5,2'-difluoro-5'-(6-phénylpyridazin-4-yl)biphényl-2-carbonitrile;
le 3,2'-difluoro-5'-(6-phénylpyridazin-4-yl)biphényl-2-carbonitrile;
le 6,2'-difluoro-5'-[6-(4-fluorophényl)pyridazin-4-yl]biphényl-2-carbonitrile;
le 4-fluoro-3'-(6-phénylpyridazin-4-yl)biphényl-2-carbonitrile;
le 6,2'-difluoro-5'-[6-(thién-2-yl)pyridazin-4-yl]biphényl-2-carbonitrile;
le 6,2'-difluoro-5'-[6-(4-méthoxyphényl)pyridazin-4-yl]biphényl-2-carbonitrile;
le 5'-[6-(3-chlorophényl)pyridazin-4-yl]-6,2'-difluorobiphényl-2-carbonitrile;
le 6,2'-difluoro-5'-[6-(pyridin-3-yl)pyridazin-4-yl]biphényl-2-carbonitrile;
le 5'-[6-(4-chlorophényl)pyridazin-4-yl]-6,2'-difluorobiphényl-2-carbonitrile;
le 6,2'-difluoro-5'-[6-(pyridin-4-yl)pyridazin-4-yl]biphényl-2-carbonitrile;
la 5-[3-(3,5-difluoropyridin-2-yl)-4-fluorophényl]-3-(4-fluorophényl)pyridazine;
la 5-[4-fluoro-3-(3-fluoropyridin-2-yl)phényl]-3-(2-fluorophényl)pyridazine;
la 5-[3-(3,5-difluoropyridin-2-yl)-4-fluorophényl]-3-(2-fluorophényl)pyridazine;
la 5-[4-fluoro-3-(3-fluoropyridin-2-yl)phényl]-3-(pyridin-3-yl)pyridazine;
la 5-[3-(3,5-difluoropyridin-2-yl)-4-fluorophényl]-3-(3-fluorophényl)pyridazine;
la 3-(2,4-difluorophényl)-5-[3-(3,5-difluoropyridin-2-yl)-4-fluorophényl]pyridazine;
la 5-[3-(3,5-difluoropyridin-2-yl)-4-fluorophényl]-3-(3-méthoxyphényl)pyridazine;
le 6,2'-difluoro-5'-[6-(2-fluorophényl)pyridazin-4-yl]biphényl-2-carbonitrile;
le 6,2'-difluoro-5'-[6-(3-fluorophényl)pyridazin-4-yl]biphényl-2-carbonitrile;
le 5'-[6-(2-chlorophényl)pyridazin-4-yl]-6,2'-difluorobiphényl-2-carbonitrile;
le 4-fluoro-3'-[6-(4-méthoxyphényl)pyridazin-4-yl]biphényl-2-carbonitrile;
la 5-[3-(3,5-difluoropyridin-2-yl)-4-fluorophényl]-3-(4-méthoxyphényl)pyridazine;
la 3-(4-chlorophényl)-5-[3-(3,5-difluoropyridin-2-yl)-4-fluorophényl]pyridazine;
le 2-{5-[3-(3,5-difluoropyridin-2-yl)-4-fluorophényl]pyridazin-3-yl}-5-fluorobenzonitrile; la 3-(4-chlorophényl)-5-[4-fluoro-3-(3-fluoropyridin-2-yl)phényl]pyridazine;
la 5-[4-fluoro-3-(3-fluoropyridin-2-yl)phényl]-3-(furan-3-yl)pyridazine;
la 5-[4-fluoro-3-(3-fluoropyridin-2-yl)phényl]-3-(furan-2-yl)pyridazine;
la 3-(2,3-difluorophényl)-5-[4-fluoro-3-(3-fluoropyridin-2-yl)phényl]pyridazine;
la 5-[4-fluoro-3-(3-fluoropyridin-2-yl)phényl]-3-(thién-3-yl)pyridazine;
la 5-[4-fluoro-3-(3-fluoropyridin-2-yl)phényl]-3-(thién-2-yl)pyridazine;
la 3-(2,5-difluorophényl)-5-[4-fluoro-3-(3-fluoropyridin-2-yl)phényl]pyridazine;
la 3-(3,4-difluorophényl)-5-[4-fluoro-3-(3-fluoropyridin-2-yl)phényl]pyridazine;
le 4-{5-[4-fluoro-3-(3-fluoropyridin-2-yl)phényl]pyridazin-3-yl}benzonitrile;
le 3'-(6-éthylaminopyridazin-4-yl)-4-fluorobiphényl-2-carbonitrile;
le 4-fluoro-3'-(6-isopropylaminopyridazin-4-yl)biphényl-2-carbonitrile;
le 4-fluoro-3'-(6-propylaminopyridazin-4-yl)biphényl-2-carbonitrile;
le 3'-(6-cyclopropylaminopyridazin-4-yl)-4-fluorobiphényl-2-carbonitrile;
le 3'-(6-allylaminopyridazin-4-yl)-4-fluorobiphényl-2-carbonitrile;
le 3'-(6-benzylaminopyridazin-4-yl)-4-fluorobiphényl-2-carbonitrile;
le 4-fluoro-3'-(6-méthylaminopyridazin-4-yl)biphényl-2-carbonitrile;
le 4-fluoro-3'-(6-méthoxypyridazin-4-yl)biphényl-2-carbonitrile;
le 3'-(6-éthoxypyridazin-4-yl)-4-fluorobiphényl-2-carbonitrile;
le 3'-(6-benzyloxypyridazin-4-yl)-4-fluorobiphényl-2-carbonitrile;
la 5-[4-fluoro-3-(2-méthyl-2H-[1,2,4]triazol-3-ylméthoxy)phényl]-3-phénylpyridazine;
la 5-[4-fluoro-3-(1-méthyl-3-trifluorométhyl-1H-pyrazol-4-ylméthoxy)phényl]-3-phényl-pyridazine;
la 5-[4-fluoro-3-(pyridin-4-ylméthoxy)phényl]-3-phénylpyridazine;
la 5-[4-fluoro-3-(pyridin-3-ylméthoxy)phényl]-3-phénylpyridazine;
la 5-[4-fluoro-3-(3-fluoropyridin-2-yl)phényl]-3-(pyridin-4-yl)pyridazine;
la 5-[4-fluoro-3-(3-fluoropyridin-2-yl)phényl]-3-(pyrazin-2-yl)pyridazine;
la 5-[4-fluoro-3-(3-fluoropyridin-2-yl)phényl]-3-(thiazol-2-yl)pyridazine;
la 5-[4-fluoro-3-(3-fluoropyridin-2-yl)phényl]-3-(pyridin-2-yl)pyridazine;
la 5-[4-fluoro-3-(3-fluoropyridin-2-yl)phényl]-3-(3-fluoropyridin-4-yl)pyridazine;
la 5-[4-fluoro-3-(3-fluoropyridin-2-yl)phényl]-3-(1H-[1,2,3]triazol-4-yl)pyridazine;
l'éthylester d'acide 5-[4-fluoro-3-(3-fluoropyridin-2-yl)phényl]pyridazine-3-carboxylique;
le 5-[3-(3,5-difluoropyridin-2-yl)-4-fluorophényl]-3-(2-fluorophényl)pyridazine-1-oxyde;
la 3-(2,6-difluorophényl)-5-[4-fluoro-3-(3-fluoropyridin-2-yl)phényl]pyridazine;
et sels pharmaceutiquement acceptables de ceux-ci.

8. Composé selon l'une quelconque des revendications 1 à 6, choisi parmi:
la 3-(4-chloro-2-fluorophényl)-5-[3-(3,5-difluoropyridin-2-yl)-4-fluorophényl]pyridazine;
la 5-[3-(3,5-difluoropyridin-2-yl)-4-fluorophényl]-3-(2-fluoro-4-trifluorométhylphényl)pyridazine;
la 5-[3-(3,5-difluoropyridin-2-yl)-4-fluorophényl]-3-(2-fluoro-4-méthylphényl)pyridazine;
la 3-(3,5-difluoropyridin-2-yl)-5-[3-(3,5-difluoropyridin-2-yl)-4-fluorophényl]pyridazine;
le 5-[4-fluoro-3-(3-fluoropyridin-2-yl)phényl]pyridazin-3-ylester d'acide trifluorométhane-sulfonique;
la 3-éthylsulfanyl-5-[4-fluoro-3-(3-fluoropyridin-2-yl)phényl]pyridazine;
la 3-tert-butylsulfanyl-5-[4-fluoro-3-(3-fluoropyridin-2-yl)phényl]pyridazine;
la 5-[3-(3,5-difluoropyridin-2-yl)-4-fluorophényl]-3-(3-fluoropyridin-4-yl)pyridazine;
la 5-[3-(3,5-difluoropyridin-2-yl)-4-fluorophényl]-3-(3-fluoropyridin-2-yl)pyridazine;
la 5-[4-fluoro-3-(3-fluoropyridin-2-yl)phényl]-3-(3-fluoropyridin-2-yl)pyridazine;
le 5-[4-fluoro-3-(3-fluoropyridin-2-yl)phényl]-3-(3-fluoropyridin-4-yl)pyridazine-1-oxyde;
la 5-[4-fluoro-3-(3-fluoropyridin-2-yl)phényl]-3-(3-fluoro-1-oxypyridin-4-yl)pyridazine;
la 5-[2,4-difluoro-3-(3,5-difluoropyridin-2-yl)phényl]-3-(3,5-difluoropyridin-4-yl)pyridazine;
la 5-[3-(3,5-difluoropyridin-2-yl)-4-fluorophényl]-3-(2-fluoro-4-méthoxyphényl)pyridazine;
la 5-[4-fluoro-3-(3-fluoropyridin-2-yl)phényl]-3-(2-fluoro-4-méthoxyphényl)pyridazine;
la 3-(3,5-difluoropyridin-4-yl)-5-[4-fluoro-3-(3-fluoropyridin-2-yl)phényl]pyridazine;
la 3-(3,5-difluoropyridin-2-yl)-5-[4-fluoro-3-(3-fluoropyridin-2-yl)phényl]pyridazine;
la 3-(3,5-difluoropyridin-4-yl)-5-[3-(3,5-difluoropyridin-2-yl)-4-fluorophényl]pyridazine;
et sels pharmaceutiquement acceptables de ceux-ci.

9. Composé selon l'une quelconque des revendications 1 à 6, choisi parmi:
la 3-(3,5-difluoro-1-oxypyridin-4-yl)-5-[3-(3,5-difluoropyridin-2-yl)-4-fluorophényl]pyridazine;
le 5'-[6-(3,5-difluoropyridin-2-yl)pyridazin-4-yl]-2'-fluorobiphényl-2-carbonitrile;
le 5'-[6-(3,5-difluoropyridin-4-yl)pyridazin-4-yl]-2'-fluorobiphényl-2-carbonitrile;
le 4,2'-difluoro-5'-[6-(3,5-difluoropyridin-4-yl)pyridazin-4-yl]biphényl-2-carbonitrile;
le 4,2'-difluoro-5'-[6-(3,5-difluoropyridin-2-yl)pyridazin-4-yl]biphényl-2-carbonitrile;
le 2-{5-[6-(3,5-difluoropyridin-4-yl)pyridazin-4-yl]-2-fluorophényl}nicotinonitrile;
le 2-{5-[6-(3,5-difluoropyridin-2-yl)pyridazin-4-yl]-2-fluorophényl}nicotinonitrile;
le 2'-fluoro-5'-[6-(2-oxopyrrolidin-1-yl)pyridazin-4-yl]biphényl-2-carbonitrile;
le 2'-fluoro-5'-[6-(2-oxo-2H-pyridin-1-yl)pyridazin-4-yl]biphényl-2-carbonitrile;
le 6,2'-difluoro-5'-[6-(3,5-difluoropyridin-2-yl)pyridazin-4-yl]biphényl-2-carbonitrile;
la 3-(3,5-difluoropyridin-2-yl)-5-(4-fluoro-3-trifluorométhylphényl)pyridazine;
la 3-(3,5-difluoropyridin-2-yl)-5-(6-fluoro-2'-trifluorométhylbiphényl-3-yl)pyridazine;
la 5-(6,2'-difluorobiphényl-3-yl)-3-(3,5-difluoropyridin-2-yl)pyridazine;
la 3-(3,5-difluoropyridin-2-yl)-5-(6,2',4'-trifluorobiphényl-3-yl)pyridazine;
la 5-[3-(3,5-difluoropyridin-2-yl)-4-fluorophényl]-3-(2,4,6-trifluorophényl)pyridazine;
et sels pharmaceutiquement acceptables de ceux-ci.

10. Composition pharmaceutique comprenant un composé de la formule 1 selon l'une quelconque des revendications précédentes, ou un N-oxyde de celui-ci ou un sel pharmaceutiquement acceptable de celui-ci, en association avec un support pharmaceutiquement acceptable.

11. Utilisation d'un composé selon l'une quelconque des revendications 1 à 9, ou d'un N-oxyde de celui-ci ou d'un sel pharmaceutiquement acceptable de celui-ci, pour la fabrication d'un médicament pour le traitement et/ou la prévention de troubles neurologiques.

12. Procédé pour la préparation d'un composé selon la revendication 1, qui comprend:
(A) la réaction d'un composé de la formule III avec un composé de la formule IV: 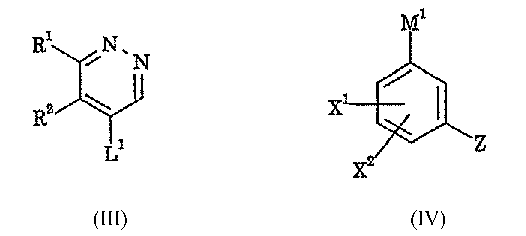 dans lesquelles formules X¹, X², Z, R¹ et R² sont tels que définis dans la revendication 1, L¹ représente un groupe partant approprié, et M¹ représente une fraction d'acide boronique -B(OH)₂ ou un ester cyclique de celui-ci formé avec un diol organique, ou M¹ représente -Sn(Alk)₃ où Alk représente un groupe alkyle C₁₋₆, ou M¹ représente -ZnHal où Hal représente un halogène; en présence d'un catalyseur métal de transition; ou
(B) la réaction d'un composé de la formule V avec un composé de la formule VI: 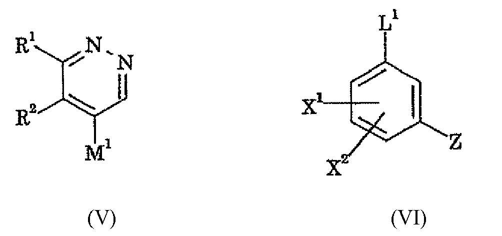 dans lesquelles formules X¹, X², Z, R¹ et R² sont tels que définis dans la revendication 1, et L¹ et M¹ sont tels que définis ci-dessus; en présence d'un catalyseur métal de transition; ou
(C) la réaction d'un composé de la formule VII avec un composé de la formule VIII: 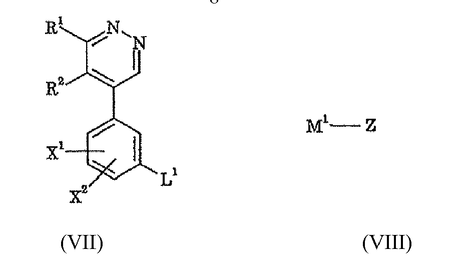 dans lesquelles formules X¹, X², Z, R¹ et R² sont tels que définis dans la revendication 1, et L¹ et M¹ sont tels que définis ci-dessus; en présence d'un catalyseur métal de transition; ou
(D) la réaction d'un composé de la formule IX avec un composé de la formule X: 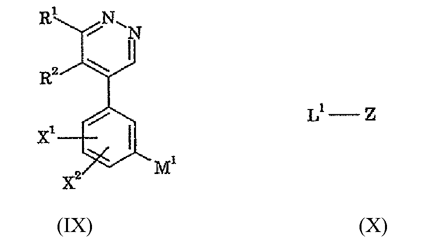 dans lesquelles formules X¹, X², Z, R¹ et R² sont tels que définis dans la revendication 1, et L¹ et M¹ sont tels que définis ci-dessus; en présence d'un catalyseur métal de transition; ou
(E) la réaction d'un composé de la formule XI avec un composé de la formule XII: 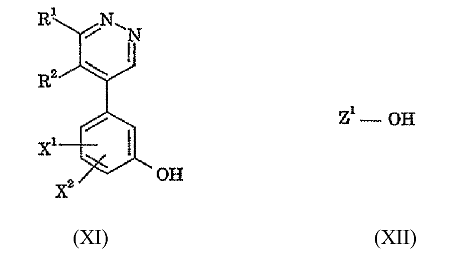 dans lesquelles formules X¹, X², R¹ et R² sont tels que définis dans la revendication 1, et Z¹ représente un groupe alkyle C₁₋₆ ou un groupe hétéroaryl-alkyle C₁₋₆ optionnellement substitué; en présence de triphénylphosphine et d'un dialkylazodicarboxylate; ou
(F) la réaction d'un composé de la formule XIV avec un composé de la formule XV: 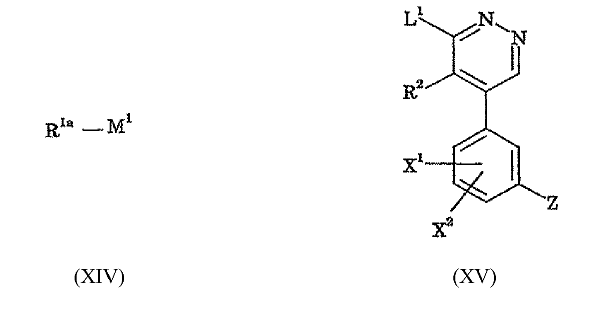 dans lesquelles formules X¹, X², Z et R² sont tels que définis dans la revendication 1, L¹ et M¹ sont tels que définis ci-dessus, et R^{1a} représente une fraction aryle ou hétéroaryle; en présence d'un catalyseur métal de transition; ou
(G) la réaction d'un composé de la formule XVI avec un composé de la formule XVII: 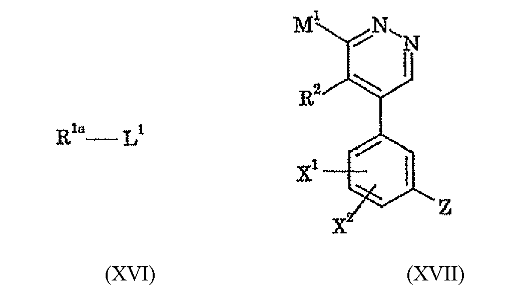 dans lesquelles formules X¹, X² Z et R² sont tels que définis dans la revendication 1, et R^{1a}, L¹ et M¹ sont tels que définis ci-dessus; en présence d'un catalyseur métal de transition; ou
(H) la réaction d'un composé de la formule XVIII: 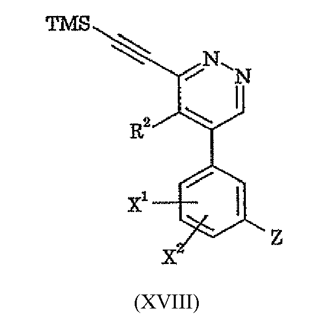 dans laquelle X¹, X², Z et R² sont tels que définis dans la revendication 1, et TMS est une abréviation pour triméthylsilanyle; avec de l'azide de sodium; ou
(J) la réaction d'un composé de la formule XV telle que définie ci-dessus avec un composé de la formule R^{a}-OH, dans laquelle R^{a} est tel que défini dans la revendication 1; ou
(K) la réaction d'un composé de la formule XV telle que définie ci-dessus avec un sel de la formule R^{a}S-Na⁺, dans laquelle R^{a} est tel que défini dans la revendication 1; ou
(L) la réaction d'un composé de la formule XV telle que définie ci-dessus avec un composé de la formule H-NR^{a}R^{b}, dans laquelle R^{a} et R^{b} sont tels que définis dans la revendication 1; ou
(M) la réaction d'un composé de la formule XV telle que définie ci-dessus avec du dioxyde de carbone et un composé de la formule R^{a}-OH, dans laquelle R^{a} est tel que défini dans la revendication 1; en présence d'un catalyseur métal de transition; ou
(N) la réaction d'un composé de la formule VII ci-dessus, dans laquelle L¹ représente un atome d'halogène, avec du cyanure de zinc; en présence d'un catalyseur métal de transition; ou
(P) la réaction d'un composé de la formule XXII: 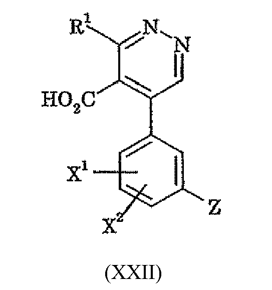 dans laquelle X¹, X², Z et R¹ sont tels que définis dans la revendication 1; avec du diazométhane; ou
(Q) la réaction d'un composé de la formule XXIII: 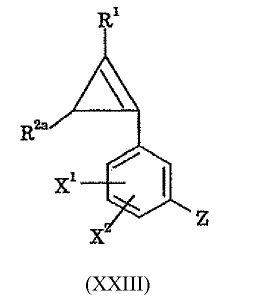 dans laquelle X¹, X², Z et R¹ sont tels que définis dans la revendication 1, et R^{2a} représente un groupe alcoxycarbonyle C₂₋₆; avec du diazométhane; et
(R) par la suite, s'il y a lieu, la conversion d'un composé de la formule I initialement obtenu en un composé supplémentaire de la formule I par des méthodes standards.

13. Composé selon l'une quelconque des revendications 1 à 9, ou N-oxyde de celui-ci ou sel pharmaceutiquement acceptable de celui-ci, pour une utilisation dans une méthode de traitement du corps humain par une thérapie.
